# EUROPEAN PATENT APPLICATION

(11) **EP 4 512 892 A1**
(43) Date of publication of application: **26.02.2025**
(21) Application number: 23791850.3
(22) Date of filing: 18.04.2023
(51) Int. Cl.: C12N 9/24, C07K 19/00, C12N 5/10, C12N 15/56, C12N 15/62

(54) **GLYCOPROTEIN HAVING REDUCED NUMBER OF MANNOSE-6-PHOSPHATES**

(30) Priority: 19.04.2022 JP 2022069114
(71) Applicant: JCR Pharmaceuticals Co., Ltd., Ashiya-shi Hyogo 659-0021 (JP)
(72) Inventor: YANASAKA, Ryota, Kobe-shi, Hyogo 651-2241 (JP); TAKAHASHI, Kenichi, Kobe-shi, Hyogo 651-2241 (JP)
(74) Representative: Balder IP Law, S.L.
(86) International application number: PCT/JP2023/015436
(87) International publication number: WO 2023/204202

(57) **Abstract**

Problem

To provide a method for producing a glycoprotein having N-linked sugar chains containing M6Ps in a wild type as a protein (M6P low-modified protein) in which the M6Ps originally contained in the glycoprotein are deleted or the number thereof is reduced, the M6P low-modified protein, and a method for utilizing the M6P low-modified protein.

Resolution means

A protein in which at least one of one or more N-linked sugar chains that is linked to Asn is deleted by adding a mutation to at least one amino acid sequence represented by Asn-Xaa-Yaa (where Xaa represents an amino acid other than proline, and Yaa represents threonine or serine) contained in an amino acid sequence of a glycoprotein having, in a wild type, one or more N-linked sugar chains binding thereto, the one or more N-linked sugar chains each containing one or more mannose-6-phosphate (M6Ps).

## Description

### Technical Field

In one embodiment, the present invention relates to a protein (M6P low-modified protein) in which mannose-6-phosphates (M6Ps) originally contained in a sugar chain of a glycoprotein having an N-linked sugar chain containing M6Ps in a wild type are deleted or the number thereof is reduced. In another embodiment, the M6P low-modified protein is bound to an antibody against a receptor present on the surface of a cerebrovascular endothelial cell to make a fusion protein, which is allowed to pass through the blood-brain barrier (BBB) via the binding to the receptor. In the glycoprotein or fusion protein, M6Ps which should be originally contained in a portion corresponding to a glycoprotein constituting the glycoprotein or fusion protein are deleted or the number thereof is reduced. Thus, when administered in vivo, the glycoprotein or fusion protein can be inhibited from being taken up into a cell expressing M6Ps via an M6P receptor, which can suppress a decrease in the number of the receptors present on the surface of the cell.

### Background Art

Some glycoproteins having an N-linked sugar chain containing M6Ps are taken up into a cell via an M6P receptor (M6PR) present on the cell. Examples of such a protein to which an N-linked sugar chain containing M6Ps is added include a lysosomal enzyme. A lysosomal enzyme is a generic term for a plurality of acidic hydrolases contained in a lysosome, which is an organelle in an animal cell, and a substrate to be decomposed is determined depending on a type of the enzyme. A genetic defect in any of the lysosomal enzymes causes a lysosomal disease (LSD) in which a substrate to be decomposed accumulates intracellularly due to the lack or deficiency of the enzyme. Corresponding to the type of a lysosomal enzyme that is genetically deficient, a different lysosomal disease develops. For a patient with such a lysosomal disease, enzyme replacement therapy (ERT) is performed in which an insufficient or deficient enzyme is administered exogenously as a drug. Many lysosomal enzymes each have a sugar chain structure containing an M6P at a specific site, and are transported to lysosomes by binding to M6P receptors present on the surfaces of cells or in Golgi bodies. An M6P is an important ligand for transport of lysosomal enzymes to lysosomes, and the transport of lysosomal enzymes to lysosomes in the enzyme replacement therapy depends on this pathway via M6P receptors. Accordingly, lysosomal enzymes produced as recombinant proteins to be used in the usual enzyme replacement therapy need to be produced as those containing M6Ps in their sugar chains to be efficiently transported to lysosomes.

On the other hand, in such enzyme replacement therapy, the enzyme administered to the patient is unlikely to migrate from capillaries to the brain due to presence of the blood-brain barrier (BBB), which is a mechanism for limiting exchange of substances such as polymers between the blood and the tissue fluid of the brain via the capillary endothelium (cerebrovascular endothelial cells) in the brain. Thus, administration of formulations containing these enzymes has limited efficacy against disorder in cells and tissues of the nervous system, including the brain and spinal cord. For example, for mucopolysaccharidosis type I (Hurler's syndrome), which is a hereditary metabolic disease caused by deficiency of α-L-iduronidase, enzyme replacement therapy, which involves intravenous replacement of recombinant α-L-iduronidase, has been practiced as a treatment for this disease. However, this is not effective for marked central nervous system (CNS) abnormalities observed in the Hurler's syndrome because the enzyme cannot pass through the blood-brain barrier. As a method for enabling a polymer substance to reach the brain through the blood-brain barrier, various methods have been reported in which a polymer substance is modified to have affinity for a membrane protein present on endothelial cells of cerebral capillaries. Examples of the membrane protein present on endothelial cells of cerebral capillaries include receptors for compounds such as insulin, transferrin, insulin-like growth factors (IGF-I and IGF-II), LDL, and leptin.

For example, a technique has been reported in which a nerve growth factor (NGF) is synthesized in the form of a fusion protein with insulin, and this fusion protein is allowed to pass through the blood-brain barrier via binding to an insulin receptor (Patent Documents 1 to 3). Further, a technique has been reported in which a nerve growth factor (NGF) is synthesized in the form of a fusion protein with an anti-insulin receptor antibody, and this fusion protein is allowed to pass through the blood-brain barrier via binding to an insulin receptor (Patent Documents 1 and 4). Furthermore, a technique has been reported in which a nerve growth factor (NGF) is synthesized in the form of a fusion protein with transferrin, and this fusion protein is allowed to pass through the blood-brain barrier via binding to a transferrin receptor (TfR) (Patent Document 5). In addition, a technique has been reported in which a nerve growth factor (NGF) is synthesized in the form of a fusion protein with an anti-transferrin receptor antibody (anti-TfR antibody), and this fusion protein is allowed to pass through the blood-brain barrier via binding to TfR (Patent Documents 1 and 6).

Not only lysosomal enzymes but also other substances taken up into cells via M6P receptors are transported to lysosomes. In many cases, a substance transported to a lysosome is decomposed in the lysosome. Thus, a technique has been reported in which using an antibody modified with an M6P, an antigen specifically recognized by the antibody is taken up by a cell via an M6P receptor, transported to a lysosome, and decomposed (Non-Patent Documents 1 and 2).

### Citation List

### Patent Documents

Patent Document 1: US 5154924
Patent Document 2: JP 2011-144178 A
Patent Document 3: US 2004/0101904 A1
Patent Document 4: JP 2006-511516 T
Patent Document 5: JP H06-228199 A
Patent Document 6: US 5977307

### Non-Patent Documents

Non-Patent Document 1: Banik SM. et al., Nature. 584 (7820). 291-297 (2020)
Non-Patent Document 2: Pei J. et al., J Med Chem. 64 (7). 3493-3507 (2021)

### Summary of Invention

### Technical Problem

In one embodiment, an object of the present invention is to provide a method for producing a protein (M6P low-modified protein) in which M6Ps originally contained in a sugar chain of a glycoprotein having an N-linked sugar chain containing M6Ps in a wild type are deleted or the number thereof is reduced, the M6P low-modified protein, and a method for utilizing the M6P low-modified protein. In one embodiment, another object of the present invention is to provide a method for producing an M6P low-modified protein as a conjugate or a fusion protein with a ligand for a protein present on a cell (excluding an M6P receptor, referred to herein as a "target protein"), the fusion protein, and a method for using the fusion protein. When administered in vivo, the fusion protein binds to the target protein and is then taken up into the cell to exhibit physiological activity, while the number of the target proteins present on the cell is not significantly reduced. The object of the present invention is not limited thereto and is disclosed throughout the present specification.

### Solution to Problem

As a result of intensive studies in the research for the above purpose, the present inventors have found that when cells expressing hTfR and M6Ps are cultured with addition of a fusion protein obtained by binding human iduronate-2-sulfatase (hIDS) having a mutation for deleting M6Ps originally contained in a wild type to an anti-human transferrin receptor antibody (anti-hTfR antibody), a decrease of hTfR present on the cells can be prevented and physiological activity originally possessed by the original wild-type hIDS can be maintained, as compared with a fusion protein obtained by binding the wild-type hIDS to the anti-hTfR antibody, thereby completing the present invention. That is, the present invention includes the following.
1. A glycoprotein including:
   in a wild type, one or more N-linked sugar chains binding thereto, and
   the one or more N-linked sugar chains each containing one or more mannose-6-phosphates (M6Ps); wherein
   the protein includes deletion of at least one of the M6Ps.
2. The protein according to 1, wherein at least one of the one or more N-linked sugar chains is deleted.
3. The protein according to 1 or 2, wherein the glycoprotein contains a mutation in such a manner that at least one of the one or more N-linked sugar chains is deleted from an amino acid sequence of a wild-type glycoprotein corresponding to the glycoprotein.
4. The protein according to 3, wherein the mutation of the amino acid sequence includes at least one mutation selected from the group consisting of the following (1-a) to (1-c) with respect to an amino acid sequence (consensus sequence) represented by Asn-Xaa-Yaa (where Xaa represents an amino acid other than proline, and Yaa represents threonine or serine) containing asparagine (Asn) to which the N-linked sugar chain binds, and
   the mutation newly generates no consensus sequence to which an N-linked sugar chain binds:
   (1-a) deletion of Asn or substitution of Asn with an amino acid other than asparagine;
   (1-b) substitution of an amino acid represented by Xaa with proline; and
   (1-c) substitution of an amino acid represented by Yaa with an amino acid other than threonine and serine.
5. The protein according to 3, wherein a mutation is added in such a manner that at least one of the one or more N-linked sugar chains that is linked to Asn is deleted by adding a mutation to at least one amino acid sequence represented by Asn-Xaa-Yaa (where Xaa represents an amino acid other than proline, and Yaa represents threonine or serine) contained in the amino acid sequence of the glycoprotein.
6. The protein according to any one of 1 to 5, wherein the protein is a lysosomal enzyme.
7. The protein according to 6, wherein the lysosomal enzyme is selected from the group consisting of iduronate-2-sulfatase, α-L-iduronidase, glucocerebrosidase, β-galactosidase, GM2 activating proteins, β-hexosaminidase A, β-hexosaminidase B, N-acetylglucosamine-1-phosphotransferase, a-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl-CoAα-glucosaminide N-acetyltransferase, N-acetylglucosamine-6-sulfatase, acid ceramidase, amylo-1,6-glucosidase, sialidase, palmitoylprotein thioesterase-1, tripeptidylpeptidase-1, hyaluronidase-1, acid α-glucosidase, CLN1, and CLN2.
8. The protein according to 6 or 7, wherein the lysosomal enzyme is a human lysosomal enzyme.
9. The protein according to 8, wherein the human lysosomal enzyme is human iduronate-2-sulfatase (hIDS).
10. The protein according to 9, wherein the hIDS includes at least one mutation selected from the group consisting of the following (2-a) to (2-c) with respect to an amino acid sequence represented by Asn221-Ile222-Thr223 corresponding to from asparagine at position 221 to threonine at position 223 in an amino acid sequence of a wild-type hIDS represented by SEQ ID NO: 1, and
   the at least one mutation newly generates no consensus sequence to which an N-linked sugar chain binds:
   (2-a) deletion of Asn221 or substitution of Asn221 with an amino acid other than asparagine;
   (2-b) substitution of Ile222 with proline; and
   (2-c) substitution of Thr223 with an amino acid other than threonine and serine.
11. The protein according to 9, wherein the hIDS is mutated in such a manner that the N-linked sugar chain binding to the asparagine at position 221 is deleted by adding a mutation to the amino acid sequence corresponding to from the asparagine at position 221 to the threonine at position 223 in the amino acid sequence of the wild-type hIDS represented by SEQ ID NO: 1.
12. The protein according to 10 or 11 wherein the hIDS further includes a mutation selected from the group consisting of the following (2'-a) to (2'-h) in an amino acid sequence other than the amino acid sequence corresponding to from asparagine at position 221 to threonine at position 223 in the amino acid sequence of the wild-type hIDS represented by SEQ ID NO: 1, and
   the mutation newly generates no consensus sequence to which an N-linked sugar chain binds:
   (2'-a) substitution of 1 to 10 amino acids with another amino acid or other amino acids;
   (2'-b) deletion of 1 to 10 amino acids;
   (2'-c) a combination of the substitution of (2'-a) and the deletion of (2'-b);
   (2'-d) addition of 1 to 10 amino acids into the amino acid sequence or to an N-terminus or a C-terminus of the amino acid sequence;
   (2'-e) a combination of the substitution of (2'-a) and the addition of (2'-d);
   (2'-f) a combination of the deletion of (2'-b) and the addition of (2'-d);
   (2'-g) a combination of the substitution of (2'-a), the deletion of (2'-b), and the addition of (2'-d); and
   (2'-h) exhibition of an identity of 80% or more.
13. The protein according to 9, wherein the hIDS includes at least one mutation selected from the group consisting of the following (3-a) to (3-c) with respect to an amino acid sequence represented by Asn255-Ile256-Ser257 corresponding to from asparagine at position 255 to serine at position 257 in the amino acid sequence of the wild-type hIDS represented by SEQ ID NO: 1, and
   the mutation newly generates no consensus sequence to which an N-linked sugar chain binds:
   (3-a) deletion of Asn255 or substitution of Asn 255 with an amino acid other than Asn;
   (3-b) substitution of Ile256 with proline; and
   (3-c) substitution of Ser257 with an amino acid other than threonine and serine.
14. The protein according to 9, wherein the hIDS is mutated in such a manner that the N-linked sugar chain binding to the asparagine at position 255 is deleted by adding a mutation to the amino acid sequence corresponding to from the asparagine at position 255 to the serine at position 257 in the amino acid sequence of the wild-type hIDS represented by SEQ ID NO: 1.
15. The protein according to 13 or 14, wherein the hIDS further includes a mutation selected from the group consisting of the following (3'-a) to (3'-h) in an amino acid sequence other than the amino acid sequence corresponding to from asparagine at position 255 to serine at position 257 in the amino acid sequence of the wild-type hIDS represented by SEQ ID NO: 1, and
   the mutation newly generates no consensus sequence to which an N-linked sugar chain binds:
   (3'-a) substitution of 1 to 10 amino acids with another amino acid or other amino acids;
   (3'-b) deletion of 1 to 10 amino acids;
   (3'-c) a combination of the substitution of (3'-a) and the deletion of (3'-b);
   (3'-d) addition of 1 to 10 amino acids into an amino acid sequence or to an N-terminus or a C-terminus of the amino acid sequence;
   (3'-e) a combination of the substitution of (3'-a) and the addition of (3'-d);
   (3'-f) a combination of the deletion of (3'-b) and the addition of (3'-d);
   (3'-g) a combination of the substitution of (3'-a), the deletion of (3'-b), and the addition of (3'-d); and
   (3'-h) exhibition of an identity of 80% or more.
16. The protein according to 9, wherein the hIDS has an amino acid sequence selected from the following (4-a) to (4-e) and has no new consensus sequence to which an N-linked sugar chain binds:
   (4-a) an amino acid sequence exhibiting, in the amino acid sequence except for amino acids at positions 221 and 255, an identity of 80% or more, 85% or more, 90% or more, 95% or more, 98% or more, or 99% or more, to an amino acid sequence of an hIDS mutant represented by SEQ ID NO: 4 in which asparagines at positions 221 and 255 in the amino acid sequence of the wild-type hIDS represented by SEQ ID NO: 1 are each substituted with glutamine;
   (4-b) the amino acid sequence of the hIDS mutant represented by SEQ ID NO: 4 in which asparagines at positions 221 and 255 in the amino acid sequence of the wild-type hIDS represented by SEQ ID NO: 1 are each substituted with glutamine;
   (4-c) an amino acid sequence exhibiting, in the amino acid sequence except for amino acids at positions 223 and 257, an identity of 80% or more, 85% or more, 90% or more, 95% or more, 98% or more, or 99% or more, to an amino acid sequence of an hIDS mutant represented by SEQ ID NO: 5 in which threonine at position 223 and serine at position 257 in the amino acid sequence of the wild-type hIDS represented by SEQ ID NO: 1 are each substituted with alanine;
   (4-d) the amino acid sequence of the hIDS mutant represented by SEQ ID NO: 5 in which threonine at position 223 and serine at position 257 in the amino acid sequence of the wild-type hIDS represented by SEQ ID NO: 1 are each substituted with alanine; and
   (4-e) an amino acid sequence of an hIDS mutant represented by SEQ ID NO: 6 in which asparagines at positions 221 and 255 are each substituted with glutamine, and threonine at position 223 and serine at position 257 are each substituted with alanine in the amino acid sequence of the wild-type hIDS represented by SEQ ID NO: 1.
17. The protein according to any one of 9 to 16, having an enzyme activity of 20% or more as compared with the wild-type hIDS.
18. The protein according to any one of 9 to 16, having an enzyme activity of 50% or more as compared with the wild-type hIDS.
19. The protein according to 8, wherein the human lysosomal enzyme is human α-L-iduronidase (hIDUA).
20. The protein according to 19, wherein the hIDUA includes at least one mutation selected from the group consisting of the following (5-a) to (5-c) with respect to an amino acid sequence represented by Asn311-Thr312-Thr313 corresponding to from asparagine at position 311 to threonine at position 313 in an amino acid sequence of a wild-type hIDUA represented by SEQ ID NO: 7, and
   the mutation newly generates no consensus sequence to which an N-linked sugar chain binds:
   (5-a) deletion of Asn311 or substitution of Asn311 with an amino acid other than asparagine;
   (5-b) substitution of Thr312 with proline; and
   (5-c) substitution of Thr313 with an amino acids other than threonine and serine.
21. The protein according to 19, wherein the hIDUA is mutated in such a manner that the N-linked sugar chain binding to the asparagine at position 311 is deleted by adding a mutation to the amino acid sequence corresponding to from the asparagine at position 311 to the threonine at position 313 in the amino acid sequence of the wild-type hIDUA represented by SEQ ID NO: 7.
22. The protein according to 20 or 21, wherein the hIDUA further includes a mutation selected from the group consisting of the following (5'-a) to (5'-h) in an amino acid sequence other than the amino acid sequence corresponding to from asparagine at position 311 to threonine at position 313 in the amino acid sequence of the wild-type hIDUA represented by SEQ ID NO: 7, and
   the mutation newly generates no consensus sequence to which an N-linked sugar chain binds:
   (5'-a) substitution of 1 to 10 amino acids with another amino acid or other amino acids;
   (5'-b) deletion of 1 to 10 amino acids;
   (5'-c) a combination of the substitution of (5'-a) and the deletion of (5'-b);
   (5'-d) addition of 1 to 10 amino acids into an amino acid sequence or to an N-terminus or a C-terminus of the amino acid sequence;
   (5'-e) a combination of the substitution of (5'-a) and the addition of (5'-d);
   (5'-f) a combination of the deletion of (5'-b) and the addition of (5'-d);
   (5'-g) a combination of the substitution of (5'-a), the deletion of (5'-b), and the addition of (5'-d); and
   (5'-h) exhibition of an identity of 80% or more.
23. The protein according to 19, wherein the hIDUA includes at least one mutation selected from the group consisting of the following (6-a) to (6-c) with respect to an amino acid sequence represented by Asn426-Arg427-Ser428 corresponding to from asparagine at position 426 to serine at position 428 in the amino acid sequence of the wild-type hIDUA represented by SEQ ID NO: 7, and
   the at least one mutation newly generates no consensus sequence to which an N-linked sugar chain binds:
   (6-a) deletion of Asn426 or substitution of Asn426 with an amino acid other than asparagine;
   (6-b) substitution of Arg427 with proline; and
   (6-c) substitution of Ser428 with an amino acid other than threonine and serine.
24. The protein according to 19, wherein the hIDUA is mutated in such a manner that the N-linked sugar chain binding to the asparagine at position 426 is deleted by adding a mutation to the amino acid sequence corresponding to from the asparagine at position 426 to the serine at position 428 in the amino acid sequence of the wild-type hIDUA represented by SEQ ID NO: 7.
25. The protein according to 23 or 24, wherein the hIDUA further includes a mutation selected from the group consisting of the following (6'-a) to (6'-h) in an amino acid sequence other than the amino acid sequence corresponding to from the asparagine at position 426 to the serine at position 428 in the amino acid sequence of the wild-type hIDUA represented by SEQ ID NO: 7, and
   the mutation newly generates no consensus sequence to which an N-linked sugar chain binds:
   (6'-a) substitution of 1 to 10 amino acids with another amino acid or other amino acids;
   (6'-b) deletion of 1 to 10 amino acids;
   (6'-c) a combination of the substitution of (6'-a) and the deletion of (6'-b);
   (6'-d) addition of 1 to 10 amino acids into an amino acid sequence or to an N-terminus or a C-terminus of the amino acid sequence;
   (6'-e) a combination of the substitution of (6'-a) and the addition of (6'-d);
   (6'-f) a combination of the deletion of (6'-b) and the addition of (6'-d);
   (6'-g) a combination of the substitution of (6'-a), the deletion of (6'-b), and the addition of (6'-d); and
   (6'-h) exhibition of an identity of 80% or more.
26. The protein according to 19, wherein the hIDUA has an amino acid sequence selected from the following (7-a) to (7-e) and has no new consensus sequence to which an N-linked sugar chain binds:
   (7-a) an amino acid sequence exhibiting, in the amino acid sequence except for amino acids at positions 311 and 426, an identity of 80% or more, 85% or more, 90% or more, 95% or more, 98% or more, or 99% or more, to an amino acid sequence of an hIDUA mutant represented by SEQ ID NO: 10 in which both asparagines at positions 311 and 426 in the amino acid sequence of the wild-type hIDUA represented by SEQ ID NO: 7 are each substituted with glutamine;
   (7-b) the amino acid sequence of the hIDUA mutant represented by SEQ ID NO: 10 in which both asparagines at positions 311 and 426 in the amino acid sequence of the wild-type hIDUA represented by SEQ ID NO: 7 are each substituted with glutamine;
   (7-c) an amino acid sequence exhibiting, in the amino acid sequence except for amino acids at positions 313 and 428, an identity of 80% or more, 85% or more, 90% or more, 95% or more, 98% or more, or 99% or more, to an amino acid sequence of an hIDUA mutant represented by SEQ ID NO: 11 in which threonine at position 313 and serine at position 428 in the amino acid sequence of the wild-type hIDUA represented by SEQ ID NO: 7 are each substituted with alanine;
   (7-d) the amino acid sequence of the hIDUA mutant represented by SEQ ID NO: 11 in which threonine at position 313 and serine at position 428 in the amino acid sequence of the wild-type hIDUA represented by SEQ ID NO: 7 are each substituted with alanine; and
   (7-e) an amino acid sequence of a hIDUA mutant represented by SEQ ID NO: 12 in which both asparagines at positions 311 and 426 in the amino acid sequence of the wild-type hIDUA represented by SEQ ID NO: 7 are each substituted with glutamine, and threonine at position 313 and serine at position 428 are each substituted with alanine.
27. The protein according to any one of 19 to 26, having an enzyme activity of 20% or more as compared with the wild-type hIDUA.
28. The protein according to any one of 19 to 26, having an enzyme activity of 50% or more as compared with the wild-type hIDUA.
29. The protein according to 8, wherein the human lysosomal enzyme is human heparan N-sulfatase (hSGSH).
30. The protein according to 29, wherein the hSGSH includes at least one mutation selected from the group consisting of the following (8-a) to (8-c) with respect to an amino acid sequence represented by Asn244-Asp245-Thr246 corresponding to from asparagine at position 244 to threonine at position 246 in an amino acid sequence of a wild-type hSGSH represented by SEQ ID NO: 13, and
   the at least one mutation newly generates no consensus sequence to which an N-linked sugar chain binds:
   (8-a) deletion of Asn244 or substitution of Asn244 with an amino acid other than asparagine;
   (8-b) substitution of Asp245 with proline; and
   (8-c) substitution of Thr246 with an amino acid other than threonine and serine.
31. The protein according to 29, wherein the hSGSH is mutated in such a manner that the N-linked sugar chain binding to the asparagine at position 244 is deleted by adding a mutation to the amino acid sequence corresponding to from the asparagine at position 244 to the threonine at position 246 in the amino acid sequence of the wild-type hSGSH represented by SEQ ID NO: 13.
32. The protein according to 30 or 31, wherein the hSGSH further includes a mutation selected from the group consisting of the following (8'-a) to (8'-h) in an amino acid sequence other than the amino acid sequence corresponding to from asparagine at position 244 to threonine at position 246 in the amino acid sequence of the wild-type hSGSH represented by SEQ ID NO: 13, and
   the mutation newly generates no consensus sequence to which an N-linked sugar chain binds:
   (8'-a) substitution of 1 to 10 amino acids with another amino acid or other amino acids;
   (8'-b) deletion of 1 to 10 amino acids;
   (8'-c) a combination of the substitution of (8'-a) and the deletion of (8'-b);
   (8'-d) addition of 1 to 10 amino acids into the amino acid sequence or to an N-terminus or a C-terminus of the amino acid sequence;
   (8'-e) a combination of the substitution of (8'-a) and the addition of (8'-d);
   (8'-f) a combination of the deletion of (8'-b) and the addition of (8'-d);
   (8'-g) a combination of the substitution of (8'-a), the deletion of (8'-b) and the addition of (2'-d); and
   (8'-h) exhibition of an identity of 80% or more.
33. The protein according to 29, wherein the hSGSH has an amino acid sequence selected from the following (9-a) to (9-e), and has no new consensus sequence to which a N-linked sugar chain binds:
   (9-a) an amino acid sequence exhibiting, in the amino acid sequence except for an amino acid at position 244, an identity of 80% or more, 85% or more, 90% or more, 95% or more, 98% or more, or 99% or more, to an amino acid sequence of an hSGSH mutant represented by SEQ ID NO: 16 in which asparagine at position 244 is substituted with glutamine in the amino acid sequence of the wild-type hSGSH represented by SEQ ID NO: 13;
   (9-b) the amino acid sequence of the hSGSH mutant represented by SEQ ID NO: 16 in which asparagine at position 244 in the amino acid sequence of the wild-type hSGSH represented by SEQ ID NO: 13 is substituted with glutamine;
   (9-c) an amino acid sequence exhibiting, in the amino acid sequence except for an amino acid at position 246, an identity of 80% or more, 85% or more, 90% or more, 95% or more, 98% or more, or 99% or more, to an amino acid sequence of an hSGSH mutant represented by SEQ ID NO: 17 in which threonine at position 246 in the amino acid sequence of the wild-type hSGSH represented by SEQ ID NO: 13 is substituted with alanine;
   (9-d) the amino acid sequence of the hSGSH mutant represented by SEQ ID NO: 17 in which threonine at position 246 in the amino acid sequence of the wild-type hSGSH represented by SEQ ID NO: 13 is substituted with alanine; and
   (9-e) an amino acid sequence of an hSGSH mutant represented by SEQ ID NO: 18 in which asparagine at position 244 is substituted with glutamine and threonine at position 246 is substituted with alanine in the amino acid sequence of the wild-type hSGSH represented by SEQ ID NO: 13.
34. The protein according to any one of 29 to 33, having an enzyme activity of 20% or more as compared with the wild-type hSGSH.
35. The protein according to any one of 29 to 33, having an enzyme activity of 50% or more as compared with the wild-type hSGSH.
36. The protein according to 8, wherein the human lysosomal enzyme is human acid α-glucosidase (hGAA).
37. The protein according to 36, wherein the hGAA includes at least one mutation selected from the group consisting of the following (10-a) to (10-i) with respect to at least one of an amino acid sequence represented by Asn71-Leu72-Ser73 corresponding to from asparagine at position 71 to serine at position 73, an amino acid sequence of Asn164-Thr165-Thr166 corresponding to from asparagine at position 164 to threonine at position 166, and an amino acid sequence represented by Asn401-Glu402-Thr403 corresponding to from asparagine at position 401 to threonine at position 403 in an amino acid sequence of a wild-type hGAA represented by SEQ ID NO: 19, and
   the at least one mutation newly generates no consensus sequence to which an N-linked sugar chain binds:
   (10-a) deletion of Asn71 or substitution of Asn71 with an amino acid other than asparagine;
   (10-b) substitution of Leu72 with proline;
   (10-c) substitution of Ser73 with an amino acid other than threonine and serine;
   (10-d) deletion of Asn164 or substitution of Asn164 with an amino acid other than asparagine;
   (10-e) substitution of Thr165 with proline;
   (10-f) substitution of Thr166 with an amino acid other than threonine and serine;
   (10-g) deletion of Asn401 or substitution of Asn401 with an amino acid other than asparagine;
   (10-h) substitution of Glu402 with proline; and
   (10-i) substitution of Thr403 with an amino acid other than threonine and serine.
38. The protein according to 36, wherein the hGAA is mutated in such a manner that the N-linked sugar chain binding to asparagine at position 71, 164, or 401 is deleted by adding a mutation to at least one of the amino acid sequence corresponding to from asparagine at position 71 to threonine at position 73, the amino acid sequence corresponding to from asparagine at position 164 to threonine at position 166, and the amino acid sequence corresponding to from asparagine at position 401 to threonine at position 403 in the amino acid sequence of the wild-type hGAA represented by SEQ ID NO: 19.
39. The protein according to 37 or 38, wherein the hGAA further includes a mutation selected from the group consisting of the following (10'-a) to (10'-h) in an amino acid sequence other than the amino acid sequence corresponding to from asparagine at position 71 to threonine at position 73, the amino acid sequence corresponding to from asparagine at position 164 to threonine at position 166, and the amino acid sequence corresponding to from asparagine at position 401 to threonine at position 403 in the amino acid sequence of the wild-type hGAA represented by SEQ ID NO: 19, and
   the mutation newly generates no consensus sequence to which an N-linked sugar chain binds:
   (10'-a) substitution of 1 to 10 amino acids with another amino acid or other amino acids;
   (10'-b) deletion of 1 to 10 amino acids;
   (10'-c) a combination of the substitution of (10'-a) and the deletion of (10'-b);
   (10'-d) addition of 1 to 10 amino acids into the amino acid sequence or to an N-terminus or a C-terminus of the amino acid sequence;
   (10'-e) a combination of the substitution of (10'-a) and the addition of (10'-d);
   (10'-f) a combination of the deletion of (10'-b) and the addition of (10'-d);
   (10'-g) a combination of the substitution of (10'-a), the deletion of (10'-b), and the addition of (10'-d); and
   (10'-h) exhibition of an identity of 80% or more.
40. The protein according to 36, wherein the hGAA has an amino acid sequence selected from the following (11-a) to (11-e) and has no new consensus sequence to which an N-linked sugar chain binds:
   (11-a) an amino acid sequence exhibiting, in the amino acid sequence except for amino acids at positions 71, 164, and 401, an identity of 80% or more, 85% or more, 90% or more, 95% or more, 98% or more, or 99% or more, to an amino acid sequence of an hGAA mutant represented by SEQ ID NO: 22 in which asparagines at positions 71, 164, and 401 in the amino acid sequence of the wild-type hGAA represented by SEQ ID NO: 19 are each substituted with glutamine;
   (11-b) the amino acid sequence of the hGAA mutant represented by SEQ ID NO: 22 in which asparagines at positions 71, 164, and 401 in the amino acid sequence of the wild-type hGAA represented by SEQ ID NO: 19 are each substituted with glutamine;
   (11-c) an amino acid sequence exhibiting, in the amino acid sequence except for amino acids at positions 73, 166, and 403, an identity of 80% or more, 85% or more, 90% or more, 95% or more, 98% or more, or 99% or more, to an amino acid sequence of an hGAA mutant represented by SEQ ID NO: 23 in which threonines at positions 73, 166, and 403 in the amino acid sequence of the wild-type hGAA represented by SEQ ID NO: 19 are each substituted with alanine;
   (11-d) the amino acid sequence of the hGAA mutant represented by SEQ ID NO: 23 in which threonines at positions 73, 166, and 403 in the amino acid sequence of the wild-type hGAA represented by SEQ ID NO: 19 are each substituted with alanine; and
   (11-e) an amino acid sequence of an hGAA mutant represented by SEQ ID NO: 24 in which asparagines at positions 71, 164, and 401 are each substituted with glutamine and threonines at positions 73, 166, and 403 are each substituted with alanine in the amino acid sequence of the wild-type hGAA represented by SEQ ID NO: 19.
41. The protein according to any one of 36 to 40, having an enzyme activity of 20% or more as compared with the wild-type hGAA.
42. The protein according to any one of 36 to 40, having an enzyme activity of 50% or more as compared with the wild-type hGAA.
43. One or more nucleic acid molecules including a gene encoding the protein described in any one of 1 to 42.
44. One or more expression vectors including the nucleic acid molecule described in 43.
45. One or more mammalian cells transformed with the expression vector described in 44.
46. A method for producing a protein, including:
   culturing the mammalian cell described in 45 in a serum-free medium.
47. A fusion protein of the protein described in any one of 1 to 42 and a ligand having affinity for a protein present on a vascular endothelial cell.
48. The fusion protein according to 47, wherein the protein present on the vascular endothelial cell is selected from the group consisting of an insulin receptor, a transferrin receptor, a leptin receptor, a lipoprotein receptor, and an IGF receptor.
49. The fusion protein according to 47 or 48, wherein the ligand having affinity for the protein present on the vascular endothelial cell is an antibody.
50. The fusion protein according to 49 wherein the antibody is an antibody against one selected from the group consisting of an insulin receptor, a transferrin receptor, a leptin receptor, a lipoprotein receptor, and an IGF receptor.
51. The fusion protein according to 49 or 50, wherein the antibody is any one of a Fab antibody, an F(ab')₂ antibody, an F(ab') antibody, a single domain antibody, a single chain antibody, or an Fc antibody.
52. The fusion protein according to any one of 49 to 51, wherein the protein described in any one of 1 to 42 binds to either a C-terminus side or an N-terminus side of a light chain of the antibody directly or via a linker.
53. The fusion protein according to any one of 49 to 51, wherein the protein described in any one of 1 to 42 binds to either a C-terminus side or an N-terminus side of a heavy chain of the antibody directly or via a linker.
54. The fusion protein according to any one of 49 to 53, wherein the protein described in any one of 1 to 42 binds to either a C-terminus side or an N-terminus side of the light chain or either the C-terminus side or the N-terminus side of the heavy chain of the antibody via a linker.
55. The fusion protein according to 54, wherein the linker contains from 1 to 50 amino acid residues.
56. The fusion protein according to 55, wherein the linker contains an amino acid sequence selected from the group consisting of one glycine, one serine, an amino acid sequence Gly-Ser, an amino acid sequence Ser-Ser, an amino acid sequence Gly-Gly-Ser, an amino acid sequence Gly-Gly-Gly, an amino acid sequence of SEQ ID NO: 25, an amino acid sequence of SEQ ID NO: 26, an amino acid sequence of SEQ ID NO: 27, and an amino acid sequence containing successively from 1 to 10 of these amino acid sequences.
57. The fusion protein of any one of 47 to 56, wherein the ligand is selected from the group consisting of insulin, transferrin, leptin, lipoprotein, **IGF-I,** and IGF-II.
58. The fusion protein according to any one of 47 to 57, wherein the cell is a muscle cell or a cerebrovascular endothelial cell.
59. One or more nucleic acid molecules including a gene encoding the fusion protein described in any one of 47 to 58.
60. One or more expression vectors including the nucleic acid molecule described in 59.
61. One or more mammalian cells transformed with the expression vector described in 60.
62. A method for producing a fusion protein, the method including:
   culturing the mammalian cell described in 61 in a serum-free medium.
63. A method for preventing a decrease in the number of receptors on a cerebrovascular endothelial cell, the method including:
   administering the fusion protein described in any one of 47 to 58 instead of a first fusion protein of a protein to which an N-linked sugar chain containing a mannose-6-phosphate (M6P) binds and an antibody against a receptor on the cerebrovascular endothelial cell, the decrease occurring when the first fusion protein is administered in vivo.
64. A pharmaceutical composition containing the fusion protein described in any one of 47 to 58 as an active ingredient.

### Advantageous Effects of Invention

In a case where a fusion protein of a ligand having affinity for a protein present on a cell (excluding an M6P receptor, hereinafter referred to as a "target protein") and a glycoprotein containing an M6P is administered in vivo, the target protein tends to decrease after the administration. According to one embodiment of the present invention, a decrease in target protein can be suppressed when the fusion protein is administered in vivo by using an M6P low-modified protein as the fusion protein. The decrease in target protein may result in a side effect. For example, in a case where the target protein is an insulin receptor, a decrease in the number of the insulin receptors may cause a decrease in insulin sensitivity, and in a case where the target protein is a transferrin receptor, a decrease in the number of the transferrin receptors may cause inhibition of iron uptake into a cell. When the M6P low-modified protein is used, it is possible to reduce the extent of these phenomena that may be caused by administration of the original fusion protein.

### Brief Description of Drawings

FIG. 1 is a schematic diagram illustrating a structure of a pCI MCS-modified vector (plasmid).
FIG. 2 is a schematic diagram illustrating a structure of a Dual (+)pCI-neo vector (plasmid).
FIG. 3 is a schematic diagram illustrating a structure of a Dual (+)pCI-neo (Fab-IDS (WT)) vector (plasmid).
FIG. 4 is a schematic diagram illustrating a structure of a Dual (+)pCI-neo (Fab-IDS (m1)) vector (plasmid).
FIG. 5 is a schematic diagram illustrating a structure of a Dual (+)pCI-neo (Fab-IDS (m2)) vector (plasmid).
FIG. 6 is a diagram showing measurement results of an expression level of hTfR on a cell membrane when each Fab-IDS is allowed to act on a cell (Example 8). The horizontal axis represents a fluorescence intensity (free unit), and the vertical axis represents the number of cells for each fluorescence intensity. Blank, Fab-IDS (WT), Fab-IDS (m1), and Fab-IDS (m2) indicate measurement results when a buffer solution, Fab-IDS (WT), Fab-IDS (m1), and Fab-IDS (m2) are allowed to act on cells, respectively.

### Description of Embodiments

In protein biosynthesis, a protein translated on the basis of mRNA information may have a functional group such as acetate, phosphate, lipid, or carbohydrate binding to a specific amino acid side chain of the protein, which is referred to as post-translational modification. The post-translational modification includes glycosylation. As the sugar chain added in the glycosylation, there exist an N-linked sugar chain binding to the side chain of an asparagine residue and an O-linked sugar chain binding to the side chain of a serine or threonine residue. Examples of the types of sugars to be added include monosaccharides, disaccharides, and polysaccharides. Examples of the monosaccharides include glucose, mannose, galactose, N-acetyl-D-glucosamine, N-acetyl-D-galactosamine, xylose, sialic acid, glucuronic acid, iduronic acid, and fucose, examples of the disaccharides include maltose, trehalose, and lactose, and examples of the polysaccharides include amylose, glycogen, cellulose, and chitin. The sugars constituting these sugar chains may be chemically modified in vivo by phosphorylation, methylation, acetylation, or the like. A mannose-6-phosphate (M6P) is one of such chemically modified saccharides.

One embodiment of the present invention relates to a glycoprotein having an N-linked sugar chain in which the number of mannose-6-phosphates contained in one molecule is reduced. Another embodiment of the present invention relates to a glycoprotein in which the number of N-linked sugar chains contained in one molecule is reduced.

As used herein, "adding a mutation to an amino acid sequence of a protein" refers to substitution, deletion, and/or addition of one or more amino acid residues to the amino acid sequence of the protein of wild type (as used herein, "addition" of an amino acid residue means addition of a residue to a terminus or internal portion of the sequence), and a protein to which such a mutation is added is referred to as a "mutant-type protein".

In a case of substituting an amino acid residue or amino acid residues with another amino acid residue or amino acid residues, the number of amino acid residues to be substituted is 1 to 10, 1 to 5, or 1 to 3, for example, 1 or 2. In a case of deleting an amino acid residue or amino acid residues, the number of amino acid residues to be deleted is 1 to 10, 1 to 5, or 1 to 3, for example, 1 or 2. In a case of deleting an amino acid residue or amino acid residues, the amino acid residue or amino acid residues at the N-terminus may be deleted. In this case, the number of amino acid residues to be deleted is 1 to 10, 1 to 5, or 1 to 3, for example, 1 or 2. In a case of deleting an amino acid residue or amino acid residues, the amino acid residue or amino acid residues at the C-terminus may be deleted. In this case, the number of amino acid residues to be deleted is 1 to 10, 1 to 5, or 1 to 3, for example, 1 or 2. Furthermore, the substitution and deletion of an amino acid residue or amino acid residues may be combined.

In a case where an amino acid residue or amino acid residues are added to an amino acid sequence of a protein, one or more amino acid residues are added into the amino acid sequence of the protein or to the N-terminus side or the C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined.

Examples of mutant-type proteins into which at least two types of the mutations among these three types of mutations, i.e., substitution, deletion, and addition of an amino acid or amino acids, are introduced in combination include, but are not limited to, the following (i) to (iv):
(i) a protein having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence of the protein (except for an amino acid sequence completely identical to that of the wild-type protein);
(ii) a protein having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence of the protein (except for the amino acid sequence completely identical to that of the wild-type protein);
(iii) a protein having an amino acid sequence obtained by performing deletion of 0 to 3 amino acid residues, substitution of 0 to 3 amino acid residues with another amino acid residues or other amino acid residues, and further addition of 0 to 3 amino acid residues, with respect to the amino acid sequence of the protein (except for the amino acid sequence completely identical to that of the wild-type protein); and
(iv) a protein having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence of the protein (except for the amino acid sequence completely identical to that of the wild-type protein).

The original protein also includes the above-described wild-type or mutant-type protein in which an amino acid constituting the protein is modified with a sugar chain. The original protein also includes the above-described wild-type or mutant-type protein in which amino acids constituting the protein are modified with phosphoric acid. The original protein also includes the above-described wild-type or mutant-type protein in which amino acids constituting the protein are modified with a substance other than a sugar chain and phosphoric acid. The original protein also includes the above-described wild-type or mutant-type protein in which the side chain of an amino acid constituting the protein is converted by a substitution reaction or the like. Such conversion includes, but is not limited to, conversion of a cysteine residue to formylglycine.

That is, a protein modified with a sugar chain is included in a protein having an amino acid sequence before the modification. A protein modified with phosphoric acid is included in a protein having the original amino acid sequence before the modification with phosphoric acid. A protein modified with a moiety other than a sugar chain and phosphoric acid is also included in the protein having the original amino acid sequence before the modification. A protein in which the side chain of an amino acid constituting the protein is converted by a substitution reaction or the like is also included in the protein having the original amino acid sequence before the conversion. Such conversion includes, but is not limited to, conversion of a cysteine residue to formylglycine.

**In** the present invention, the term "mutant-type protein" refers to a protein in which one or more amino acid residues are substituted, deleted, and/or added with respect to the amino acid sequence of a normal wild-type protein (as used herein, "addition" of an amino acid residue means addition of the residue to a terminus or an internal portion of the sequence), and which does not completely lose the function as the original wild-type protein. However, when a "protein" is simply referred to in the present specification, a mutant-type protein is also included in the original protein unless it is distinguished by being specifically described as a wild-type protein or the like. Preferred mutant-type proteins in the present invention include those having an amino acid sequence in which one or more amino acid residues are substituted with another amino acid residue or other amino acid residues, deleted, or added, with respect to the amino acid sequence of a wild-type protein. **In** a case of substituting one or more amino acid residues in the amino acid sequence with another amino acid or other amino acids, the number of amino acid residues to be substituted is 1 to 10, 1 to 5, or 1 to 3, for example, 1 or 2. **In** a case of deleting an amino acid residue or amino acid residues, the number of amino acid residues to be deleted is 1 to 10, 1 to 5, or 1 to 3, for example, 1 or 2. **In** addition, the mutant-type protein may be a protein in which the substitution and the deletion of an amino acid residue or amino acid residues are combined.

**In** a case of adding an amino acid residue or amino acid residues to the amino acid sequence of a wild-type protein, one or more amino acid residues are added into the amino acid sequence of the wild-type protein or to the N-terminus side or the C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined. That is, the mutant-type protein may be a protein in which at least two types of mutations among three types of mutations, i.e., substitution, deletion, and addition of an amino acid or amino acids, are introduced in combination, with respect to the amino acid sequence of the wild-type protein.

Examples of the mutant-type protein in which at least two types of mutations among these three types of mutations, i.e., substitution, deletion, and addition of an amino acid or amino acids, are introduced in combination include, but are not limited to, the following (i) to (iv):
(i) a protein having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence of a wild-type protein (except for an amino acid sequence completely identical to that of the wild-type protein);
(ii) a protein having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence of the wild-type protein (except for the amino acid sequence completely identical to that of the wild-type protein);
(iii) a protein having an amino acid sequence obtained by performing deletion of 0 to 3 amino acid residues, substitution of 0 to 3 amino acid residues with another amino acid residue or other amino acid residues, and further addition of 0 to 3 amino acid residues, with respect to the amino acid sequence of the wild-type protein (except for the amino acid sequence completely identical to that of the wild-type protein); and
(iv) a protein having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence of the wild-type protein (except for the amino acid sequence completely identical to that of the wild-type protein).

The position and form (deletion, substitution, and addition) of each mutation in the mutant-type protein as compared with those of the normal wild-type protein can be easily confirmed by the alignment of the amino acid sequences of both the proteins.

The amino acid sequence of the mutant-type protein preferably exhibits an identity of 80% or more, an identity of 85% or more, an identity of 90% or more, or an identity of 95% or more, and exhibits, for example, an identity of 98% or more, or 99% or more, to the amino acid sequence of the wild-type protein.

The identity between the amino acid sequence of the wild-type protein and the amino acid sequence of the mutant-type protein can be easily calculated using a known homology calculation algorithm. Examples of such an algorithm include BLAST (Altschul SF. J Mol. Biol. 215. 403-10, (1990)), the similarity search method of Pearson and Lipman (Proc. Natl. Acad. Sci. USA. 85. 2444 (1988)), and the local homology algorithm of Smith and Waterman (Adv. Appl. Math. 2. 482-9 (1981)).

The substitution of an amino acid in the amino acid sequence of the wild-type protein or mutant-type protein with another amino acid occurs, for example, in a family of amino acids that are related in the side chains and chemical properties the amino acid. Such a substitution in a family of amino acids is expected not to result in a significant change in the function of the original protein (i.e., to be a conservative amino acid substitution). Examples of such a family of amino acids include the following (1) to (12):
(1) aspartic acid and glutamic acid, which are acidic amino acids;
(2) histidine, lysine, and arginine, which are basic amino acids;
(3) phenylalanine, tyrosine, tryptophan, which are aromatic amino acids;
(4) serine and threonine, which are amino acids with a hydroxyl group (hydroxyamino acids);
(5) methionine, alanine, valine, leucine, and isoleucine, which are hydrophobic amino acids;
(6) cysteine, serine, threonine, asparagine, and glutamine, which are neutral hydrophilic amino acids;
(7) glycine and proline, which are amino acids affecting the orientation of peptide chains;
(8) asparagine and glutamine, which are amide amino acids (polar amino acids);
(9) alanine, leucine, isoleucine, and valine, which are aliphatic amino acids;
(10) alanine, glycine, serine, and threonine, which are amino acids with a small side chain;
(11) alanine and glycine, which are amino acids with a particularly small side chain; and
(12) valine, leucine, and isoleucine, which are amino acids having a branched chain.

In the post-translational modification of a protein, N-acetylglucosamine is β-bonded to an amide moiety which is the side chain of an asparagine residue contained in the amino acid sequence of the protein, and a sugar such as mannose, galactose, or sialic acid may be extended therefrom to form a sugar chain. Such a sugar chain added to an asparagine residue in the amino acid sequence of a protein is referred to as an N-linked sugar chain. The N-linked sugar chain is not added to all asparagine residues in the amino acid sequence of a protein. The binding site of the N-linked sugar chain is limited to an asparagine residue contained in a sequence (consensus sequence) consisting of three consecutive amino acid residues in the amino acid sequence of a protein, in which an asparagine residue, an amino acid residue other than proline, and a threonine residue or a serine residue are arranged in this order from the N-terminus side, that is, a sequence represented by [Asn-Xaa-Thr/Ser] (where Xaa is an amino acid other than Pro). However, the N-linked sugar chain is not always added to asparagine residues contained in all consensus sequences in the amino acid sequence of a protein, but the N-linked sugar chain may be added to asparagine residues contained in all consensus sequences in the amino acid sequence of a protein.

The term "consensus sequence" as used herein refers to only a sequence consisting of three consecutive amino acid residues represented by [Asn-Xaa-Thr/Ser] (where Xaa is an amino acid other than Pro), which is an addition site of the N-linked sugar chain in the amino acid sequence of a protein, although the term "consensus sequence" originally refers to a sequence common to base sequences of genes and amino acid sequences of proteins among a plurality of different organisms and having an identical or similar action. All sequences represented by [Asn-Xaa-Thr/Ser] (where Xaa is an amino acid other than Pro) present in the amino acid sequence of a protein are referred to as a consensus sequence, and whether or not a sugar chain is to be actually added and whether or not it is added are not taken into consideration. The consensus sequence as used herein is also referred to as "Asn-Xaa-Yaa" (where Xaa represents an amino acid other than proline and Yaa represents threonine or serine).

As used herein, the term "modifying a consensus sequence" refers to adding a mutation to three amino acid residues constituting a sequence represented by [Asn-Xaa-Thr/Ser] (where Xaa is an amino acid other than Pro) in the amino acid sequence of a protein to obtain a sequence containing no consensus sequence. Examples of the mutation for modifying a consensus sequence include the following (a) to (e). That is, in the consensus sequence,
(a) deletion of Asn or substitution of Asn with an amino acid other than Asn,
(b) addition of an amino acid other than Asn between Asn and Xaa,
(c) deletion of Xaa or substitution of Xaa with Pro,
(d) addition of an amino acid other than Thr and Ser between Xaa and Thr/Ser, or
(e) deletion of Thr/Ser or substitution of Thr/Ser with an amino acid other than Thr and Ser.

When a consensus sequence is modified, in a case where the amino acid residue represented by Xaa (amino acid residue other than Pro) in the consensus sequence to be modified is a threonine residue or a serine residue, that is, in a case where the consensus sequence is a sequence represented by [Asn-Thr/Ser-Thr/Ser], for example, when an amino acid other than Asn and Pro is added between Asn and Xaa in the mutation of (b), a consensus sequence exists after the mutation. Thus, in a case where the amino acid residue (amino acid residue other than Pro) represented by Xaa in the consensus sequence is a threonine residue or a serine residue, examples of the mutation for obtaining a sequence including no consensus sequence by modifying the consensus sequence include the following (f) to (j). That is, in the consensus sequence,
(f) deletion of Asn or substitution of Asn with an amino acid other than Asn,
(g) addition of Pro between Asn and Xaa,
(h) deletion of Xaa or substitution of Xaa with Pro,
(i) addition of an amino acid other than Thr and Ser between Xaa and Thr/Ser, or
(j) deletion of Thr/Ser or substitution of Thr/Ser with an amino acid other than Thr and Ser.

When the mutations (a) to (j) are introduced into the consensus sequence, a new consensus sequence may be generated in relation to the sequence on the N-terminus side and/or the C-terminus side of the consensus sequence. The mutation that newly generates such a new consensus sequence, that is, an amino acid sequence represented by Asn-Xaa-Yaa (where Xaa represents an amino acid other than proline, and Yaa represents threonine or serine) is excluded from the mutations (a) to (j) described above.

For example, apart from the above case, when the consensus sequence is modified, in a case where an amino acid residue following the C-terminus side of the consensus sequence to be modified is a threonine residue or a serine residue, that is, the sequence consisting of a total of four amino acid residues, i.e., the consensus sequence and the amino acid residues consecutive to the C-terminus side of the consensus sequence, is a sequence represented by [Asn-Xaa-Thr/Ser-Thr/Ser] (where Xaa is an amino acid other than Pro), a consensus sequence exists even after the mutation, for example, when Asn is added between Xaa and Thr/Ser in the mutation of (d) or when Thr/Ser is deleted in the mutation of (e). Accordingly, in a case where the amino acid residue following the C-terminus side of the consensus sequence is a threonine residue or a serine residue, examples of the mutation for obtaining a sequence including no consensus sequence by modifying the consensus sequence include the following mutations (k) to (o). That is, in the consensus sequence,
(k) deletion of Asn or substitution of Asn with an amino acid other than Asn,
(l) addition of an amino acid other than Asn between Asn and Xaa,
(m) substitution of Xaa with Pro,
(n) addition of an amino acid other than Thr, Ser, and Asn between Xaa and Thr/Ser, or
(o) substitution of Thr/Ser with an amino acid other than Thr and Ser.

Further, for example, when the consensus sequence is modified, in a case where the amino acid residue represented by Xaa (amino acid residue other than Pro) in the consensus sequence to be modified is a threonine residue or a serine residue and the amino acid residue following the C-terminus side of the consensus sequence is a threonine residue or a serine residue, that is, in a case where the sequence consisting of a total of four amino acid residues of the consensus sequence and the amino acid residues consecutive to the C-terminus side thereof is a sequence represented by [Asn-Thr/Ser-Thr/Ser-Thr/Ser], for example, the consensus sequence is present even after the mutation when an amino acid other than Asn and Pro is added between Asn and Xaa in the mutation of (b), when Asn is added between Xaa and Thr/Ser in the mutation of (d), or when Thr/Ser is deleted in the mutation of (e), a consensus residue exists even after the mutation. Accordingly, in a case where the amino acid residue represented by Xaa (amino acid residue other than Pro) in the consensus sequence is a threonine residue or a serine residue and the amino acid residue following the C-terminus side of the consensus sequence is a threonine residue or a serine residue, examples of the mutation for obtaining a sequence including no consensus sequence by modifying the consensus sequence include the following (p) to (t). That is, in the consensus sequence,
(p) deletion of Asn or substitution of Asn with an amino acid other than Asn,
(q) addition of Pro between Asn and Xaa,
(r) substitution of Xaa with Pro,
(s) addition of an amino acid other than Thr, Ser, and Asn between Xaa and Thr/Ser, or
(t) substitution of Thr/Ser with an amino acid other than Thr and Ser.

Further, for example, when the consensus sequence is modified, in a case where the amino acid residue represented by Xaa (amino acid residue other than Pro) in the consensus sequence to be modified is a threonine residue or a serine residue and the amino acid residue following the N-terminus side of the consensus sequence is an asparagine residue, that is, in a case where the sequence consisting of a total of four amino acid residues of the consensus sequence and one amino acid residue consecutive to the C-terminus side thereof is a sequence represented by [Asn-Asn-Xaa-Thr/Ser], deletion of Asn constituting the consensus sequence results in a new consensus sequence and is thus excluded.

The modification of the consensus sequence exemplified above can alternatively be expressed as follows. That is, a protein having a modified consensus sequence is a protein including at least one mutation selected from the group consisting of the following (1-a) to (1-c) with respect to an amino acid sequence represented by Asn-Xaa-Yaa (where Xaa represents an amino acid other than proline, and Yaa represents threonine or serine) containing asparagine to which an N-linked sugar chain binds in the amino acid sequence of the wild-type protein, wherein the mutation newly generates no consensus sequence to which an N-linked sugar chain binds:
(1-a) deletion of Asn or substitution of Asn with an amino acid other than asparagine;
(1-b) substitution of the amino acid represented by Xaa with proline; and
(1-c) substitution of the amino acid represented by Yaa with an amino acid other than threonine and serine.

In other words, a protein having a modified consensus sequence can be said to be a protein in which at least one of amino acid sequences represented by Asn-Xaa-Yaa (where Xaa represents an amino acid other than proline, and Yaa represents threonine or serine) contained in the amino acid sequence of a wild-type protein in which N-linked sugar chains bind to Asn is mutated in such a manner that at least one of the N-linked sugar chains is deleted.

When there is a plurality of consensus sequences in the amino acid sequence of a protein, "modifying a consensus sequence" means that it is sufficient that one of the plurality of consensus sequences is modified, and not all the other consensus sequences need to be modified. However, a plurality of consensus sequences may be modified, and all the consensus sequences may be modified.

In a case where a consensus sequence to which an N-linked sugar chain is added is modified in the amino acid sequence of a protein, the amino acid sequence becomes a sequence that contains no consensus sequence, and thus no N-linked sugar chain is added to the site. Accordingly, in a case where a consensus sequence to which an N-linked sugar chain containing an M6P is added is modified in the amino acid sequence of a protein, the sequence contains no consensus sequence, and thus no N-linked sugar chain containing an M6P is added to the site. A protein to which an N-linked sugar chain containing an M6P is added may be taken up into a cell via an M6P receptor present on the surface of the cell. In such a protein, when the consensus sequence to which the N-linked sugar chain containing an M6P is added is modified, the affinity between the protein and the M6P receptor is reduced or eliminated, and uptake into the cell hardly occurs or does not occur at all. Accordingly, in a case where a protein to which an N-linked sugar chain containing an M6P is added by post-translational modification is artificially produced and administered in vivo for the purpose of exhibiting physiological activity in a living body, it is usually essential to produce the protein as a protein to which an N-linked sugar chain containing an M6P is added while maintaining the consensus sequence in the protein to ensure uptake of the protein into a cell. This is true even when a protein is produced as a mutant-type protein having an amino acid sequence obtained by adding a mutation to a wild-type amino acid sequence for some reason or as a fusion protein in which a protein is fused with another protein having another function, and it is common technical knowledge to produce the protein as a protein to which an N-linked sugar chain containing an M6P is added while maintaining the consensus sequence in the protein.

In a case where a protein is administered as it is into blood or the like for the purpose of exhibiting physiological activity in vivo, the protein is less likely to migrate from capillaries to the brain due to the presence of the blood-brain barrier (BBB), which is a mechanism for limiting exchange of substances such as polymers between blood and brain tissue fluid via the endothelium of capillaries in the brain. Thus, the effects may be limited on disorder to cells and tissues of the nervous system including the brain and spinal cord. Accordingly, a protein is made into a conjugate or fusion protein obtained by binding the protein to a ligand having affinity for a receptor (excluding an M6P receptor) on a cerebrovascular endothelial cell, for example, an antibody against the receptor, whereby the protein can pass through the blood-brain barrier and exhibit the function in the brain. As with a transferrin receptor and an insulin receptor, most receptors on cerebrovascular endothelial cells are present on various cells other than the cerebrovascular endothelial cells. Some of these cells have M6P receptors. Thus, in a case where the protein is a glycoprotein to which an N-linked sugar chain containing an M6P is added, a ligand moiety of the fusion protein has binding affinity for a receptor on a cell, and a glycoprotein moiety thereof has binding affinity for an M6P receptor on the same cell. In such a case, the fusion protein also binds to the M6P receptor while the ligand remains the binding to the receptor on the cell, and can be taken up into the cell together with these receptors. Then, the receptor binding to the fusion protein via the ligand taken up into the cell together with the fusion protein is transported to the lysosome together with the fusion protein and decomposed, and is not recycled. This results in a decrease in the number of receptors on the cell. This attenuates transduction of signals to be transmitted into the cell through binding between the receptor on the cell and a substance to which the receptor on the cell should originally bind, for example, insulin when the receptor on the cell is an insulin receptor, or transferrin when the receptor on the cell is a transferrin receptor. The attenuation of signal transduction through the receptor on the cell may cause resistance to the ligand for the receptor. For example, a decrease in the number of insulin receptors results in a decrease in insulin sensitivity, and a decrease in the number of transferrin receptors results in inhibition of iron uptake into a cell.

In addition, some types of proteins are less likely to be taken up into a muscle tissue when they are administered as they are into blood or the like for the purpose of exhibiting physiological activity in vivo. When such a protein is bound to a ligand having affinity for a receptor on a muscle cell, for example, an antibody against the receptor to form a conjugate or a fusion protein, the protein can efficiently exert its function in the muscle tissue. Preferred examples of such a receptor include, but are not limited to, the insulin receptor and the transferrin receptor. In a case where the protein is a glycoprotein to which the N-linked sugar chain containing an M6P is added, the same problem as that of the above-described fusion protein of the ligand having affinity for the receptor on the cerebrovascular endothelial cell and the glycoprotein to which the N-linked sugar chain containing an M6P is added may occur. Here, the muscle cell may constitute a skeletal muscle or a smooth muscle.

Furthermore, in a case where a certain type of protein is caused to exhibit physiological activity in a specific cell, the protein can be caused to exhibit the function efficiently in the specific cell by forming a conjugate or a fusion protein in which the protein is bound to a ligand having affinity for the protein present on the cell. In a case where the protein is a glycoprotein to which the N-linked sugar chain containing an M6P is added, the same problem as that of the above-described fusion protein of the ligand having affinity for the receptor on the cerebrovascular endothelial cell and the glycoprotein to which the N-linked sugar chain containing an M6P is added may occur.

Further, in a case where a certain type of protein is caused to exhibit physiological activity without particularly limiting the tissue, the protein can be caused to exhibit the function efficiently in a wide variety of tissues by forming a conjugate or a fusion protein in which the protein is bound to a ligand having affinity for a protein universally present on a cell. In a case where the protein is a glycoprotein to which the N-linked sugar chain containing an M6P is added, the same problem as that of the above-described fusion protein of the ligand having affinity for the receptor on the cerebrovascular endothelial cell and the glycoprotein to which the N-linked sugar chain containing an M6P is added may occur.

To avoid such a symptom, in one embodiment of the present invention, a glycoprotein to which N-linked sugar chains containing one or more mannose-6-phosphates (M6Ps) bind in the wild type is made to be a protein in which at least one N-linked sugar chain containing M6Ps originally added to the protein is deleted by adding a mutation to the amino acid sequence of the protein. Further, in an embodiment, a glycoprotein to which N-linked sugar chains containing one or more mannose-6-phosphates (M6Ps) bind in the wild type is made to be a protein in which at least one M6P or at least one N-linked sugar chain is deleted by chemically treating the protein. The wording "chemically treating" as used herein means that a chemical, a catalyst, an enzyme, or the like is caused to act on a glycoprotein to cause a decomposition reaction or a substitution reaction, thereby cleaving an M6P or an N-linked sugar chain from the glycoprotein. At this time, in a case where an enzyme is used, for example, peptide N-glycanase, endo-β-N-acetylglucosaminidase, or endo-β-mannosidase, which is known as an endo-type enzyme that acts around a site where an N-linked sugar chain binds to an asparagine residue, can be suitably used.

A protein in which at least one N-linked sugar chain containing an M6P is deleted has reduced or lost affinity for the M6P receptor. Thus, in a case where the protein is bound to a ligand having affinity for a protein present on a cell (excluding the M6P receptor, referred to herein as a "target protein"), even when the protein is administered in vivo, it is taken up into a cell mainly via only the target protein because of reduced or lost affinity for the M6P receptor. At this time, the target protein taken up into the cell together with the fusion protein is not transported to a lysosome and can be prevented from being decomposed in the lysosome.

Note that in one embodiment of the present invention, a protein obtained by deleting at least one M6P of a glycoprotein having an N-linked sugar chain including M6Ps in the wild type is referred to as an "M6P low-modified protein". A conjugate and a fusion protein in which such an M6P low-modified protein is bound to a ligand are also included in the M6P low-modified protein. In a case where the glycoprotein having an N-linked sugar chain containing M6Ps in the wild-type is iduronate-2-sulfatase (IDS), a protein obtained by deleting at least one M6P is referred to as an "M6P low-modified IDS". The same is true for other glycoproteins. There is no particular limitation on the species of an animal from which the wild-type protein that is the origin of the M6P low-modified protein is derived, but a mammal is preferred, and a human is more preferred.

In the present invention, there is no particular limitation on the protein in which an M6P or an N-linked sugar chain is deleted or whose amino acid sequence is mutated, but the protein is a protein which can exhibit physiological activity in vivo, particularly a protein which should be allowed to reach the inside of the brain to exert the function but cannot be expected to exert the function in the brain by intravenous administration because it cannot pass through the blood-brain barrier as it is, and a glycoprotein to which N-linked sugar chains containing one or more mannose-6-phosphates (M6P) binds in the wild type. Examples of such a protein include, but are not limited to, a lysosomal enzyme. Examples of the lysosomal enzyme include iduronate-2-sulfatase, α-L-iduronidase, glucocerebrosidase, β-galactosidase, GM2 activating proteins, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl-CoA α-glucosaminide N-acetyltransferase, N-acetylglucosamine-6-sulfatase, acid ceramidase, amylo-1,6-glucosidase, sialidase, palmitoylprotein thioesterase-1, tripeptidylpeptidase-1, hyaluronidase-1, acid α-glucosidase, CLN1, and CLN2.

The lysosomal enzyme to which a ligand having affinity for a protein present on a cerebrovascular endothelial cell is bound can be used as a therapeutic agent for central nervous system disorder in lysosomal diseases. An antibody against a receptor on a cerebrovascular endothelial cell is one of the ligands. The lysosomal enzymes with which such a ligand is fused can be used as follows: iduronate-2-sulfatase (IDS) as a therapeutic agent for central nervous system disorder in Hunter syndrome;
α-L-iduronidase (IDUA) as a therapeutic agent for central nervous system disorder in Hurler's syndrome or Hurler-Scheie syndrome;
glucocerebrosidase (GBA) as a therapeutic agent for central nervous system disorder in Gaucher disease;
β-galactosidase as a therapeutic agent for central nervous system disorder in GM1-gangliosidosis types 1 to 3;
GM2 activating proteins as a therapeutic agent for central nervous system disorder in GM2-gangliosidosis AB variant;
β-hexosaminidase A as a therapeutic agent for central nervous system disorder in Sandhoff disease and Tay-Sachs disease;
β-hexosaminidase B as a therapeutic agent for central nervous system disorder in Sandhoff disease;
N-acetylglucosamine-1-phosphotransferase as a therapeutic agent for central nervous system disorder in I-cell disease;
α-mannosidase (LAMAN) as a therapeutic agent for central nervous system disorder in α-mannosidosis;
β-mannosidase as a therapeutic agent for central nervous system disorder in β-mannosidosis;
galactosylceramidase (GALC) as a therapeutic agent for central nervous system disorder in Krabbe disease;
saposin C as a therapeutic agent for central nervous system disorder in Gaucher's disease-like storage disease;
arylsulfatase A (ARSA) as a therapeutic agent for central nervous system disorder in metachromatic white matter degeneration (metachromatic leukodystrophy);
α-L-fucosidase (FUCA1) as a therapeutic agent for central nervous system disorder in fucosidosis;
aspartylglucosaminidase as a therapeutic agent for central nervous system disorder in aspartylglucosaminuria;
α-N-acetylgalactosaminidase as a therapeutic agent for central nervous system disorder in Schindler disease and Kawasaki disease;
acid sphingomyelinase (ASM) as a therapeutic agent for central nervous system disorder in Niemann-Pick disease;
α-galactosidase A as a therapeutic agent for central nervous system disorder in Fabry disease;
β-glucuronidase (GUSB) as a therapeutic agent for central nervous system disorder in Sly syndrome;
heparan N-sulfatase (SGSH), α-N-acetylglucosaminidase (NAGLU), acetyl-CoA α-glucosaminide N-acetyltransferase, and N-acetylglucosamine-6-sulfatase as therapeutic agents for central nervous system disorder in Sanfilippo syndrome;
acid ceramidase (AC) as a therapeutic agent for central nervous system disorder in Farber disease;
amylo-1,6-glucosidase as a therapeutic agent for central nervous system disorder in Cori's disease (Forbes-Cori's disease);
sialidase as a therapeutic agent for central nervous system disorder in sialidase deficiency;
palmitoylprotein thioesterase-1 (PPT-1) as a therapeutic agent for central nervous system disorder in neuronal ceroid lipofuscinosis or Santavuori-Haltia disease;
tripeptidylpeptidase-1 (TPP-1) as a therapeutic agent for central nervous system disorder in neuronal ceroid lipofuscinosis or Jansky-Bielschowsky disease;
hyaluronidase-1 as a therapeutic agent for central nervous system disorder in hyaluronidase deficiency;
acid α-glucosidase (GAA) as a therapeutic agent for central nervous system disorder in Pompe disease; and
CLN1 and CLN2 as therapeutic agents for central nervous system disorder in Batten disease.

Wild-type human iduronate-2-sulfatase (hIDS) is one of lysosomal enzymes and composed of 525 amino acid residues represented by SEQ ID NO: 1. hIDS has activity of hydrolyzing sulfate ester bonds of heparan sulfate and dermatan sulfate which belong to glycosaminoglycan. In a patient with Hunter syndrome having genetic abnormality in this enzyme, partial decomposition products of heparan sulfate and dermatan sulfate accumulate in tissues such as the liver and the spleen due to metabolic abnormality of heparan sulfate and dermatan sulfate, resulting in symptoms such as skeletal abnormality. Hunter syndrome is also referred to as mucopolysaccharidosis type II (MPS II). In addition, a patient with Hunter syndrome may have central nervous system disorder. hIDS bound to a ligand having affinity for a protein on a cerebrovascular endothelial cell can be used as a therapeutic agent for central nervous system disorder associated with Hunter syndrome.

As used herein, the term "human iduronate-2-sulfatase" or "hIDS" encompasses not only the normal wild-type hIDS composed of 525 amino acid residues represented by SEQ ID NO: 1, but also, without particular distinction, a mutant of hIDS corresponding to that in which one or more amino acid residues are substituted, deleted, and/or added with respect to the amino acid sequence represented by SEQ ID NO: 1 (as used herein, "addition" of an amino acid residue means addition of a residue to a terminus or an internal portion of the sequence) as long as the mutant has a function as the normal wild-type hIDS, such as having an enzyme activity capable of decomposing heparan sulfate and dermatan sulfate. The wild-type hIDS is encoded by, for example, a gene having the base sequence represented by SEQ ID NO: 2. In a case of substituting an amino acid residue or amino acid residues with another amino acid residue or amino acid residues, the number of amino acid residues to be substituted is 1 to 10, 1 to 5, or 1 to 3, for example, 1 or 2. In a case of deleting an amino acid residue or amino acid residues, the number of amino acid residues to be deleted is 1 to 10, 1 to 5, or 1 to 3, for example, 1 or 2. In a case of deleting an amino acid residue or amino acid residues, the amino acid residue or amino acid residues at the N-terminus may be deleted. In this case, the number of amino acid residues to be deleted is 1 to 10, 1 to 5, or 1 to 3, for example, 1 or 2. Furthermore, the substitution and deletion of an amino acid residue or amino acid residues may be combined.

In a case of adding an amino acid residue or amino acid residues to the amino acid sequence represented by SEQ ID NO: 1, one or more amino acid residues are added into the amino acid sequence of hIDS or to the N-terminus side or C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined. Normal wild-type hIDS is biosynthesized as a precursor composed of 550 amino acid residues, and a leader peptide composed of 25 amino acid residues from the N-terminus is removed to give hIDS represented by SEQ ID NO: 1. In a case of adding an amino acid to the N-terminus side of hIDS, the amino acid may be derived from the leader peptide. In this case, Gly is added to the N-terminus in a case of adding one amino acid, Leu-Gly in a case of adding two amino acids, and Ala-Leu-Gly in a case of adding three amino acids.

Examples of the mutant of hIDS in which at least two types of mutations among these three types of mutations, i.e., substitution, deletion, and addition of an amino acid or amino acids, are introduced in combination include, but are not limited to, the following (i) to (iv):
(i) a mutant having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence represented by SEQ ID NO: 1;
(ii) a mutant having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence represented by SEQ ID NO: 1;
(iii) a mutant having an amino acid sequence obtained by performing deletion of 0 to 3 amino acid residues, substitution of 0 to 3 amino acid residues with another amino acid residue or other amino acid residues, and further addition of 0 to 3 amino acid residues, with respect to the amino acid sequence represented by SEQ ID NO: 1; and
(iv) a mutant having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence represented by SEQ ID NO: 1.

The above-described wild-type or mutant-type hIDS in which an amino acid constituting the hIDS is modified with a sugar chain is also hIDS. The above-described wild-type or mutant-type hIDS in which an amino acid constituting the hIDS is modified with phosphoric acid is also hIDS. The above-described wild-type or mutant-type hIDS in which an amino acid constituting the hIDS is modified with a moiety other than a sugar chain and phosphoric acid is also hIDS. The above-described wild-type or mutant-type hIDS in which the side chain of an amino acid constituting the hIDS is converted by a substitution reaction or the like is also hIDS. Such conversion includes, but is not limited to, conversion of a cysteine residue to formylglycine.

That is, hIDS modified with a sugar chain is encompassed in hIDS having the amino acid sequence before the modification. hIDS modified with phosphoric acid is encompassed in hIDS having the original amino acid sequence before modification with phosphoric acid. hIDS modified with a moiety other than a sugar chain and phosphoric acid is also encompassed in hIDS having the original amino acid sequence before the modification. Those in which the side chain of an amino acid constituting hIDS is converted by a substitution reaction or the like are also encompassed in hIDS having the original amino acid sequence before the conversion. Such conversion includes, but is not limited to, conversion of a cysteine residue to formylglycine.

In the present invention, the term "human iduronate-2-sulfatase" (hIDS mutant) refers to a mutant in which one or more amino acid residues are substituted, deleted, and/or added with respect to the amino acid sequence of the normal wild-type hIDS (the amino acid sequence represented by SEQ ID NO: 1) (as used herein, "addition" of an amino acid residue means addition of a residue to a terminus or an internal portion of the sequence), and has a function as the normal wild-type hIDS, such as having an enzyme activity capable of decomposing heparan sulfate and dermatan sulfate. In the present invention, the hIDS mutant maintains 10% or more, 20% or more, 30% or more, 40% or more, 50% or more, or 60% or more of the enzyme activity, and preferably maintains 20% or more, or 50% or more of the enzyme activity, as compared with the wild-type hIDS. Preferred hIDS mutants in the present invention include those in which one or more amino acid residues are substituted with another amino acid residue or other amino acid residues, deleted, or added, with respect to the amino acid sequence represented by SEQ ID NO: 1. In a case of substituting an amino acid residue or amino acid residues in the amino acid sequence with another amino acid residue or other amino acid residues, the number of amino acid residues to be substituted is 1 to 10, 1 to 5, or 1 to 3, and for example, 1 or 2. In a case of deleting an amino acid residue or amino acid residues, the number of amino acid residues to be deleted is 1 to 10, 1 to 5, or 1 to 3, for example, 1 or 2. The hIDS mutant may be an hIDS mutant in which the substitution and deletion of an amino acid residue or amino acid residues are combined.

In a case of adding an amino acid residue or amino acid residues to the amino acid sequence represented by SEQ ID NO: 1, one or more amino acid residues are added into the amino acid sequence of hIDS or to the N-terminus side or C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined. That is, the hIDS mutant may be an hIDS mutant in which at least two types of mutations among three types of mutations, i.e., substitution, deletion, and addition of an amino acid or amino acids, are introduced in combination, with respect to the amino acid sequence represented by SEQ ID NO: 1.

Examples of the mutant of hIDS in which at least two types of mutations among these three types of mutations, i.e., substitution, deletion, and addition of an amino acid or amino acids, are introduced in combination include, but are not limited to, the following (i) to (iv):
(i) a mutant having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence represented by SEQ ID NO: 1 (provided that wild-type hIDS is excluded);
(ii) a mutant having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence represented by SEQ ID NO: 1 (provided that wild-type hIDS is excluded);
(iii) a mutant having an amino acid sequence obtained by performing deletion of 0 to 3 amino acid residues, substitution of 0 to 3 amino acid residues with another amino acid residue or other amino acid residues, and further addition of 0 to 3 amino acid residues, with respect to the amino acid sequence represented by SEQ ID NO: 1 (provided that wild-type hIDS is excluded); and
(iv) a mutant having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence represented by SEQ ID NO: 1 (provided that wild-type hIDS is excluded).

The position and form (deletion, substitution, and addition) of each mutation in various hIDS mutants as compared with those of the normal wild-type hIDS can be easily confirmed by the alignment of the amino acid sequences of hIDS of the wild-type and hIDS of the hIDS mutant.

The amino acid sequence of the hIDS mutant preferably exhibits an identity of 80% or more, an identity of 85% or more, an identity of 90% or more, or an identity of 95% or more, and for example, an identity of 98% or more, or an identity of 99% or more, to the amino acid sequence of the normal wild-type hIDS represented by SEQ ID NO: 1.

In one embodiment, the protein of the present invention is a glycoprotein obtained by adding a mutation to the wild-type amino acid sequence of a glycoprotein to which N-linked sugar chains containing one or more mannose-6-phosphates (M6Ps) bind in the wild type to delete at least one N-linked sugar chain binding to the glycoprotein, that is, an M6P low-modified protein. As the glycoprotein to which N-linked sugar chains containing one or more mannose-6-phosphates (M6Ps) bind in the wild type, hIDS can be used, and a mutation is added to the amino acid sequence of the wild-type hIDS to modify the consensus sequence, whereby an M6P low-modified hIDS in which at least one N-linked sugar chain binding to hIDS is deleted can be prepared. In the consensus sequence of the wild-type hIDS represented by SEQ ID NO: 1, the site to which an N-linked sugar chains containing M6P binds is an asparagine residue contained in each of the consensus sequence starting from asparagine at position 221 and the consensus sequence starting from asparagine at position 255. Accordingly, the consensus sequence starting from asparagine at position 221 and/or the consensus sequence starting from asparagine at position 255 in the amino acid sequence of the wild-type hIDS represented by SEQ ID NO: 1 is modified, whereby the M6P low-modified hIDS in which the N-linked sugar chain(s) containing M6Ps binding to hIDS is (are) deleted can be prepared. The M6P low-modified hIDS can be prepared by adding a mutation to not only the wild-type hIDS but also the above-described hIDS mutant.

Preferred embodiments of the M6P low-modified hIDS include the following (I-a) to (I-e):
(I-a) an M6P low-modified hIDS in which asparagine at position 221 in the amino acid sequence of the wild-type hIDS represented by SEQ ID NO: 1 is deleted or substituted with an amino acid other than asparagine;
(I-b) an M6P low-modified hIDS in which an amino acid other than asparagine is added between asparagine at position 221 and isoleucine at position 222 in the amino acid sequence of the wild-type hIDS represented by SEQ ID NO: 1;
(I-c) an M6P low-modified hIDS in which isoleucine at position 222 is deleted or substituted with proline in the amino acid sequence of the wild-type hIDS represented by SEQ ID NO: 1;
(I-d) an M6P low-modified hIDS in which an amino acid other than threonine and serine is added between isoleucine at position 222 and threonine at position 223 in the amino acid sequence of the wild-type hIDS represented by SEQ ID NO: 1; and
(I-e) an M6P low-modified hIDS in which threonine at position 223 is deleted or substituted with an amino acid other than threonine and serine in the amino acid sequence of the wild-type hIDS represented by SEQ ID NO: 1.

The M6P low-modified hIDS shown in the above (I-a) to (I-e) may further contain a mutation selected from the group consisting of the following (I'-a) to (I'-h) in addition to the deleted, added or substituted amino acid(s):
(I'-a) substitution of an amino acid residue or amino acid residues constituting the amino acid sequence of the M6P low-modified hIDS with another amino acid residue or other amino acid residues, in which the number of the substituted amino acid residues is 1 to 10, 1 to 5, or 1 to 3, for example, 1 or 2;
(I'-b) deletion of an amino acid residue or amino acid residues constituting the amino acid sequence of the M6P low-modified hIDS, in which the number of the deleted amino acid residues is 1 to 10, 1 to 5, or 1 to 3, for example, 1 or 2;
(I'-c) a combination of the substitution of (I'-a) and the deletion of (I'-b);
(I'-d) addition of one or more amino acid residues into the amino acid sequence of the M6P low-modified hIDS or to the N-terminus side or the C-terminus side of the amino acid sequence of the M6P low-modified hIDS, in which the number of the added amino acid residues is 1 to 10, 1 to 5, or 1 to 3, for example, 1 or 2;
(I'-e) a combination of the substitution of (I'-a) and the addition of (I'-d);
(I'-f) a combination of the deletion of (I'-b) and the addition of (I'-d);
(I'-g) a combination of the substitution of (I'-a), the deletion of (I'-b), and the addition of (I'-d); and
(I'-h) exhibition of an identity of 80% or more, 85% or more, 90% or more, 95% or more, 98% or more, or 99% or more, to the amino acid sequence of the M6P low-modified hIDS.

Preferred embodiments of the M6P low-modified hIDS include the following (II-a) to (II-e):
(II-a) an M6P low-modified hIDS in which asparagine at position 255 in the amino acid sequence of the wild-type hIDS represented by SEQ ID NO: 1 is deleted or substituted with an amino acid other than asparagine;
(II-b) an M6P low-modified hIDS in which an amino acid other than asparagine is added between asparagine at position 255 and isoleucine at position 256 in the amino acid sequence of the wild-type hIDS represented by SEQ ID NO: 1;
(II-c) an M6P low-modified hIDS in which isoleucine at position 256 is deleted or substituted with proline in the amino acid sequence of the wild-type hIDS represented by SEQ ID NO: 1;
(II-d) an M6P low-modified hIDS in which an amino acid other than threonine and serine is added between isoleucine at position 256 and serine at position 257 in the amino acid sequence of the wild-type hIDS represented by SEQ ID NO: 1; and
(II-e) an M6P low-modified hIDS in which serine at position 257 is deleted or substituted with an amino acid other than threonine and serine in the amino acid sequence of the wild-type hIDS represented by SEQ ID NO: 1.

The M6P low-modified hIDS represented by the above (II-a) to (II-e) may further contain a mutation selected from the group consisting of the following (II'-a) to (II'-h) in addition to the deleted, added or substituted amino acid(s):
(II'-a) substitution of an amino acid residue or amino acid residues constituting the amino acid sequence of the M6P low-modified hIDS with another amino acid residue or other amino acid residues, in which the number of the substituted amino acid residues is 1 to 10, 1 to 5, or 1 to 3, for example, 1 or 2;
(II'-b) deletion of an amino acid residue or amino acid residues constituting the amino acid sequence of the M6P low-modified hIDS, in which the number of the deleted amino acid residues is 1 to 10, 1 to 5, or 1 to 3, for example, 1 or 2;
(II'-c) a combination of the substitution of (II'-a) and the deletion of (II'-b);
(II'-d) addition of one or more amino acid residues into the amino acid sequence of the M6P low-modified hIDS or to the N-terminus side or the C-terminus side of the amino acid sequence of the M6P low-modified hIDS, in which the number of the added amino acid residues is 1 to 10, 1 to 5, or 1 to 3, for example, 1 or 2;
(II'-e) a combination of the substitution of (II'-a) and the addition of (II'-d);
(II'-f) a combination of the deletion of (II'-b) and the addition of (II'-d);
(II'-g) a combination of the substitution of (II'-a), the deletion of (II'-b), and the addition of (II'-d); and
(II'-h) exhibition of an identity of 80% or more, 85% or more, 90% or more, 95% or more, 98% or more, or 99% or more, to the amino acid sequence of the M6P low-modified hIDS.

Alternatively, as a preferred embodiment of such an M6P low-modified hIDS, there is exemplified hIDS including at least one mutation selected from the group consisting of the following (2-a) to (2-c) with respect to the amino acid sequence represented by Asn-Xaa-Yaa corresponding to from asparagine at position 221 to threonine at position 223 in the amino acid sequence of the wild-type hIDS represented by SEQ ID NO: 1, in which the mutation newly generates no consensus sequence to which an N-linked sugar chain binds:
(2-a) deletion of Asn or substitution of Asn with an amino acid other than asparagine;
(2-b) substitution of the amino acid represented by Xaa with proline; and
(2-c) substituting of the amino acid represented by Yaa with an amino acid other than threonine and serine.

That is, one preferred embodiment of the M6P low-modified hIDS can be said to be mutated hIDS in which a mutation is added to the amino acid sequence corresponding to from asparagine at position 221 to threonine at position 223 in the amino acid sequence of the wild-type hIDS represented by SEQ ID NO: 1 in such a manner that the N-linked sugar chain binding to asparagine at position 221 is deleted.

The M6P low-modified hIDS of the present invention may further include a mutation selected from the group consisting of the following (2'-a) to (2'-h) in an amino acid sequence other than the amino acid sequence corresponding to from asparagine at position 221 to threonine at position 223 in the amino acid sequence of the wild-type hIDS represented by SEQ ID NO: 1, in which the mutation newly generates no consensus sequence to which an N-linked sugar chain binds:
(2'-a) substitution of 1 to 10 amino acids with another amino acid or other amino acids;
(2'-b) deletion of 1 to 10 amino acids;
(2'-c) a combination of the substitution of (2'-a) and the deletion of (2'-b);
(2'-d) addition of 1 to 10 amino acids into the amino acid sequence or to the N-terminus or C-terminus of the amino acid sequence;
(2'-e) a combination of the substitution of (2'-a) and the addition of (2'-d);
(2'-f) a combination of the deletion of (2'-b) and the addition of (2'-d);
(2'-g) a combination of the substitution of (2'-a), the deletion of (2'-b), and the addition of (2'-d); and
(2'-h) exhibition of an identity of 80% or more.

Furthermore, as a preferred embodiment of such an M6P low-modified hIDS, there is exemplified hIDS including at least one mutation selected from the group consisting of the following (3-a) to (3-c) with respect to the amino acid sequence represented by Asn-Xaa-Yaa corresponding to from asparagine at position 255 to serine at position 257 in the amino acid sequence of the wild-type hIDS represented by SEQ ID NO: 1, in which the mutation newly generates no consensus sequence to which an N-linked sugar chain binds:
(3-a) deletion of Asn or substitution of Asn with an amino acid other than asparagine;
(3-b) substitution of the amino acid represented by Xaa with proline; and
(3-c) substitution of the amino acid represented by Yaa with an amino acid other than threonine and serine.

That is, one preferred embodiment of the M6P low-modified hIDS can be said to be mutated hIDS in which a mutation is added to the amino acid sequence corresponding to from asparagine at position 255 to serine at position 257 in the amino acid sequence of the wild-type hIDS represented by SEQ ID NO: 1 in such a manner that the N-linked sugar chain binding to asparagine at position 255 is deleted.

Furthermore, the M6P low-modified hIDS of the present invention may further include a mutation selected from the group consisting of the following (3'-a) to (3'-h) in an amino acid sequence other than the amino acid sequence corresponding to from asparagine at position 255 to serine at position 257 in the amino acid sequence of the wild-type hIDS represented by SEQ ID NO: 1, in which the mutation newly generates no consensus sequence to which an N-linked sugar chain binds:
(3'-a) substitution of 1 to 10 amino acids with another amino acid or other amino acids;
(3'-b) deletion of 1 to 10 amino acids;
(3'-c) a combination of the substitution of (3'-a) and the deletion of (3'-b);
(3'-d) addition of 1 to 10 amino acids into the amino acid sequence or to the N-terminus or C-terminus of the amino acid sequence;
(3'-e) a combination of the substitution of (3'-a) and the addition of (3'-d);
(3'-f) a combination of the deletion of (3'-b) and the addition of (3'-d);
(3'-g) a combination of the substitution of (3'-a), the deletion of (3'-b), and the addition of (3'-d); and
(3'-h) exhibition of an identity of 80% or more.

Preferred embodiments of the M6P low-modified hIDS include, for example, those having the amino acid sequences shown in the following (4-a) to (4-e) and having no new consensus sequence to which an N-linked sugar chain binds:
(4-a) an amino acid sequence exhibiting, in the amino acid sequence except for amino acids at positions 221 and 255, an identity of 80% or more, 85% or more, 90% or more, 95% or more, 98% or more, or 99% or more, to an amino acid sequence of hIDS represented by SEQ ID NO: 4 in which asparagines at positions 221 and 255 in the amino acid sequence of the wild-type hIDS represented by SEQ ID NO: 1 are each substituted with glutamine;
(4-b) the amino acid sequence of the M6P low-modified hIDS represented by SEQ ID NO: 4 in which asparagines at positions 221 and 255 in the amino acid sequence of the wild-type hIDS represented by SEQ ID NO: 1 are each substituted with glutamine;
(4-c) an amino acid sequence exhibiting, in the amino acid sequence except for amino acids at positions 223 and 257, an identity of 80% or more, 85% or more, 90% or more, 95% or more, 98% or more, or 99% or more, to an amino acid sequence of M6P low-modified hIDS represented by SEQ ID NO: 5 in which threonine at position 223 and serine at position 257 in the amino acid sequence of the wild-type hIDS represented by SEQ ID NO: 1 are each substituted with alanine;
(4-d) the amino acid sequence of the M6P low-modified hIDS represented by SEQ ID NO: 5 in which threonine at position 223 and serine at position 257 in the amino acid sequence of the wild-type hIDS represented by SEQ ID NO: 1 are each substituted with alanine; and
(4-e) an amino acid sequence of M6P low-modified hIDS represented by SEQ ID NO: 6 in which asparagines at positions 221 and 255 are each substituted with glutamine and threonine at position 223 and serine at position 257 is substituted with alanine in the amino acid sequence of the wild-type hIDS represented by SEQ ID NO: 1.

The M6P low-modified hIDS maintains 10% or more, 20% or more, 30% or more, 40% or more, 50% or more, or 60% or more of the enzyme activity, and preferably maintains 20% or more, or 50% or more of the enzyme activity, as compared with the wild-type hIDS.

The wild-type human α-L-iduronidase (hIDUA) is one of lysosomal enzymes and composed of 628 amino acid residues represented by SEQ ID NO: 7. hIDUA is a lysosomal enzyme that hydrolyzes iduronic acid bonds present in dermatan sulfate and heparan sulfate molecules. Hurler's syndrome is also referred to as mucopolysaccharidosis type I (MPS type I), and is caused by accumulation of intracellular dermatan sulfate and the like associated with deficiency of α-L-iduronidase activity in lysosomes. A patient with Hurler's syndrome may have central nervous system disorder. hIDUA bound to a ligand having affinity for a protein on a cerebrovascular endothelial cell can be used as a therapeutic agent for central nervous system disorder associated with Hurler's syndrome.

As used herein, the term "human α-L-iduronidase" or "hIDUA" encompasses not only the normal wild-type hIDUA composed of 628 amino acid residues represented by SEQ ID NO: 7, but also, without particular distinction, a mutant of hIDUA corresponding to that in which one or more amino acid residues are substituted, deleted, and/or added with respect to the amino acid sequence represented by SEQ ID NO: 7 (as used herein, "addition" of an amino acid residue means addition of a residue to a terminus or an internal portion of the sequence), as long as the mutant has a function as the normal wild-type hIDUA, such as having an enzyme activity capable of decomposing heparan sulfate and dermatan sulfate. The wild-type hIDUA is encoded by, for example, a gene having a base sequence represented by SEQ ID NO: 8. In a case of substituting an amino acid residue or amino acid residues with another amino acid residue or amino acid residues, the number of amino acid residues to be substituted is 1 to 10, 1 to 5, or 1 to 3, for example, 1 or 2. In a case of deleting an amino acid residue or amino acid residues, the number of amino acid residues to be deleted is 1 to 10, 1 to 5, or 1 to 3, for example, 1 or 2. In a case of deleting an amino acid residue or amino acid residues, the amino acid residue or amino acid residues at the N-terminus may be deleted. In this case, the number of amino acid residues to be deleted is 1 to 10, 1 to 5, or 1 to 3, for example, 1 or 2. Furthermore, the substitution and deletion of an amino acid residue or amino acid residues may be combined.

In a case of adding an amino acid residue or amino acid residues to the amino acid sequence represented by SEQ ID NO: 7, one or more amino acid residues are added into the amino acid sequence of hIDUA or to the N-terminus side or the C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined. Normal wild-type hIDUA is biosynthesized as a precursor composed of 653 amino acid residues, and a leader peptide composed of 25 amino acid residues from the N-terminus represented by SEQ ID NO: 9 is removed to give hIDUA. In a case where an amino acid is added to the N-terminus side of hIDUA, the amino acid may be derived from the leader peptide. In this case, Pro is added to the N-terminus in a case of adding one amino acid, Ala-Pro in a case of adding two amino acids, or Val-Ala-Pro in a case of adding three amino acids.

Examples of the mutant of hIDUA in which at least two types of mutations among these three types of substitution, deletion, and addition of an amino acid or amino acids are introduced in combination include, but are not limited to, the following (i) to (iv):
(i) a mutant having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence represented by SEQ ID NO: 7;
(ii) a mutant having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence represented by SEQ ID NO: 7;
(iii) a mutant having an amino acid sequence obtained by performing deletion of 0 to 3 amino acid residues, substitution of 0 to 3 amino acid residues with another amino acid residue or other amino acid residues, and further addition of 0 to 3 amino acid residues, with respect to the amino acid sequence represented by SEQ ID NO: 7; and
(iv) a mutant having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence represented by SEQ ID NO: 7.

The above-described wild-type or mutant-type hIDUA in which an amino acid constituting the hIDUA is modified with a sugar chain is also hIDUA. The above-described wild-type or mutant-type hIDUA in which an amino acid constituting the hIDUA is modified with phosphoric acid is also hIDUA. The above-described wild-type or mutant-type hIDUA in which an amino acid constituting the hIDUA is modified with a moiety other than a sugar chain and phosphoric acid is also hIDUA. The above-described wild-type or mutant-type hIDUA in which the side chain of an amino acid constituting the hIDUA is converted by a substitution reaction or the like is also hIDUA. Such conversion includes, but is not limited to, conversion of a cysteine residue to formylglycine.

That is, hIDUA modified with a sugar chain is encompassed in hIDUA having the amino acid sequence before the modification. hIDUA modified with phosphoric acid is encompassed in hIDUA having the original amino acid sequence before modification with phosphoric acid. hIDUA modified with a moiety other than a sugar chain and phosphoric acid is also encompassed in hIDUA having the original amino acid sequence before the modification. Those in which the side chain of an amino acid constituting hIDUA is converted by a substitution reaction or the like are also encompassed in hIDUA having the original amino acid sequence before the conversion. Such conversion includes, but is not limited to, conversion of a cysteine residue to formylglycine.

In the present invention, the term "human α-L-iduronidase" (hIDUA mutant) refers to a mutant in which one or more amino acid residues are substituted, deleted, and/or added, with respect to the amino acid sequence of the normal wild-type hIDUA (the amino acid sequence represented by SEQ ID NO: 7) (as used herein, "addition" of an amino acid residue means addition of a residue to a terminus or an internal portion of the sequence), and has a function as the normal wild-type hIDUA such as having enzyme activity capable of decomposing heparan sulfate and dermatan sulfate. In the present invention, the hIDUA mutant maintains 10% or more, 20% or more, 30% or more, 40% or more, 50% or more, or 60% or more of the enzyme activity, and preferably maintains 20% or more, or 50% or more of the enzyme activity, as compared with the wild-type hIDUA. Preferred hIDUA mutants in the present invention include those in which one or more amino acid residues are substituted with another amino acid residue or other amino acid residues, deleted, or added, with respect to the amino acid sequence of SEQ ID NO: 7. In a case of substituting an amino acid residue or amino acid residues in the amino acid sequence with another amino acid residue or other amino acid residues, the number of amino acid residues to be substituted is 1 to 10, 1 to 5, or 1 to 3, and for example, 1 or 2. In a case of deleting an amino acid residue or amino acid residues, the number of amino acid residues to be deleted is 1 to 10, 1 to 5, or 1 to 3, for example, 1 or 2. The hIDUA mutant may be an hIDUA mutant in which the substitution and deletion of an amino acid residue or amino acid residues are combined.

In a case of adding an amino acid residue or amino acid residues to the amino acid sequence represented by SEQ ID NO: 7, one or more amino acid residues are added into the amino acid sequence of hIDUA or to the N-terminus side or the C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined. That is, the hIDUA mutant may be an hIDUA mutant in which at least two types of mutations among three types of mutations, i.e., substitution, deletion, and addition of an amino acid or amino acids, are introduced in combination, with respect to the amino acid sequence represented by SEQ ID NO: 7.

Examples of the mutant of hIDUA in which at least two types of mutations among these three types of substitution, deletion, and addition of an amino acid or amino acids are introduced in combination include, but are not limited to, the following (i) to (iv):
(i) a mutant having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence represented by SEQ ID NO: 7 (provided that the wild-type hIDUA is excluded);
(ii) a mutant having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence represented by SEQ ID NO: 7 (provided that the wild-type hIDUA is excluded);
(iii) a mutant having an amino acid sequence obtained by performing deletion of 0 to 3 amino acid residues, substitution of 0 to 3 amino acid residues with another amino acid residue or other amino acid residues, and further addition of 0 to 3 amino acid residues, with respect to the amino acid sequence represented by SEQ ID NO: 7 (provided that the wild-type hIDUA is excluded); and
(iv) a mutant having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence represented by SEQ ID NO: 7 (provided that the wild-type hIDUA is excluded).

The position and form (deletion, substitution, and addition) of each mutation as compared with those of the wild-type hIDUA can be easily confirmed by the alignment of the amino acid sequences of the wild-type hIDUA and the mutant-type hIDUA.

The amino acid sequence of the hIDUA mutant preferably has an identity of 80% or more, an identity of 85% or more, an identity of 90% or more, or an identity of 95% or more, and for example, an identity of 98% or more, or an identity of 99% or more, to the amino acid sequence of the normal wild-type hIDUA represented by SEQ ID NO: 7.

In one embodiment, the protein of the present invention is a glycoprotein obtained by adding a mutation to the wild-type amino acid sequence of a glycoprotein to which N-linked sugar chains containing one or more mannose-6-phosphates (M6Ps) bind in the wild type to delete at least one N-linked sugar chain binding to the glycoprotein, that is, an M6P low-modified protein. As the glycoprotein to which N-linked sugar chains containing one or more mannose-6-phosphates (M6Ps) bind in the wild type, hIDUA can be used, and a mutation is added to the amino acid sequence of the wild-type hIDUA to modify the consensus sequence, whereby an M6P low-modified hIDUA in which at least one N-linked sugar chain binding to hIDUA is deleted can be prepared. In the consensus sequence of the wild-type hIDUA represented by SEQ ID NO: 7, the site to which an N-linked sugar chain containing M6P binds is an asparagine residue contained in each of the consensus sequence starting from asparagine at position 311 and the consensus sequence starting from asparagine at position 426. Thus, the consensus sequence starting from asparagine at position 311 and/or the consensus sequence starting from asparagine at position 426 in the amino acid sequence of the wild-type hIDUA represented by SEQ ID NO: 7 is modified, whereby an M6P low-modified hIDUA in which the N-linked sugar chain containing M6P binding to hIDUA is deleted can be prepared. The M6P low-modified hIDUA can be prepared by adding a mutation to not only the wild-type hIDUA but also the above-described hIDUA mutant.

Preferred embodiments of such an M6P low-modified hIDUA include the following (III-a) to (III-e):
(III-a) an M6P low-modified hIDUA in which asparagine at position 311 is deleted or substituted with an amino acid other than asparagine in the amino acid sequence of the wild-type hIDUA represented by SEQ ID NO: 7;
(III-b) an M6P low-modified hIDUA in which proline is added between asparagine at position 311 and threonine at position 312 in the amino acid sequence of the wild-type hIDUA represented by SEQ ID NO: 7;
(III-c) an M6P low-modified hIDUA in which threonine at position 312 is substituted with proline in the amino acid sequence of the wild-type hIDUA represented by SEQ ID NO: 7;
(III-d) an M6P low-modified hIDUA in which an amino acid other than threonine, serine, and asparagine is added between threonine at position 312 and threonine at position 313 in the amino acid sequence of the wild-type hIDUA represented by SEQ ID NO: 7; and
(III-e) an M6P low-modified hIDUA in which threonine at position 313 is substituted with an amino acid other than threonine and serine in the amino acid sequence of the wild-type hIDUA represented by SEQ ID NO: 7.

The M6P low-modified hIDUA shown in the above (III-a) to (III-e) may further include a mutation selected from the group consisting of the following (III'-a) to (III'-h) in addition to the deleted, added, or substituted amino acid(s):
(III'-a) substitution of an amino acid residue or amino acid residues constituting the amino acid sequence of the M6P low-modified hIDUA with another amino acid residue or other amino acid residues, in which the number of the substituted amino acid residues is 1 to 10, 1 to 5, or 1 to 3, for example, 1 or 2;
(III'-b) deletion of an amino acid residue or amino acid residues constituting the amino acid sequence of the M6P low-modified hIDUA, in which the number of the deleted amino acid residues is 1 to 10, 1 to 5, or 1 to 3, for example, 1 or 2;
(III'-c) a combination of the substitution of (III'-a) and the deletion of (III'-b);
(III'-d) addition of one or more amino acid residues into the amino acid sequence of the M6P low-modified hIDUA or to the N-terminus side or the C-terminus side of the amino acid sequence of the M6P low-modified hIDUA, in which the number of the added amino acid residues is 1 to 10, 1 to 5, or 1 to 3, for example, 1 or 2;
(III'-e) a combination of the substitution of (III'-a) and the addition of (III'-d);
(III'-f) a combination of the deletion of (III'-b) and the addition of (III'-d);
(III'-g) a combination of the substitution of (III'-a), the deletion of (III'-b), and the addition of (III'-d); and
(III'-h) exhibition of an identity of 80% or more, 85% or more, 90% or more, 95% or more, 98% or more, or 99% or more, to the amino acid sequence of the M6P low-modified hIDUA.

Preferred embodiments of the M6P low-modified hIDUA include the following (IV-a) to (IV-e):
(IV-a) an M6P low-modified hIDUA in which asparagine at position 426 is deleted or substituted with an amino acid other than asparagine in the amino acid sequence of the wild-type hIDUA represented by SEQ ID NO: 7;
(IV-b) an M6P low-modified hIDUA in which an amino acid other than asparagine is added between asparagine at position 426 and arginine at position 427 in the amino acid sequence of the wild-type hIDUA represented by SEQ ID NO: 7;
(IV-c) an M6P low-modified hIDUA in which arginine at position 427 is deleted or substituted with proline in the amino acid sequence of the wild-type hIDUA represented by SEQ ID NO: 7;
(IV-d) an M6P low-modified hIDUA in which an amino acid other than threonine and serine is added between arginine at position 427 and serine at position 428 in the amino acid sequence of the wild-type hIDUA represented by SEQ ID NO: 7; and
(IV-e) an M6P low-modified hIDUA in which serine at position 428 is deleted or substituted with an amino acid other than threonine and serine in the amino acid sequence of the wild-type hIDUA represented by SEQ ID NO: 7.

The M6P low-modified hIDUA shown in the above (IV-a) to (IV-e) may further include a mutation selected from the group consisting of the following (IV'-a) to (IV'-h) in addition to the deleted, added, or substituted amino acid(s):
(IV'-a) substitution of an amino acid residue or amino acid residues constituting the amino acid sequence of the M6P low-modified hIDUA with another amino acid residue, in which the number of the substituted amino acid residues is 1 to 10, 1 to 5, or 1 to 3, for example, 1 or 2;
(IV'-b) deletion of an amino acid residue or amino acid residues constituting the amino acid sequence of the M6P low-modified hIDUA, in which the number of the deleted amino acid residues is 1 to 10, 1 to 5, or 1 to 3, for example, 1 or 2;
(IV'-c) a combination of the substitution of (IV'-a) and the deletion of (IV'-b);
(IV'-d) addition of one or more amino acid residues into the amino acid sequence of the M6P low-modified hIDUA or to the N-terminus side or the C-terminus side of the amino acid sequence of the M6P low-modified hIDUA, in which the number of the added amino acid residues is 1 to 10, 1 to 5, or 1 to 3, for example, 1 or 2;
(IV'-e) a combination of the substitution of (IV'-a) and the addition of (IV'-d);
(IV'-f) a combination of the deletion of (IV'-b) and the addition of (IV'-d);
(IV'-g) a combination of the substitution of (IV'-a), the deletion of (IV'-b), and the addition of (IV'-d); and
(IV'-h) exhibition of an identity of 80% or more, 85% or more, 90% or more, 95% or more, 98% or more, or 99% or more, to the amino acid sequence of the M6P low-modified hIDUA.

Alternatively, as a preferred embodiment of such an M6P low-modified hIDUA, there is exemplified an hIDUA including at least one mutation selected from the group consisting of the following (5-a) to (5-c) with respect to the amino acid sequence represented by Asn-Xaa-Yaa corresponding to from asparagine at position 311 to threonine at position 313 in the amino acid sequence of the wild-type hIDUA represented by SEQ ID NO: 7, in which the mutation newly generates no consensus sequence to which an N-linked sugar chain binds:
(5-a) deletion of Asn or substitution of Asn with an amino acid other than asparagine;
(5-b) substitution of the amino acid represented by Xaa with proline; and
(5-c) substitution of the amino acid represented by Yaa with an amino acid other than threonine and serine.

That is, one preferred embodiment of the M6P low-modified hIDUA can be said to be mutated hIDUA in which a mutation is added to the amino acid sequence corresponding to from asparagine at position 311 to threonine at position 313 in the amino acid sequence of the wild-type hIDUA represented by SEQ ID NO: 7 in such a manner that the N-linked sugar chain binding to the asparagine at position 311 is deleted.

Further, the M6P low-modified hIDUA of the present invention may further include a mutation selected from the group consisting of the following (5'-a) to (5'-h) in an amino acid sequence other than the amino acid sequence corresponding to the region from asparagine at position 311 to threonine at position 313 in the amino acid sequence of the wild-type hIDUA represented by SEQ ID NO: 7, in which the mutation newly generates no consensus sequence to which an N-linked sugar chain binds:
(5'-a) a substitution of 1 to 10 amino acids with another amino acid or other amino acids;
(5'-b) a deletion of 1 to 10 amino acids;
(5'-c) a combination of the substitution of (5'-a) and the deletion of (5'-b);
(5'-d) an addition of 1 to 10 amino acids into the amino acid sequence or to the N-terminus or C-terminus of the amino acid sequence;
(5'-e) a combination of the substitution of (5'-a) and the addition of (5'-d);
(5'-f) a combination of the deletion of (5'-b) and the addition of (5'-d);
(5'-g) a combination of the substitution of (5'-a), the deletion of (5'-b), and the addition of (5'-d); and
(5'-h) an exhibition of an identity of 80% or more.

Furthermore, as a preferred embodiment of such an M6P low-modified hIDUA, there is exemplified an hIDUA including at least one mutation selected from the group consisting of the following (6-a) to (6-c) with respect to the amino acid sequence represented by Asn-Xaa-Yaa corresponding to from asparagine at position 426 to serine at position 428 in the amino acid sequence of the wild-type hIDUA represented by SEQ ID NO: 7, in which the mutation newly generates no consensus sequence to which an N-linked sugar chain binds:
(6-a) deletion of Asn or substitution of Asn with an amino acid other than asparagine;
(6-b) substitution of the amino acid represented by Xaa with proline; and
(6-c) substitution of the amino acid represented by Yaa with an amino acid other than threonine and serine.

That is, one preferred embodiment of the M6P low-modified hIDUA can be said to be mutated hIDUA in which a mutation is added to the amino acid sequence corresponding to from asparagine at position 426 to serine at position 428 in the amino acid sequence of the wild-type hIDUA represented by SEQ ID NO: 7 in such a manner that the N-linked sugar chain binding to the asparagine at position 426 is deleted.

Further, the M6P low-modified hIDUA in the present invention may further include a mutation selected from the group consisting of the following (6'-a) to (6'-h) in an amino acid sequence other than the amino acid sequence corresponding to from asparagine at position 426 to serine at position 428 in the amino acid sequence of the wild-type hIDUA represented by SEQ ID NO: 7, in which the mutation newly generates no consensus sequence to which an N-linked sugar chain binds:
(6'-a) substitution of 1 to 10 amino acids with another amino acid or other amino acids;
(6'-b) deletion of 1 to 10 amino acids;
(6'-c) a combination of the substitution of (6'-a) and the deletion of (6'-b);
(6'-d) addition of 1 to 10 amino acids into the amino acid sequence or to the N-terminus or C-terminus of the amino acid sequence;
(6'-e) a combination of the substitution of (6'-a) and the addition of (6'-d);
(6'-f) a combination of the deletion of (6'-b) and the addition of (6'-d);
(6'-g) a combination of the substitution of (6'-a), the deletion of (6'-b), and the addition of (6'-d); and
(6'-h) exhibition of an identity of 80% or more.

Preferred embodiments of the M6P low-modified hIDUA include, for example, those having the amino acid sequences represented by the following (7-a) to (7-e) and having no new consensus sequence to which an N-linked sugar chain binds:
(7-a) an amino acid sequence exhibiting an identity of 80% or more, 85% or more, 90% or more, 95% or more, 98% or more, or 99% or more, to the amino acid sequence of the M6P low-modified hIDUA represented by SEQ ID NO: 10 in which both asparagines at positions 311 and 426 in the amino acid sequence of the wild-type hIDUA represented by SEQ ID NO: 7 are each substituted with glutamine, in the amino acid sequence except for amino acids at positions 311 and 426;
(7-b) the amino acid sequence of the M6P low-modified hIDUA represented by SEQ ID NO: 10 in which both asparagine at positions 311 and 426 in the amino acid sequence of the wild-type hIDUA represented by SEQ ID NO: 7 are substituted with glutamine;
(7-c) an amino acid sequence exhibiting, in the amino acid sequence except for amino acids at positions 313 and 428, an identity of 80% or more, 85% or more, 90% or more, 95% or more, 98% or more, or 99% or more, to the amino acid sequence of the M6P low-modified hIDUA represented by SEQ ID NO: 11 in which threonine at position 313 and serine at position 428 in the amino acid sequence of the wild-type hIDUA represented by SEQ ID NO: 7 are each substituted with alanine;
(7-d) the amino acid sequence of the M6P low-modified hIDUA represented by SEQ ID NO: 11 in which threonine at position 313 and serine at position 428 in the amino acid sequence of the wild-type hIDUA represented by SEQ ID NO: 7 are each substituted with alanine; and
(7-e) the amino acid sequence of the M6P low-modified hIDUA represented by SEQ ID NO: 12, in which both asparagines at positions 311 and 426 in the amino acid sequence of the wild-type hIDUA represented by SEQ ID NO: 7 are each substituted with glutamine, and threonine at position 313 and serine at position 428 is substituted with alanine.

Such an M6P low-modified hIDUA maintains 10% or more, 20% or more, 30% or more, 40% or more, 50% or more, or 60% or more of the enzyme activity, and preferably maintains 20% or more, or 50% or more of the enzyme activity, as compared with the wild-type hIDUA.

Wild-type human heparan N-sulfatase (hSGSH) is one of lysosomal enzymes and composed of 482 amino acid residues represented by SEQ ID NO: 13. Sanfilippo's syndrome is also referred to as mucopolysaccharidosis type IIIA (MPS IIIA) and is caused by accumulation of intracellular heparan sulphate associated with deficiency of SGSH activity in lysosomes. However, deficiencies in other enzymes such as α-N-acetylglucosaminidase may also be pathogenic. A patient with Sanfilippo's syndrome may have central nervous system disorder. hSGSH bound to a ligand having affinity for a protein on a cerebrovascular endothelial cell can be used as a therapeutic agent for central nervous system disorder associated with Sanfilippo's syndrome.

As used herein, the term "human heparan N-sulfatase" or "hSGSH" encompasses not only the normal wild-type hSGSH composed of 482 amino acid residues represented by SEQ ID NO: 13, but also, without particular distinction, a mutant of hSGSH corresponding to that in which one or more amino acid residues are substituted, deleted, and/or added with respect to the amino acid sequence represented by SEQ ID NO: 13 (as used herein, "addition" of an amino acid residue means addition of a residue to a terminus or an internal portion of the sequence), as long as the mutant has a function as the normal wild-type hSGSH, such as having an enzyme activity capable of decomposing heparan sulfate. The wild-type hSGSH is encoded by, for example, the base sequence of SEQ ID NO: 14. In a case of substituting an amino acid residue or amino acid residues with another amino acid residue or amino acid residues, the number of amino acid residues to be substituted is 1 to 10, 1 to 5, or 1 to 3, for example, 1 or 2. In a case of deleting an amino acid residue or amino acid residues, the number of amino acid residues to be deleted is 1 to 10, 1 to 5, or 1 to 3, for example, 1 or 2. In a case of deleting an amino acid residue or amino acid residues, the amino acid residue or amino acid residues at the N-terminus may be deleted. In this case, the number of amino acid residues to be deleted is 1 to 10, 1 to 5, or 1 to 3, for example, 1 or 2. Furthermore, the substitution and deletion of an amino acid residue or amino acid residues may be combined.

In a case of adding an amino acid residue or amino acid residues to the amino acid sequence represented by SEQ ID NO: 13, one or more amino acid residues are added into the amino acid sequence of hSGSH or to the N-terminus side or the C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined. Normal wild-type hSGSH is biosynthesized as a precursor composed of 502 amino acid residues, and a leader peptide composed of 20 amino acid residues from the N-terminus represented by SEQ ID NO: 15 is removed to give hSGSH. In a case of adding an amino acid to the N-terminus side of hSGSH, the amino acid may be derived from the leader peptide. In this case, Ala is added to the N-terminus in a case of adding one amino acid, Arg-Ala in a case of adding two amino acids, or Cys-Arg-Ala in a case of adding three amino acids.

Examples of the mutant of hSGSH in which at least two types of mutations among these three types of substitution, deletion, and addition of an amino acid or amino acids are introduced in combination include, but are not limited to, the following (i) to (iv):
(i) a mutant having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence represented by SEQ ID NO: 13;
(ii) a mutant having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence represented by SEQ ID NO: 13;
(iii) a mutant having an amino acid sequence obtained by performing deletion of 0 to 3 amino acid residues, substitution of 0 to 3 amino acid residues with another amino acid residue or other amino acid residues, and further addition of 0 to 3 amino acid residues, with respect to the amino acid sequence represented by SEQ ID NO: 13; and
(iv) a mutant having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence represented by SEQ ID NO: 13.

The above-described wild-type or mutant-type hSGSH in which an amino acid constituting the hSGSH is modified with a sugar chain is also hSGSH. The above-described wild-type or mutant-type hSGSH in which an amino acid constituting the hSGSH is modified with phosphoric acid is also hSGSH. The above-described wild-type or mutant-type hSGSH in which an amino acid constituting the hSGSH is modified with a moiety other than a sugar chain and phosphoric acid is also hSGSH. The above-described wild-type or mutant-type hSGSH in which the side chain of an amino acid constituting the hSGSH is converted by a substitution reaction or the like is also hSGSH. Such conversion includes, but is not limited to, conversion of a cysteine residue to formylglycine.

That is, the hSGSH modified with a sugar chain is encompassed in hSGSH having the amino acid sequence before the modification. The hSGSH modified with phosphoric acid is also encompassed in hSGSH having the amino acid sequence before the modification with phosphoric acid. The hSGSH modified with a moiety other than a sugar chain and phosphoric acid is also encompassed in hSGSH having the amino acid sequence before the modification. The hSGSH in which the side chain of an amino acid constituting hSGSH is converted by a substitution reaction or the like is also encompassed in hSGSH having the original amino acid sequence before the conversion. Such conversion includes, but is not limited to, conversion of a cysteine residue to formylglycine.

In the present invention, the term "human heparan N-sulfatase" (hSGSH mutant) refers to a mutant in which one or more amino acid residues are substituted, deleted, and/or added with respect to the amino acid sequence of the normal wild-type hSGSH (the amino acid sequence represented by SEQ ID NO: 13) (as used herein, "addition" of an amino acid residue means addition of a residue to a terminus or an internal portion of the sequence), and has a function as the normal wild-type hSGSH such as having enzyme activity capable of decomposing heparan sulfate. In the present invention, the hSGSH mutant maintains 10% or more, 20% or more, 30% or more, 40% or more, 50% or more, or 60% or more of the enzyme activity, and preferably maintains 20% or more, or 50% or more of the enzyme activity, as compared with the wild-type hSGSH. Preferred hSGSH mutants in the present invention include those in which one or more amino acid residues are substituted with another amino acid residue or other amino acid residues, deleted, or added, with respect to the amino acid sequence represented by SEQ ID NO: 13. In a case of substituting an amino acid residue or amino acid residues with another amino acid residue or other amino acid residues, the number of amino acid residues to be substituted is 1 to 10, 1 to 5, or 1 to 3, and for example, 1 or 2. In a case of deleting an amino acid residue or amino acid residues, the number of amino acid residues to be deleted is 1 to 10, 1 to 5, or 1 to 3, for example, 1 or 2. The hSGSH mutant may be an hSGSH mutant in which the substitution and deletion of an amino acid residue or amino acid residues are combined.

In a case of adding an amino acid residue or amino acid residues to the amino acid sequence represented by SEQ ID NO: 13, one or more amino acid residues are added into the amino acid sequence of hSGSH or to the N-terminus side or the C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined. That is, the hSGSH mutant may be an hSGSH mutant in which at least two types of mutations among three types of mutations, i.e., substitution, deletion, and addition of an amino acid or amino acids, are introduced in combination into the amino acid sequence represented by SEQ ID NO: 13.

Examples of the mutant of hSGSH in which at least two types of mutations among these three types of substitution, deletion, and addition of an amino acid or amino acids are introduced in combination include, but are not limited to, the following (i) to (iv):
(i) a mutant having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence represented by SEQ ID NO: 13 (provided that wild-type hSGSH is excluded);
(ii) a mutant having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence represented by SEQ ID NO: 13 (provided that wild-type hSGSH is excluded);
(iii) a mutant having an amino acid sequence obtained by performing deletion of 0 to 3 amino acid residues, substitution of 0 to 3 amino acid residues with another amino acid residue or other amino acid residues, and further addition of 0 to 3 amino acid residues, with respect to the amino acid sequence represented by SEQ ID NO: 13 (provided that wild-type hSGSH is excluded); and
(iv) a mutant having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence represented by SEQ ID NO: 13 (provided that wild-type hSGSH is excluded).

The position and form (deletion, substitution, and addition) of each mutation of various hSGSH mutants as compared with those of the wild-type hSGSH can be easily confirmed by the alignment of the amino acid sequences of the wild-type hSGSH and the mutant-type hSGSH.

The amino acid sequence of the hSGSH mutant preferably exhibits an identity of 80% or more, an identity of 85% or more, an identity of 90% or more, or an identity of 95% or more, and for example, an identity of 98% or more, or an identity of 99%, to the amino acid sequence of the wild-type hSGSH represented by SEQ ID NO: 13.

In one embodiment, the protein of the present invention is a glycoprotein obtained by adding a mutation to the wild-type amino acid sequence of a glycoprotein to which N-linked sugar chains containing one or more mannose-6-phosphates (M6Ps) bind in the wild type to delete at least one N-linked sugar chain binding to the glycoprotein, that is, an M6P low-modified protein. As a glycoprotein to which N-linked sugar chains containing one or more mannose-6-phosphates (M6Ps) bind in the wild type, hSGSH can be used, and a mutation is added to the amino acid sequence of the wild-type hSGSH to modify the consensus sequence, whereby an M6P low-modified hSGSH in which at least one N-linked sugar chain binding to hSGSH is deleted can be prepared. In the consensus sequence of the wild-type hSGSH represented by SEQ ID NO: 13, the site to which an N-linked sugar chain containing M6P binds is an asparagine residue contained in the consensus sequence starting from asparagine at position 244. Accordingly, the consensus sequence starting from asparagine at position 244 in the amino acid sequence of the wild-type hSGSH represented by SEQ ID NO: 13 is modified, whereby an M6P low-modified hSGSH in which an N-linked sugar chain containing M6P binding to hSGSH is deleted can be prepared. The M6P low-modified hSGSH can be prepared by adding a mutation to not only the wild-type hSGSH but also the above-described hSGSH mutant.

As a preferred embodiment of such an M6P low-modified hSGSH, there is exemplified an hSGSH including at least one mutation selected from the group consisting of the following (8-a) to (8-c) with respect to the amino acid sequence represented by Asn-Xaa-Yaa corresponding to from asparagine at position 244 to threonine at position 246 in the amino acid sequence of the wild-type hSGSH represented by SEQ ID NO: 13, in which the mutation newly generates no consensus sequence to which an N-linked sugar chain binds:
(8-a) deletion of Asn or substitution of Asn with an amino acid other than asparagine;
(8-b) substitution of the amino acid represented by Xaa with proline; and
(8-c) substitution of the amino acid represented by Yaa with an amino acid other than threonine and serine.

That is, one preferred embodiment of the M6P low-modified hSGSH can be said to be mutated hSGSH in which a mutation is added to an amino acid sequence corresponding to from asparagine at position 244 to threonine at position 246 in the amino acid sequence of the wild-type hIDUA represented by SEQ ID NO: 13 in such a manner that the N-linked sugar chain binding to the asparagine at position 244 is deleted.

Further, the M6P low-modified hSGSH in the present invention may further include a mutation selected from the group consisting of the following (8'-a) to (8'-h) in an amino acid sequence other than the amino acid sequence corresponding to from asparagine at position 244 to threonine at position 246 in the amino acid sequence of the wild-type hSGSH represented by SEQ ID NO: 13, in which the mutation newly generates no consensus sequence to which an N-linked sugar chain binds:
(8'-a) substitution of 1 to 10 amino acids with another amino acid or other amino acids;
(8'-b) deletion of 1 to 10 amino acids;
(8'-c) a combination of the substitution of (8'-a) and the deletion of (8'-b);
(8'-d) addition of 1 to 10 amino acids into the amino acid sequence or to the N-terminus or C-terminus of the amino acid sequence;
(8'-e) a combination of the substitution of (8'-a) and the addition of (8'-d);
(8'-f) a combination of the deletion of (8'-b) and the addition of (8'-d);
(8'-g) a combination of the substitution of (8'-a), the deletion of (8'-b), and the addition of (2'-d); and
(8'-h) exhibition of an identity of 80% or more.

Preferred embodiments of the M6P low-modified hSGSH include, for example, those having the amino acid sequences represented by the following (9-a) to (9-e) and having no new consensus sequence to which an N-linked sugar chain binds:
(9-a) an amino acid sequence exhibiting, in the amino acid sequence except for amino acids at position 244, an identity of 80% or more, 85% or more, 90% or more, 95% or more, 98% or more, or 99% or more, to the amino acid sequence of the M6P low-modified hSGSH represented by SEQ ID NO: 16 in which asparagine at position 244 in the amino acid sequence of the wild-type hSGSH represented by SEQ ID NO: 13 is substituted with glutamine;
(9-b) the amino acid sequence of the M6P low-modified hSGSH represented by SEQ ID NO: 16 in which asparagine at position 244 in the amino acid sequence of the wild-type hSGSH represented by SEQ ID NO: 13 is substituted with glutamine;
(9-c) an amino acid sequence exhibiting, in the amino acid sequence except for an amino acid at position 246, an identity of 80% or more, 85% or more, 90% or more, 95% or more, 98% or more, or 99% or more, to the amino acid sequence of the M6P low-modified hSGSH represented by SEQ ID NO: 17 in which threonine at position 246 in the amino acid sequence of the wild-type hSGSH represented by SEQ ID NO: 13 is substituted with alanine;
(9-d) the amino acid sequence of the M6P low-modified hSGSH represented by SEQ ID NO: 17 in which threonine at position 246 in the amino acid sequence of the wild-type hSGSH represented by SEQ ID NO: 13 is substituted with alanine; and
(9-e) the amino acid sequence of the M6P low-modified hSGSH represented by SEQ ID NO: 18, in which asparagine at position 244 is substituted with glutamine, and threonine at position 246 is substituted with alanine in the amino acid sequence of the wild-type hSGSH represented by SEQ ID NO: 13.

Such an M6P low-modified hSGSH maintains 10% or more, 20% or more, 30% or more, 40% or more, 50% or more, or 60% or more of the enzyme activity, and preferably maintains 20% or more, or 50% or more of the enzyme activity, as compared with the wild-type hSGSH.

Wild-type human acid α-glucosidase (hGAA) is one of lysosomal enzymes and composed of 883 amino acid residues represented by SEQ ID NO: 19. hGAA is a lysosomal enzyme that hydrolyzes α-1,4 or α-1,6 linkage present in glycogen. Pompe disease is also referred to as glycogen storage disease type II (GSD II) and is caused by accumulation of intracellular glycogen and the like associated with deficiency of α-glucosidase activity in lysosome. A patient with Pompe disease may have central nervous system disorder. hGAA bound to a ligand having affinity for a protein on a cerebrovascular endothelial cell can be used as a therapeutic agent for central nervous system disorder associated with Pompe disease.

As used herein, the term "human acid α-glucosidase" or "hGAA" encompasses not only the normal wild-type hGAA composed of 883 amino acid residues represented by SEQ ID NO: 19, but also, without particular distinction, a mutant of hGAA corresponding to that in which one or more amino acid residues are substituted, deleted, and/or added with respect to the amino acid sequence represented by SEQ ID NO: 19 (as used herein, "addition" of an amino acid residue means addition of a residue to a terminus or an internal portion of the sequence), as long as the mutant has a function as the normal wild-type hGAA such as having enzyme activity capable of decomposing glycogen. Wild-type hGAA is encoded by, for example, a gene having the base sequence represented by SEQ ID NO: 20. In a case of substituting an amino acid residue or amino acid residues with another amino acid residue or amino acid residues, the number of amino acid residues to be substituted is 1 to 10, 1 to 5, or 1 to 3, for example, 1 or 2. In a case of deleting an amino acid residue or amino acid residues, the number of amino acid residues to be deleted is 1 to 10, 1 to 5, or 1 to 3, for example, 1 or 2. In a case of deleting an amino acid residue or amino acid residues, the amino acid residue or amino acid residues at the N-terminus may be deleted. In this case, the number of amino acid residues to be deleted is 1 to 10, 1 to 5, or 1 to 3, for example, 1 or 2. Furthermore, the substitution and deletion of an amino acid residue or amino acid residues may be combined.

In a case of adding an amino acid residue or amino acid residues to the amino acid sequence represented by SEQ ID NO: 19, one or more amino acid residues are added into the amino acid sequence of hGAA or to the N-terminus side or the C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined. Normal wild-type hGAA is biosynthesized as a precursor composed of 952 amino acid residues, and a leader peptide composed of 69 amino acid residues from the N-terminus represented by SEQ ID NO: 21 is removed to give hGAA. In a case of adding an amino acid to the N-terminus side of hGAA, the amino acid may be derived from the leader peptide. In this case, Gln is added to the N-terminus in a case of adding one amino acid, Ala-Gln in a case of adding two amino acids, or Asp-Ala-Gln in a case of adding three amino acids.

Examples of the mutant of hGAA in which at least two types of mutations among these three types of substitution, deletion, and addition of an amino acid or amino acids are introduced in combination include, but are not limited to, the following (i) to (iv):
(i) a mutant having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence represented by SEQ ID NO: 19;
(ii) a mutant having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence represented by SEQ ID NO: 19;
(iii) a mutant having an amino acid sequence obtained by performing deletion of 0 to 3 amino acid residues, substitution of 0 to 3 amino acid residues with another amino acid residue or other amino acid residues, and further addition of 0 to 3 amino acid residues, with respect to the amino acid sequence represented by SEQ ID NO: 19; and
(iv) a mutant having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence represented by SEQ ID NO: 19.

The above-described wild-type or mutant-type hGAA in which an amino acid constituting the hGAA is modified with a sugar chain is also hGAA. The above-described wild-type or mutant-type hGAA in which an amino acid constituting the hGAA is modified with phosphoric acid is also hGAA. The above-described wild-type or mutant-type hGAA in which an amino acid constituting the hGAA is modified with a moiety other than a sugar chain and phosphoric acid is also hGAA. The above-described wild-type or mutant-type hGAA in which the side chain of an amino acid constituting the hGAA is converted by a substitution reaction or the like is also hGAA. Such conversion includes, but is not limited to, conversion of a cysteine residue to formylglycine.

That is, hGAA modified with a sugar chain is encompassed in hGAA having the amino acid sequence before the modification. hGAA modified with phosphoric acid is encompassed in hGAA having the original amino acid sequence before the modification with phosphoric acid. hGAA modified with a moiety other than a sugar chain and phosphoric acid is also encompassed in hGAA having the original amino acid sequence before the modification. Those in which the side chain of an amino acid constituting hGAA is converted by a substitution reaction or the like are also encompassed in hGAA having the original amino acid sequence before the conversion. Such conversion includes, but is not limited to, conversion of a cysteine residue to formylglycine.

In the present invention, the term "human acid α-glucosidase" (hGAA mutant) refers to a mutant in which one or more amino acid residues are substituted, deleted, and/or added with respect to the amino acid sequence of the normal wild-type hGAA (the amino acid sequence represented by SEQ ID NO: 19) (as used herein, "addition" of an amino acid residue means addition of a residue to a terminus or an internal portion of the sequence), and has a function as the normal wild-type hGAA, such as having enzyme activity capable of decomposing glycogen. In the present invention, the hGAA mutant maintains 10% or more, 20% or more, 30% or more, 40% or more, 50% or more, or 60% or more of the enzyme activity, and preferably maintains 20% or more, or 50% or more of the enzyme activity, as compared with the wild-type hGAA. Preferred hGAA mutants in the present invention include those in which one or more amino acid residues are substituted with another amino acid residue or other amino acid residues, deleted, or added, with respect to the amino acid sequence represented by SEQ ID NO: 19. In a case of substituting an amino acid residue or amino acid residues with another amino acid residue or other amino acid residues, the number of amino acid residues to be substituted is 1 to 10, 1 to 5, or 1 to 3, and for example, 1 or 2. In a case of deleting an amino acid residue or amino acid residues, the number of amino acid residues to be deleted is 1 to 10, 1 to 5, or 1 to 3, for example, 1 or 2. The hGAA mutant may be an hGAA mutant in which the substitution and deletion of an amino acid residue or amino acid residues are combined.

In a case of adding an amino acid residue or amino acid residues to the amino acid sequence represented by SEQ ID NO: 19, one or more amino acid residues are added into the amino acid sequence of hGAA or to the N-terminus side or the C-terminus side of the amino acid sequence. The number of amino acid residues to be added at this time is 1 to 10, 1 to 5, or 1 to 3, for example, 1 or 2. Further, addition and substitution of an amino acid residue or amino acid residues may be combined, addition and deletion of an amino acid residue or amino acid residues may be combined, or addition, substitution, and deletion of an amino acid residue or amino acid residues may be combined. That is, the hGAA mutant may be an hGAA mutant in which at least two types of mutations among three types of mutations, i.e., substitution, deletion, and addition of an amino acid or amino acids, are introduced in combination into the amino acid sequence represented by SEQ ID NO: 19.

Examples of the mutant of hGAA in which at least two types of mutations among these three types of substitution, deletion, and addition of an amino acid or amino acids are introduced in combination include, but are not limited to, the following (i) to (iv):
(i) a mutant having an amino acid sequence obtained by performing deletion of 0 to 10 amino acid residues, substitution of 0 to 10 amino acid residues with another amino acid residue or other amino acid residues, and further addition of 0 to 10 amino acid residues, with respect to the amino acid sequence represented by SEQ ID NO: 19 (provided that wild-type hGAA is excluded);
(ii) a mutant having an amino acid sequence obtained by performing deletion of 0 to 5 amino acid residues, substitution of 0 to 5 amino acid residues with another amino acid residue or other amino acid residues, and further addition of 0 to 5 amino acid residues, with respect to the amino acid sequence represented by SEQ ID NO: 19 (provided that wild-type hGAA is excluded);
(iii) a mutant having an amino acid sequence obtained by performing deletion of 0 to 3 amino acid residues, substitution of 0 to 3 amino acid residues with another amino acid residue or other amino acid residues, and further addition of 0 to 3 amino acid residues, with respect to the amino acid sequence represented by SEQ ID NO: 19 (provided that wild-type hGAA is excluded); and
(iv) a mutant having an amino acid sequence obtained by performing deletion of 0 to 2 amino acid residues, substitution of 0 to 2 amino acid residues with another amino acid residue or other amino acid residues, and further addition of 0 to 2 amino acid residues, with respect to the amino acid sequence represented by SEQ ID NO: 19 (provided that wild-type hGAA is excluded).

The position and form (deletion, substitution, and addition) of each mutation of various hGAA mutants as compared with those of the wild-type hGAA can be easily confirmed by the alignment of the amino acid sequences of the wild-type hGAA and the mutant-type hGAA.

The amino acid sequence of the hGAA mutant preferably exhibits an identity of 80% or more, an identity of 85% or more, an identity of 90% or more, or an identity of 95% or more, and for example, an identity of 98% or more, or 99%, to the amino acid sequence of the normal wild-type hGAA represented by SEQ ID NO: 19.

In one embodiment, the protein of the present invention is a glycoprotein obtained by adding a mutation to the wild-type amino acid sequence of a glycoprotein to which N-linked sugar chains containing one or more mannose-6-phosphates (M6Ps) bind in the wild type to delete at least one N-linked sugar chain binding to the glycoprotein, that is, an M6P low-modified protein. As a glycoprotein to which N-linked sugar chains containing one or more mannose-6-phosphates (M6Ps) bind in the wild type, hGAA can be used, and a mutation is added to the amino acid sequence of the wild-type hGAA to modify the consensus sequence, whereby an M6P low-modified hGAA in which at least one N-linked sugar chain binding to hGAA is deleted can be created. In the consensus sequence of the wild-type hGAA represented by SEQ ID NO: 19, the site to which an N-linked sugar chain containing M6P binds is an asparagine residue contained in each of the consensus sequence starting from asparagine at position 71, the consensus sequence starting from asparagine at position 164, and the consensus sequence starting from asparagine at position 401. Accordingly, the consensus sequence starting from asparagine at position 71, the consensus sequence starting from asparagine at position 164, and the consensus sequence starting from asparagine at position 401 in the amino acid sequence of the wild-type hGAA represented by SEQ ID NO: 19 are modified, whereby an M6P low-modified hGAA in which an N-linked sugar chain containing M6P binding to hSGSH is deleted can be created. The M6P low-modified hGAA can be prepared by adding a mutation to not only the wild-type hGAA but also the above-described hGAA mutant.

As a preferred embodiment of such an M6P low-modified hGAA, there is exemplified an hGAA including at least one mutation selected from the group consisting of the following (10-a) to (10-c) with respect to the amino acid sequence represented by Asn-Xaa-Yaa corresponding to from asparagine at position 71 to serine at position 73 in the amino acid sequence, asparagine at position 164 to threonine at position 166 in the amino acid sequence, and asparagine at position 401 to threonine at position 403 in the amino acid sequence of the wild-type hGAA represented by SEQ ID NO: 19, in which the mutation newly generates no consensus sequence to which an N-linked sugar chain binds:
(10-a) deletion of Asn or substitution of Asn with an amino acid other than asparagine;
(10-b) substitution of the amino acid represented by Xaa with proline; and
(10-c) substitution of the amino acid represented by Yaa with an amino acid other than threonine and serine.

That is, one preferred embodiment of the M6P low-modified hGAA can be said to be mutated hGAA in which a mutation is added to at least one of an amino acid sequence corresponding to from asparagine at position 71 to serine at position 73, an amino acid sequence corresponding to from asparagine at position 164 to threonine at position 166, and an amino acid sequence corresponding to from asparagine at position 401 to threonine at position 403 in the amino acid sequence of the wild-type hGAA represented by SEQ ID NO: 19 in such a manner that the N-linked sugar chain binding to the asparagine at position 71, 164, or 401 is deleted.

Further, the M6P low-modified hGAA in the present invention may further include a mutation selected from the group consisting of the following (10'-a) to (10'-h) in an amino acid sequence other than the amino acid sequence corresponding to from asparagine at position 71 to serine at position 73 in the amino acid sequence, the amino acid sequence corresponding to from asparagine at position 164 to threonine at position 166, and the amino acid sequence corresponding to from asparagine at position 401 to threonine at position 403 in the amino acid sequence of the wild-type hGAA represented by SEQ ID NO: 19, in which the mutation newly generates no consensus sequence to which an N-linked sugar chain binds:
(10'-a) substitution of 1 to 10 amino acids with another amino acid or other amino acids;
(10'-b) deletion of 1 to 10 amino acids;
(10'-c) a combination of the substitution of (10'-a) and the deletion of (10'-b);
(10'-d) addition of 1 to 10 amino acids into an amino acid sequence or to the N-terminus or C-terminus of the amino acid sequence;
(10'-e) a combination of the substitution of (10'-a) and the addition of (10'-d);
(10'-f) a combination of the deletion of (10'-b) and the addition of (10'-d);
(10'-g) a combination of the substitution of (10'-a), the deletion of (10'-b), and the addition of (10'-d); and
(10'-h) exhibition of an identity of 80% or more.

Preferred embodiments of the M6P low-modified hGAA include, for example, those having the amino acid sequences represented by the following (11-a) to (11-e) and having no new consensus sequence to which a N-linked sugar chain binds:
(11-a) an amino acid sequence exhibiting, in the amino acid sequence except for amino acids at positions 71, 164, and 401, an identity of 80% or more, 85% or more, 90% or more, 95% or more, 98% or more, or 99% or more, to the amino acid sequence of the M6P low-modified hGAA represented by SEQ ID NO: 22 in which asparagines at positions 71, 164, and 401 in the amino acid sequence of the wild-type hGAA represented by SEQ ID NO: 19 are each substituted with glutamine;
(11-b) the amino acid sequence of the M6P low-modified hGAA represented by SEQ ID NO: 22 in which asparagines at positions 71, 164, and 401 in the amino acid sequence of the wild-type hGAA represented by SEQ ID NO: 19 are each substituted with glutamine;
(11-c) an amino acid sequence exhibiting, in the amino acid sequence except for amino acids at positions 73, 166 and 403, an identity of 80% or more, 85% or more, 90% or more, 95% or more, 98% or more, or 99% or more, to the amino acid sequence of the M6P low-modified hGAA represented by SEQ ID NO: 23 in which serine at position 73 and threonines at positions 166 and 403 in the amino acid sequence of the wild-type hGAA represented by SEQ ID NO: 19 are each substituted with alanine;
(11-d) the amino acid sequence of the M6P low-modified hGAA represented by SEQ ID NO: 23 in which serine at position 73 and threonines at positions 166 and 403 in the amino acid sequence of the wild-type hGAA represented by SEQ ID NO: 19 are each substituted with alanine; and
(11-e) the amino acid sequence of the M6P low-modified hGAA represented by SEQ ID NO: 24 in which asparagines at positions 71, 164, and 401 are each substituted with glutamine, and serine at position 73 and threonines at positions 166 and 403 are each substituted with alanine, with respect to the amino acid sequence of the wild-type hGAA represented by SEQ ID NO: 19.

Such an M6P low-modified hGAA maintains 10% or more, 20% or more, 30% or more, 40% or more, 50% or more, or 60% or more of the enzyme activity, and preferably maintains 20% or more, or 50% or more of the enzyme activity, as compared with the wild-type hGAA.

M6P low-modified proteins including the M6P low-modified hIDS, the M6P low-modified hIDUA, the M6P low-modified hSGSH, and the M6P low-modified hGAA can be produced as recombinant proteins by culturing host cells transformed with expression vectors incorporating genes encoding the M6P low-modified proteins.

The host cells to be used herein are not particularly limited as long as they are capable of expressing M6P low-modified proteins by introduction of such expression vectors, and may be any of eukaryotic cells such as mammalian cells, yeasts, plant cells, and insect-derived cells, and prokaryotic cells such as E. coli and Bacillus subtilis. Mammalian cells are particularly suitable.

In a case of using mammalian cells as host cells, the type of the mammalian cells is not particularly limited, but cells derived from a human, a mouse, or a Chinese hamster are preferred, and CHO cells derived from Chinese hamster ovary cells or NS/0 cells derived from mouse myeloma are particularly preferred. As the expression vector to be used for incorporating and expressing a DNA fragment that encodes the M6P low-modified protein, any expression vector can be used without particular limitation as long as it brings about expression of the gene when introduced into mammalian cells. The gene incorporated into the expression vector is located downstream of a DNA sequence (gene expression regulatory site) capable of regulating the frequency of transcription of the gene in mammalian cells. Examples of the gene expression regulatory site that can be used in the present invention include a cytomegalovirus-derived promoter, an SV40 early promoter, a human elongation factor-1α (EF-1α) promoter, and a human ubiquitin C promoter.

Expression vectors are known in which glutamine synthetase (GS) as a selection marker is located downstream of a gene encoding a protein of interest via an internal ribosome entry site (IRES) (WO 2012/063799, WO 2013/161958). The expression vectors described in these documents can be particularly suitably used for production of the mutant-type protein of the present invention.

For example, an expression vector for expressing a protein of interest can be suitably used for production of the M6P low-modified protein, the expression vector including a first gene-expression regulatory site, a gene encoding the protein downstream thereof, an internal ribosome entry site further downstream thereof, and a gene encoding glutamine synthetase further downstream thereof, and further including a dihydrofolate reductase gene or a drug-resistance gene downstream of the first gene-expression regulatory site or a second gene-expression regulatory site different from the first gene-expression regulatory site. In this expression vector, a cytomegalovirus-derived promoter, an SV40 early promoter, a human elongation factor-1α promoter (hEF-1α promoter), and a human ubiquitin C promoter are suitably used as the first gene-expression regulatory site or the second gene-expression regulatory site, and the hEF-1α promoter is particularly suitable.

As the internal ribosome entry site, there is suitably used an internal ribosome entry site derived from a 5' untranslated region of a genome of a virus selected from the group consisting of virus belonging to the family Picornaviridae, foot-and-mouth disease virus, hepatitis A virus, hepatitis C virus, coronavirus, bovine enterovirus, Theiler's murine encephalomyelitis virus, and Coxsackie type B virus, or a gene selected from the group consisting of human immunoglobulin heavy chain binding protein gene, Drosophila antennapedia gene, and Drosophila ultrabithorax gene. The internal ribosome entry site derived from the 5' untranslated region of mouse encephalomyocarditis virus genome is particularly suitable. In a case where the internal ribosome entry site derived from the 5' untranslated region of the mouse encephalomyocarditis virus genome is used, in addition to the wild-type internal ribosome entry site, one in which a plurality of initiation codons contained in the wild-type internal ribosome entry site are partially disrupted can also be suitably used. The drug resistance gene suitably used in this expression vector is preferably a puromycin or neomycin resistance gene, and more preferably a puromycin resistance gene.

For example, an expression vector for expressing a protein of interest can be suitably used for production of the M6P low-modified protein, the expression vector including a human elongation factor-1α promoter, a gene encoding the protein downstream thereof, an internal ribosome entry site derived from the 5' untranslated region of the mouse encephalomyocarditis virus genome further downstream thereof, and a gene encoding glutamine synthetase further downstream thereof, and further including another gene expression regulatory site and a dihydrofolate reductase gene downstream thereof, wherein the internal ribosome entry site is formed by partially disrupting a plurality of initiation codons contained in a wild-type internal ribosome entry site. Examples of such an expression vector include the expression vector described in WO 2013/161958.

Further, for example, an expression vector for expressing a protein of interest can be suitably used for production of the M6P low-modified protein of the present invention, the expression vector including a human elongation factor-1α promoter, a gene encoding the protein downstream thereof, an internal ribosome entry site derived from the 5' untranslated region of the mouse encephalomyocarditis virus genome further downstream thereof, and a gene encoding glutamine synthetase further downstream thereof, and further including another gene expression regulatory site and a drug resistance gene downstream thereof, wherein the internal ribosome entry site is formed by partially disrupting a plurality of initiation codons contained in a wild-type internal ribosome entry site. Examples of such an expression vector include pE-mIRES-GS-puro described in WO 2012/063799 and pE-mIRES-GS-mNeo described in WO 2013/161958.

Three initiation codons (ATG) are present at the 3' end of the internal ribosome entry site derived from the 5' untranslated region of the wild-type mouse encephalomyocarditis virus genome. The above-described pE-mIRES-GS-puro and pE-mIRES-GS-mNeo are expression vectors having an IRES in which the initiation codons are partially disrupted.

M6P low-modified proteins including M6P low-modified hIDS, M6P low-modified hIDUA, M6P low-modified hSGSH, and M6P low-modified hGAA can be expressed in cells or a medium by culturing host cells into which expression vectors incorporating genes encoding the proteins are introduced. A method for expressing M6P low-modified proteins in a case where mammalian cells are host cells will be described in detail below.

As a medium for culturing mammalian cells, any medium can be used without particular limitation as long as mammalian cells can be cultured and proliferated, and a serum-free medium is preferably used. In the present invention, as the serum-free medium to be used as a medium for production of recombinant protein, there is suitably used a medium containing amino acid at 3 to 700 mg/L, vitamins at 0.001 to 50 mg/L, monosaccharide at 0.3 to 10 g/L, inorganic salt at 0.1 to 10000 mg/L, trace elements at 0.001 to 0.1 mg/L, nucleoside at 0.1 to 50 mg/L, fatty acid at 0.001 to 10 mg/L, biotin at 0.01 to 1 mg/L, hydrocortisone at 0.1 to 20 µg/L, insulin at 0.1 to 20 mg/L, vitamin B12 at 0.1 to 10 mg/L, putrescine at 0.01 to 1 mg/L, sodium pyruvate at 10 to 500 mg/L, and a water-soluble iron compound. If desired, thymidine, hypoxanthine, a pH indicator and an antibiotic in the related art, and the like may be added to the medium.

A DMEM/F 12 medium (a mixed medium of DMEM and F12) may be used as a basal medium as a serum-free medium used for production of recombinant proteins. These media are well known to those skilled in the art. A DMEM (HG) HAM-modified (R5) medium containing sodium hydrogen carbonate, L-glutamine, D-glucose, insulin, sodium selenite, diaminobutane, hydrocortisone, iron sulfate (II), asparagine, aspartic acid, serine, and polyvinyl alcohol may also be used as a serum-free medium. Furthermore, commercially available serum-free media such as CD OptiCHO (trade name) medium, CHO-S-SFM II medium, or CD CHO medium (Thermo Fisher Scientific Inc., old Life Technologies Corporation), EX-CELL (trade name) Advanced CHO medium, EX-CELL (trade name) 302 medium, or EX-CELL (trade name) 325-PF medium (SAFC Biosciences Inc.) can also be used as a basal medium.

The M6P low-modified protein of the present invention is characterized in that when the protein is expressed as a recombinant protein by culturing mammalian cells into which the expression vector incorporating a gene encoding the protein is introduced in the above-described serum-free medium, N-linked sugar chains containing M6P added to the protein are reduced or deleted as compared with when the wild-type protein is expressed as a recombinant protein under the same conditions. The mammalian cells to be used at this time are CHO cells, NS/0 cells, or the like, and particularly CHO cells.

The recombinant M6P low-modified protein expressed in the cells or in the medium by culturing host cells encoding the M6P low-modified protein can be separated from contaminants and purified by a method such as column chromatography. The purified M6P low-modified protein can also be made into a conjugate with a ligand having affinity for a protein present on a cell.

As a method for producing a conjugate of the M6P low-modified protein and the ligand, there is a method of binding them via a non-peptide linker or a peptide linker. As the non-peptide linker, polyethylene glycol, polypropylene glycol, a copolymer of ethylene glycol and propylene glycol, polyoxyethylated polyol, polyvinyl alcohol, a polysaccharide, dextran, polyvinyl ether, a biodegradable polymer, a lipid polymer, a chitin, hyaluronic acid, or a derivative thereof, or a combination thereof can be used. The peptide linker is a peptide chain composed of 1 to 50 amino acids binding to each other by peptide bonds or a derivative thereof, and binds the protein of the present invention to a ligand by forming covalent bonds at its N-terminus and C-terminus with either the M6P low-modified protein or the ligand, respectively.

Note that in the present invention, the term "ligand" refers to a peptide, protein, or the like having affinity for a target protein. However, those having affinity for an M6P receptor are not included in the ligand. In one embodiment of the present invention, the target protein of the ligand is a protein present on a cell surface, and particularly a protein present on the surface of a cerebrovascular endothelial cell. Examples of such a protein present on a cell surface include a nicotinic acetylcholine receptor (nAChR), a transferrin receptor (TfR), an insulin receptor (IR), a leptin receptor, an insulin-like growth factor I receptor, an insulin-like growth factor II receptor, a lipoprotein receptor, a glucose transporter 1, an organic anion transporter, a monocarboxylic acid transporter, a low-density lipoprotein receptor-related protein 1, a low-density lipoprotein receptor-related protein 8, a nicotinic acetylcholine receptor (nAChR), and a membrane-bound precursor of heparin-binding epidermal growth factor-like growth factor. Further, OATP-F can be exemplified as the organic anion transporter, and MCT-8 can be exemplified as the monocarboxylic acid transporter. In one embodiment of the present invention, the target protein of the ligand is a protein present on the surface of a muscle cell. Examples of such a protein present on the cell surface include a transferrin receptor (TfR) and an insulin-like growth factor I receptor.

As the ligand, for example, an antibody against the protein present on the cell surface is included. As the ligand, an original ligand of a receptor present on the cell surface such as transferrin, insulin, leptin, insulin-like growth factor I, insulin-like growth factor II, or lipoprotein is included. The original ligand may be a wild-type ligand or a mutant thereof as long as it has affinity for the receptor. The ligand is not limited to these, and may be an artificially constructed peptide as long as it has affinity for a protein (including a receptor) present on a cell surface.

The M6P low-modified protein in one embodiment of the present invention can also be prepared as a fusion protein with a ligand. Such a ligand can be fused to either the N-terminus side or the C-terminus side of the M6P low-modified protein. Such a fusion protein can be prepared as a recombinant protein in the same manner as the M6P low-modified protein.

In one embodiment of the present invention, the conjugate or the fusion protein of the ligand having affinity for a protein present on a vascular endothelial cell (particularly a cerebrovascular endothelial cell) and the M6P low-modified protein can exert a function in the central nervous system (CNS) through the blood-brain barrier (BBB) after binding to the cerebrovascular endothelial cell. Suitable examples of the ligand at this time include an anti-transferrin receptor (TfR) antibody and an anti-insulin receptor (IR) antibody. In a case where such a conjugate or fusion protein is administered in vivo, the conjugate or fusion protein is taken up into cells and exhibits physiological activity in the central nervous system, while in cells expressing M6P, a decrease in the number of proteins present on the cells and serving as targets of the ligand, for example, TfR and IR, is suppressed.

In one embodiment of the present invention, a conjugate or fusion protein of a ligand having affinity for a protein present on a muscle cell and the M6P low-modified protein binds to the muscle cell and is then taken up into the muscle cell, whereby the conjugate or fusion protein can exert its function. Suitable examples of the ligand at this time include an anti-transferrin receptor (TfR) antibody and an anti-insulin receptor (IR) antibody. In a case where such a conjugate or fusion protein is administered in vivo, the conjugate or fusion protein is taken up into cells and exhibits physiological activity in the cells, while the number of proteins present on muscle cells to which the conjugate or fusion protein binds, for example, TfR and IR, on the cells is not greatly reduced.

In addition, in one embodiment of the present invention, a conjugate or fusion protein of a ligand having affinity for a protein present on a specific cell and the M6P low-modified protein binds to the specific cell and is then taken up into the cell, whereby the conjugate or fusion protein can exert its function. When such a conjugate or fusion protein is administered in vivo, the conjugate or fusion protein is taken up into the specific cell and exhibits physiological activity in the cell, while the number of proteins present on the cell to which the conjugate or fusion protein binds is not greatly reduced.

In one embodiment of the present invention, a conjugate or fusion protein of a ligand having affinity for proteins present on a wide variety of cells and the M6P low-modified protein binds to a wide variety of cells and is then taken up into the cells, whereby the conjugate or fusion protein can exert its function. In a case where such a conjugate or fusion protein is administered in vivo, the conjugate or fusion protein is taken up into a wide variety of cells and exhibits physiological activity in a variety of tissues, while the number of proteins present on the cells to which the conjugate or fusion protein binds is not greatly reduced.

Hereinafter, an antibody serving as a ligand in one embodiment of the present invention will be described in detail. In the present invention, the term "antibody" mainly refers to a human antibody, a mouse antibody, a humanized antibody, an antibody derived from a camelid (including an alpaca), a chimeric antibody of a human antibody and an antibody of another mammal, and a chimeric antibody of a mouse antibody and an antibody of another mammal, but is not limited thereto as long as it has a property of specifically binding to a specific antigen, and may be an antibody fragment, an antigen-binding peptide, or the like. Further, in a case where the antibody is derived from an animal, the animal species is not particularly limited.

In the present invention, the term "human antibody" refers to an antibody whose protein is entirely encoded by a human-derived gene. However, an antibody encoded by a gene obtained by adding a mutation to an original human gene for the purpose, for example, of increasing the expression efficiency of the gene without changing the original amino acid sequence is also encompassed in the "human antibody". In addition, an antibody prepared by combining two or more genes encoding the human antibody to replace a part of one human antibody with a part of another human antibody is also a "human antibody". The human antibody often has three complementarity determining regions (CDRs) of a light chain and three complementarity determining regions (CDRs) of a heavy chain. The three CDRs of the light chain are referred to as CDR1, CDR2, and CDR3 in the order from the N-terminus side. The three CDRs of the heavy chain are also referred to as CDR1, CDR2, and CDR3 in the order from the N-terminus side. An antibody in which the antigen specificity, affinity, and the like of a human antibody are modified by replacing a CDR of one human antibody with a CDR of another human antibody is also encompassed in the "human antibody".

In the present invention, an antibody in which a mutation such as substitution, deletion, or addition is added to the amino acid sequence of the original antibody by modifying the gene of the original human antibody is also encompassed in the "human antibody". In a case where an amino acid or amino acids in the amino acid sequence of the original antibody is substituted with another amino acid or other amino acids, the number of amino acids to be substituted is preferably from 1 to 20, more preferably from 1 to 5, and even more preferably from 1 to 3. In a case where an amino acid or amino acids in the amino acid sequence of the original antibody is deleted, the number of amino acids to be deleted is preferably from 1 to 20, more preferably from 1 to 5, and even more preferably from 1 to 3. In addition, an antibody to which a mutation combining substitution and deletion of these amino acids is added is also the human antibody. In a case where an amino acid or amino acids is/are added, preferably from 1 to 20, more preferably from 1 to 5, and even more preferably from 1 to 3 amino acids are added into the amino acid sequence or to the N-terminus or the C-terminus of the original antibody. An antibody to which a mutation combining addition, substitution, and deletion of these amino acids is added is also the human antibody. The amino acid sequence of the mutated antibody preferably exhibits an identity of 80% or more, more preferably an identity of 90% or more, even more preferably an identity of 95% or more, and still more preferably an identity of 98% or more, to the amino acid sequence of the original antibody. In a case where the above-described mutation is added to a human antibody, the mutation can be added to a variable region of the antibody. In a case where the above-described mutation is added to the variable region of an antibody, the mutation may be added to any of a CDR and a framework region of the variable region, but is particularly added to the framework region. That is, the term "human-derived gene" in the present invention also encompasses a gene obtained by modifying the original human-derived gene, in addition to the original human-derived gene.

In the present invention, the term "humanized antibody" refers to an antibody in which the amino acid sequence of a part of the variable region (e.g., in particular, all, or a part of the CDRs) is derived from a non-human mammal and another region is derived from human. Examples of the humanized antibody include an antibody prepared by replacing the three complementarity determining regions (CDRs) of a light chain and the three complementarity determining regions (CDRs) of a heavy chain constituting a human antibody with CDRs of another mammal. The species of another mammal from which the CDRs to be grafted at an appropriate position of the human antibody is derived is any non-human mammal without particular limitation but is preferably a mouse, a rat, a rabbit, a horse, or a non-human primate, more preferably a mouse and a rat, and even more preferably a mouse. In addition, an antibody obtained by adding a mutation similar to the mutation that can be added to the above-described human antibody to the amino acid sequence of the original humanized antibody is also encompassed in the "humanized antibody".

In the present invention, the term "chimeric antibody" refers to an antibody in which fragments of two or more different antibodies derived from two or more different species are linked.

A chimeric antibody between a human antibody and an antibody of another mammal is an antibody in which a part of a human antibody is replaced with a part of an antibody of a mammal other than human. In a case where an antibody includes an Fc region, a Fab region, and a hinge region, which will be described below, specific examples of such a chimeric antibody include a chimeric antibody in which the Fc region is derived from a human antibody, while the Fab region is derived from an antibody of another mammal. The hinge region is derived from either a human antibody or an antibody of another mammal. Conversely, a chimeric antibody in which the Fc region is derived from another mammal while the Fab region is derived from a human antibody is included. The hinge region is derived from either a human antibody or an antibody of another mammal.

It can also be said that an antibody is composed of a variable region and a constant region. Other specific examples of the chimeric antibody include those in which the constant region (C_{H}) of the heavy chain and the constant region (C_{L}) of the light chain are derived from a human antibody, while the variable region (V_{H}) of the heavy chain and the variable region (V_{L}) of the light chain are derived from an antibody of another mammal, and, conversely, those in which the constant region (C_{H}) of the heavy chain and the constant region (C_{L}) of the light chain are derived from an antibody of another mammal, while the variable region (V_{H}) of the heavy chain and the variable region (V_{L}) of the light chain are derived from a human antibody. The organism species of another mammal is any non-human mammal without particular limitation, but is preferably a mouse, a rat, a rabbit, a horse, or a non-human primate, and for example, a mouse.

The antibody in one embodiment of the present invention has a basic structure composed of a total of four polypeptide chains, i.e., two immunoglobulin light chains (or simply "light chains") and two immunoglobulin heavy chains (or simply "heavy chains"). However, in the present invention, the term "antibody" encompasses, in addition to an antibody having this basic structure:
(1) an antibody composed of a total of two polypeptide chains, i.e., one light chain and one heavy chain;
(2) an antibody composed of the Fab region, which is obtained by deleting the Fc region from the basic structure of the original antibody, and an antibody composed of all or part of the Fab region and the hinge region (Fab, F(ab'), and F(ab'))₂:
(3) a single chain antibody in which a linker sequence is bound to the C-terminus side of a light chain and a heavy chain is further bound to the C-terminus side thereof;
(4) a single chain antibody in which a linker sequence is bound to the C-terminus side of a heavy chain and a light chain is further bound to the C-terminus side thereof;
(5) an antibody composed of the Fc region, which is obtained by deleting the Fab region from the basic structure of the original antibody, wherein the amino acid sequence of the Fc region is modified to have a property of specifically binding to a specific antigen (Fc antibody); and
(6) a single domain antibody described below.

The antibody in one embodiment of the present invention is an antibody derived from a camelid (including alpaca). Some camelid antibodies are composed of two heavy chains linked by a disulfide bond. The antibody composed of two heavy chains is called a heavy chain antibody. The VHH is an antibody composed of one heavy chain containing the variable region of the heavy chain constituting the heavy chain antibody or an antibody composed of one heavy chain lacking the constant region (CH) constituting the heavy chain antibody. The VHH is also one of the antibodies in the embodiments of the present invention. In addition, an antibody composed of two light chains linked by a disulfide bond is also one of the antibodies in the embodiments of the present invention. The antibody composed of two light chains is referred to as a light chain antibody. An antibody obtained by adding a mutation to the amino acid sequence of an antibody derived from a camelid, for reducing the antigenicity of an antibody derived from a camelid (including VHH) when administered to a human, is also the antibody according to one embodiment of the present invention. When a mutation is added to the amino acids of a camelid antibody, a mutation can be added that is similar to the mutation that can be added to the antibodies described herein.

The antibody in one embodiment of the present invention is an antibody derived from shark. The shark antibody is composed of two heavy chains linked by a disulfide bond. The antibody composed of two heavy chains is called a heavy chain antibody. The VNAR is an antibody composed of one heavy chain containing the variable region of the heavy chain constituting the heavy chain antibody or an antibody composed of one heavy chain lacking the constant region (CH) constituting the heavy chain antibody. The VNAR is also one of the antibodies in the embodiments of the present invention. An antibody obtained by adding a mutation to the amino acid sequence of an antibody derived from shark, for reducing the antigenicity of an antibody derived from shark (including VNAR) when administered to a human, is also the antibody according to one embodiment of the present invention. When a mutation is added to the amino acids of a shark antibody, a mutation can be added that is similar to the mutation that can be added to the antibodies described herein. A humanized shark antibody is also one of the antibodies in the embodiments of the present invention.

An antibody having a basic structure composed of a total of four polypeptide chains, i.e., two light chains and two heavy chains has three complementarity determining regions (CDRs) in the variable region of the light chain (V_{L}) and three complementarity determining regions (CDRs) in the variable region of the heavy chain (V_{H}). The three CDRs of the light chain are referred to as CDR1, CDR2, and CDR3 in the order from the N-terminus side. The three CDRs of the heavy chain are also referred to as CDR1, CDR2, and CDR3 in the order from the N-terminus side. However, even if a part or all of these CDRs are incomplete or absent, they are encompassed in the antibody as long as they have a property of specifically binding to a specific antigen. The regions, other than the CDRs, of the light and heavy chain variable regions (V_{L} and V_{H}) are referred to as framework regions (FRs). The FRs are referred to as FR1, FR2, FR3, and FR4 in order from the N-terminus side. Usually, the CDRs and FRs are present in the order of FR1, CDR1, FR2, CDR2, FR3, CDR3, and FR4 from the N-terminus side.

In one embodiment of the present invention, an antibody obtained by adding a mutation such as substitution, deletion, or addition to the amino acid sequence of the original antibody is also encompassed in the antibody. In a case where an amino acid or amino acids in the amino acid sequence of the original antibody is substituted with another amino acid or other amino acids, the number of amino acids to be substituted is preferably from 1 to 20, more preferably from 1 to 5, and even more preferably from 1 to 3. In a case where an amino acid or amino acids in the amino acid sequence of the original antibody is deleted, the number of amino acids to be deleted is preferably from 1 to 20, more preferably from 1 to 5, and even more preferably from 1 to 3. In addition, an antibody to which a mutation combining substitution and deletion of these amino acids is added is also the antibody. In a case where an amino acid or amino acids is/are added, preferably from 1 to 20, more preferably from 1 to 5, and even more preferably from 1 to 3 amino acids are added into the amino acid sequence or to the N-terminus or the C-terminus of the original antibody. An antibody to which a mutation combining addition, substitution, and deletion of these amino acids is added is also the antibody. The amino acid sequence of the mutated antibody preferably exhibits an identity of 80% or more, more preferably exhibits an identity of 85% or more, and even more preferably exhibits an identity of 90% or more, 95% or more, or 98% or more, to the amino acid sequence of the original antibody.

In one embodiment of the present invention, an antibody obtained by adding a mutation such as substitution, deletion, or addition to the amino acid sequence of the variable region of the original antibody is also encompassed in the antibody. In a case where an amino acid or amino acids in the amino acid sequence of the original antibody is substituted with another amino acid or other amino acids, the number of amino acids to be substituted is preferably from 1 to 10, more preferably from 1 to 5, and even more preferably from 1 to 3. In a case where an amino acid or amino acids in the amino acid sequence of the original antibody is deleted, the number of amino acids to be deleted is preferably from 1 to 10, more preferably from 1 to 5, and even more preferably from 1 to 3. In addition, an antibody to which a mutation combining substitution and deletion of these amino acids is added is also the antibody. When an amino acid or amino acids is/are added, preferably from 1 to 10, more preferably from 1 to 5, and even more preferably from 1 to 3 amino acids are added into the amino acid sequence or to the N-terminus or the C-terminus of the original antibody. An antibody to which a mutation combining addition, substitution, and deletion of these amino acids is added is also the antibody. The amino acid sequence of the mutated antibody preferably exhibits an identity of 80% or more, more preferably exhibits an identity of 85% or more, and even more preferably exhibits an identity of 90% or more, 95% or more, or 98% or more, to the amino acid sequence of the original antibody. In a case where a mutation is added to a variable region of an antibody, the mutation may be added to any of a CDR and a framework region of the variable region, but is particularly added to the framework region.

In one embodiment of the present invention, an antibody obtained by adding a mutation such as substitution, deletion, or addition to the amino acid sequence of the framework region of the variable region of the original antibody is also encompassed in the antibody. In a case where an amino acid or amino acids in the amino acid sequence of the original antibody is substituted with another amino acid or other amino acids, the number of amino acids to be substituted is preferably from 1 to 10, more preferably from 1 to 5, and even more preferably from 1 to 3. In a case where an amino acid or amino acids in the amino acid sequence of the original antibody is deleted, the number of amino acids to be deleted is preferably from 1 to 10, more preferably from 1 to 5, and even more preferably from 1 to 3. In addition, an antibody to which a mutation combining substitution and deletion of these amino acids is added is also the antibody. When an amino acid or amino acids is/are added, preferably from 1 to 10, more preferably from 1 to 5, and even more preferably from 1 to 3 amino acids are added into the amino acid sequence or to the N-terminus or the C-terminus of the original antibody. An antibody to which a mutation combining addition, substitution, and deletion of these amino acids is added is also the antibody. The amino acid sequence of the mutated antibody preferably exhibits an identity of 80% or more, more preferably exhibits an identity of 85% or more, and even more preferably exhibits an identity of 90% or more, 95% or more, or 98% or more, to the amino acid sequence of the original antibody.

In one embodiment of the present invention, an antibody obtained by adding a mutation such as substitution, deletion, or addition to the amino acid sequence of the CDR region of the variable region of the original antibody is also encompassed in the antibody. In a case where an amino acid or amino acids in the amino acid sequence of the original antibody is substituted with another amino acid or other amino acids, the number of amino acids to be substituted is preferably from 1 to 5, more preferably from 1 to 3, and even more preferably 1 or 2. In a case where an amino acid or amino acids in the amino acid sequence of the original antibody is deleted, the number of amino acids to be deleted is preferably from 1 to 5, more preferably from 1 to 3, and even more preferably 1 or 2. In addition, an antibody to which a mutation combining substitution and deletion of these amino acids is added is also the antibody. In a case where an amino acid or amino acids is/are added, preferably from 1 to 5, more preferably from 1 to 3, and even more preferably 1 or 2 amino acids are added into the amino acid sequence or to the N-terminus or the C-terminus of the original antibody. An antibody to which a mutation combining addition, substitution, and deletion of these amino acids is added is also the antibody. The amino acid sequence of the mutated antibody preferably exhibits an identity of 80% or more, more preferably exhibits an identity of 85% or more, and even more preferably exhibits an identity of 90% or more, 95% or more, or 98% or more, to the amino acid sequence of the original antibody.

The identity of the amino acid sequence of the original antibody to the amino acid sequence of a mutated antibody can be easily calculated using a known identity calculation algorithm. Examples of such an algorithm include BLAST (Altschul SF. J Mol. Biol. 215. 403-10, (1990)), the similarity search method of Pearson and Lipman (Proc. Natl. Acad. Sci. USA. 85. 2444 (1988)), and the local homology algorithm of Smith and Waterman (Adv. Appl. Math. 2. 482-9 (1981)).

In one embodiment of the present invention, the Fab refers to a molecule in which one light chain containing a variable region and a constant region of the light chain (C_{L} region) and one heavy chain containing a variable region and part 1 of a constant region of the heavy chain (C_{H}1 region) are linked by a disulfide bond between cysteine residues present in each chain. In the Fab, the heavy chain may further contain part of a hinge region in addition to the variable region and the part 1 of the constant region of the heavy chain (C_{H}1 region), but in this case the hinge region lacks a cysteine residue that is otherwise present in the hinge region and links the heavy chains of the antibody to each other. In the Fab, the light chain and the heavy chain are linked by a disulfide bond formed between a cysteine residue present in the constant region (C_{L} region) of the light chain and a cysteine residue present in the constant region (C_{H}1 region) or the hinge region of the heavy chain. The heavy chain forming the Fab is referred to as a Fab heavy chain. The Fab lacks the cysteine residue, which is otherwise present in the hinge region and links the heavy chains of the antibody to each other, and thus is composed of one light chain and one heavy chain. The light chain constituting the Fab (Fab light chain) contains the variable region and the C_{L} region. The heavy chain constituting the Fab may be composed of the variable region and the C_{H}1 region or may contain part of the hinge region in addition to the variable region and the C_{H}1 region. In this case, however, the hinge region is selected to contain no cysteine residue for linking between the heavy chains and thus does not form a disulfide bond between the two heavy chains in the hinge region. In F(ab'), the heavy chain contains, in addition to the variable region and the C_{H}1 region, all or a part of the hinge region containing a cysteine residue that links the heavy chains. F(ab')₂ refers to a molecule in which two F(ab')s are bound by a disulfide bond between cysteine residues present in their hinge regions. The heavy chain forming F(ab') or F(ab')₂ is referred to as a Fab' heavy chain. Further, a polymer such as a dimer or a trimer formed by binding a plurality of antibodies directly or via a linker is also an antibody. Furthermore, without being limited to these, the "antibody" referred to in the present invention encompasses any substance that contains a part of an antibody molecule and has the property of specifically binding to an antigen. That is, the "light chain" referred to in the present invention encompasses those derived from a light chain and having an amino acid sequence of all or a part of the variable region thereof. Also, the "heavy chain" encompasses those derived from a heavy chain and having an amino acid sequence of all or a part of the variable region thereof. Therefore, for example, a substance lacking the Fc region is also the heavy chain, as long as it has an amino acid sequence of all or a part of the variable region.

As used herein, the term "Fc" or "Fc region" refers to a region containing a fragment composed of the C_{H}2 region (part 2 of the constant region of the heavy chain) and the C_{H}3 region (part 3 of the constant region of the heavy chain) in an antibody molecule.

Furthermore, an antibody according to one embodiment of the present invention also encompasses
(7) scFab, scF(ab'), and scF(ab')₂ in which a light chain and a heavy chain constituting Fab, F(ab'), or F(ab')₂ indicated in (2) above are bound via a linker sequence to form single chain antibodies, respectively. Here, in scFab, scF(ab'), and scF(ab')₂, the linker sequence may be bound to the C-terminus side of the light chain, and the heavy chain may be further bound to the C-terminus side thereof, or the linker sequence may be bound to the C-terminus side of the heavy chain, and the light chain may be further bound to the C-terminus side thereof. Furthermore, scFv in which a variable region of a light chain and a variable region of a heavy chain are linked via a linker to form a single chain antibody is also encompassed in the antibody of the present invention. In the scFv, the linker may be bound to the C-terminus side of the variable region of the light chain and the variable region of the heavy chain may be further bound to the C-terminus side thereof, or the linker may be bound to the C-terminus side of the variable region of the heavy chain and the variable region of the light chain may be further bound to the C-terminus side thereof.

Furthermore, the "antibody" referred to herein encompasses, in addition to full-length antibodies and those described in (1) to (7) above, all form of antigen binding fragment (antibody fragment) in which a part of a full-length antibody is deleted, which is a broader concept including (1) to (7). The antigen binding fragment also encompasses heavy chain antibodies, light chain antibodies, VHH, VNAR, and partially deleted versions thereof.

The term "antigen binding fragment" refers to a fragment of an antibody that retains at least a part of its specific binding activity with an antigen. Examples of the binding fragment include Fab, Fab', F(ab')₂, a variable region (Fv), a single chain antibody (scFv) in which a heavy chain variable region (V_{H}) and a light chain variable region (V_{L}) are linked by an appropriate linker, a diabody that is a dimer of a polypeptide including a heavy chain variable region (V_{H}) and a light chain variable region (V_{L}), a minibody that is a dimer of a substance in which a part of a constant region (C_{H}3) binds to a heavy chain (H chain) of scFv, and other low-molecular-weight antibodies. However, it is not limited to these molecules as long as it has the ability to bind to an antigen.

In one embodiment of the present invention, the term "single chain antibody" refers to a protein in which a linker sequence binds to the C-terminus side of an amino acid sequence including all or a part of a variable region of a light chain, and an amino acid sequence including all or a part of a variable region of a heavy chain further binds to the C-terminus side thereof, and which can specifically bind to a specific antigen. A protein in which a linker sequence binds to the C-terminus side of an amino acid sequence including all or a part of a variable region of a heavy chain, and an amino acid sequence including all or a part of a variable region of a light chain further binds to the C-terminus side thereof, and which can specifically bind to a specific antigen is also the "single chain antibody" in the present invention. In a single chain antibody in which a light chain binds to the C-terminus side of a heavy chain via a linker sequence, the heavy chain usually lacks the Fc region. The variable region of a light chain has three complementarity determining regions (CDRs) that are responsible for the antigen specificity of an antibody. Similarly, the variable region of a heavy chain has three CDRs. These CDRs are main regions that determine the antigen specificity of an antibody. Thus, the single chain antibody preferably contains all three CDRs of a heavy chain and all three CDRs of a light chain. However, as long as the antigen-specific affinity of the antibody is maintained, a single chain antibody in which one or more CDRs are deleted can also be used.

In the single chain antibody, the linker sequence disposed between the light chain and the heavy chain of an antibody is a peptide chain composed of preferably from 2 to 50, more preferably from 8 to 50, even more preferably from 10 to 30, still more preferably from 12 to 18 or from 15 to 25, and, for example, from 15 or 25 amino acid residues. Such a linker sequence is not limited in its amino acid sequence as long as the single chain antibody formed by linking both chains thereby retains the affinity for the antigen, but is preferably composed of glycine alone or glycine and serine, and has, for example, an amino acid sequence Gly-Ser, an amino acid sequence Gly-Gly-Ser, an amino acid sequence Gly-Gly-Gly, an amino acid sequence Gly-Gly-Gly-Gly-Ser (SEQ ID NO: 25), an amino acid sequence Gly-Gly-Gly-Gly-Gly-Ser (SEQ ID NO: 26), an amino acid sequence Ser-Gly-Gly-Gly-Gly (SEQ ID NO: 27), or a sequence in which these amino acid sequences are repeated 2 to 10 times or 2 to 5 times. For example, in a case where a variable region of a light chain is bound via a linker to the C-terminus side of the amino acid sequence composed of the entire region of a variable region of a heavy chain, a linker sequence containing a total of 15 amino acids corresponding to three consecutive amino acid sequences Gly-Gly-Gly-Gly-Ser (SEQ ID NO: 25) is suitable.

In one embodiment of the present invention, the "single domain antibody" refers to an antibody having the property of specifically binding to an antigen in a single variable region. The single domain antibody encompasses antibodies whose variable region consists only of the variable region of the heavy chain (heavy chain single domain antibodies) and antibodies whose variable region consists only of the variable region of the light chain (light chain single domain antibodies). VHH and VNAR are types of single domain antibodies.

In one embodiment of the present invention, the term "human transferrin receptor" refers to a membrane protein having an amino acid sequence represented by SEQ ID NO: 28. In one embodiment, the antibody of the present invention specifically binds to a portion (extracellular region of transferrin receptor) from cysteine residue at position 89 from the N-terminus side to phenylalanine at the C-terminus in the amino acid sequence represented by SEQ ID NO: 28, but is not limited thereto.

As a method for preparing an antibody against a desired protein, a method is generally used in which a cell into which an expression vector incorporating a gene encoding the protein is introduced is used to prepare a recombinant protein, and the recombinant protein is used to immunize an animal such as a mouse. An antibody-producing cell for the recombinant protein is taken out from the immunized animal, and the antibody-producing cell is fused with a myeloma cell, whereby a hybridoma cell capable of producing an antibody against the recombinant protein can be prepared.

Alternatively, when an immune-system cell obtained from an animal such as a mouse is immunized by a desired protein using an in vitro immunization method, a cell producing an antibody against the desired protein can also be obtained. In a case of using the in vitro immunization method, the animal species from which the immune-system cell is derived is not particularly limited, but is preferably a mouse, a rat, a rabbit, a guinea pig, a dog, a cat, a horse, or a primate including a human, more preferably a mouse, a rat, or a human, and even more preferably a mouse or a human. As the immune-system cell of a mouse, for example, spleen cells prepared from the spleen of a mouse can be used. As the immune-system cell of a human, cells prepared from the peripheral blood, bone marrow, spleen, and the like of a human can be used. In a case where the immune-system cell of a human is immunized by the in vitro immunization method, a human antibody to the recombinant protein can be obtained.

Hybridoma cells capable of producing an antibody can be prepared by immunizing immune-system cells by the in vitro immunization method and then fusing the immunized cells with myeloma cells. Alternatively, an antibody gene can be artificially reconstructed by extracting mRNA from the immunized cell to synthesize cDNA, amplifying a DNA fragment containing a gene encoding light and heavy chains of immunoglobulin by a PCR reaction using the cDNA as a template, and using the amplified DNA fragment.

The hybridoma cells as obtained by the above-described method also encompass a cell that produces an antibody that recognizes a non-target protein as an antigen. In addition, not all hybridoma cells that produce an antibody against a desired protein produce an antibody that exhibits desired properties, such as high affinity for the protein.

Similarly, artificially reconstructed antibody genes also encompass a gene encoding an antibody that recognizes a non-target protein as an antigen. In addition, not all genes encoding an antibody against a desired protein encode an antibody that exhibits desired properties, such as high affinity for the protein.

Thus, a step of selecting a hybridoma cell producing an antibody having desired properties from the hybridoma cells obtained as described above is required. In addition, in the artificially reconstructed antibody genes, a step of selecting a gene encoding an antibody having desired properties from the antibody gene is required. For example, as a method for selecting a hybridoma cell producing an antibody exhibiting high affinity for a desired protein (high-affinity antibody) or a gene encoding a high-affinity antibody, a method described in detail below is effective.

For example, in a case of selecting a hybridoma cell producing an antibody having high affinity for a desired protein, a method is used in which the protein is added to a plate to be retained thereon, a culture supernatant of the hybridoma cell is added thereto, an antibody not binding to the protein is removed from the plate, and the amount of the antibody retained on the plate is measured. According to this method, the higher the affinity of the antibody contained in the culture supernatant of the hybridoma cell added to the plate for the protein, the larger the amount of the antibody retained on the plate. Thus, the amount of the antibody retained on the plate is measured, and the hybridoma cell corresponding to the plate retaining a larger amount of the antibody can be selected as a cell line producing an antibody having relatively high affinity for the protein. From the cell line thus selected, a gene encoding a high-affinity antibody can also be isolated by extracting mRNA to synthesize cDNA, and amplifying a DNA fragment containing a gene encoding an antibody against the protein by a PCR method using the cDNA as a template.

In a case where a gene encoding an antibody against a protein of interest having high affinity is selected from the above-described artificially reconstructed antibody gene, the artificially reconstructed antibody gene is once incorporated into an expression vector, and the expression vector is introduced into a host cell. At this time, a cell used as the host cell is not particularly limited, whether it is a prokaryotic cell or a eukaryotic cell, as long as it can express the antibody gene by introducing an expression vector incorporating the artificially reconstructed antibody gene, but a cell derived from a mammal such as a human, a mouse, and a Chinese hamster is preferred, and a CHO cell derived from a Chinese hamster ovary or an NS/0 cell derived from mouse myeloma is particularly preferred. The expression vector used for incorporating and expressing a gene encoding an antibody gene is not particularly limited as long as it expresses the gene when introduced into a mammalian cell. The gene incorporated into the expression vector is located downstream of a DNA sequence (gene expression regulatory site) capable of regulating the frequency of transcription of the gene in mammalian cells. Examples of the gene expression regulatory site that can be used in the present invention include a cytomegalovirus-derived promoter, an SV40 early promoter, a human elongation factor-1α (EF-1α) promoter, and a human ubiquitin C promoter.

A mammalian cell into which such an expression vector is introduced expresses the above-described artificially reconstructed antibody incorporated into the expression vector. In a case where a cell producing an antibody having high affinity for an antibody against a desired protein is selected from the cell expressing the artificially reconstructed antibody thus obtained, a method is used in which the protein is added to a plate to be retained thereon, a culture supernatant of the cell is brought into contact with the protein, an antibody not binding to the protein is removed from the plate, and the amount of the antibody retained on the plate is measured. According to this method, when the affinity of the antibody contained in the culture supernatant of the cell for the protein is higher, the amount of the antibody retained on the plate is increased. Accordingly, the amount of the antibody retained on the plate is measured, and the cell corresponding to the plate retaining a larger amount of the antibody can be selected as the cell line producing the antibody having relatively high affinity for the protein, and thus the gene encoding the antibody having high affinity for the protein can be selected. From the cell line thus selected, a gene encoding a high-affinity antibody can also be isolated by amplifying a DNA fragment containing a gene encoding an antibody against the protein using a PCR method.

As a method for producing a fusion protein of the M6P low-modified protein and an antibody in the present invention, there is a method of binding the M6P low-modified protein and the antibody via a non-peptide linker or a peptide linker. As the non-peptide linker, polyethylene glycol, polypropylene glycol, a copolymer of ethylene glycol and propylene glycol, polyoxyethylated polyol, polyvinyl alcohol, a polysaccharide, dextran, polyvinyl ether, a biodegradable polymer, a lipid polymer, a chitin, hyaluronic acid, or a derivative thereof, or a combination thereof can be used. The peptide linker is a peptide chain composed of 1 to 50 amino acids binding to each other by peptide bonds or a derivative thereof, and binds the M6P low-modified protein to an antibody by forming covalent bonds at its N-terminus and C-terminus with either the M6P low-modified protein or the antibody, respectively.

The antibody and the M6P low-modified protein can be bound to each other by binding the N-terminus or C-terminus of the M6P low-modified protein respectively to the C-terminus side or N-terminus side of a heavy chain or light chain by a peptide bond via a linker sequence or directly. The fusion protein obtained by binding the antibody and the M6P low-modified protein in this manner can be produced as a fusion protein by incorporating a DNA fragment in which cDNA encoding the M6P low-modified protein is disposed in frame on the 3' end side or 5' end side of cDNA encoding the heavy chain or light chain of the antibody directly or via cDNA encoding a linker sequence into an expression vector for a mammalian cell, and culturing the mammalian cell into which the expression vector has been introduced. In this mammalian cell, in a case where the DNA fragment encoding the M6P low-modified protein is bound to the heavy chain, an expression vector for a mammalian cell into which a cDNA fragment encoding the light chain of the antibody has been incorporated can also be introduced into the same host cell, and in a case where the DNA fragment encoding the M6P low-modified protein is bound to the light chain, an expression vector for a mammalian cell into which a cDNA fragment encoding the heavy chain of the antibody has been incorporated can also be introduced into the same host cell. In a case where the antibody is a single chain antibody, a fusion protein in which the antibody is bound to the M6P low-modified protein can be obtained by incorporating a DNA fragment in which cDNA encoding the single chain antibody is linked to the 5' end side or 3' end side of cDNA encoding the M6P low-modified protein directly or via a DNA fragment encoding a linker sequence into an expression vector (for a eukaryotic cell such as a mammalian cell or a yeast, or a prokaryotic cell such as E. coli), and expressing the fusion protein in the cell into which the expression vector has been introduced. The fusion protein of the antibody and the M6P low-modified protein can be produced as a recombinant protein by the above-described methods.

The medium for culturing the mammalian cell into which the expression vector has been introduced is not particularly limited as long as the mammalian cell can be cultured and proliferated, but a serum-free medium is preferably used. In the present invention, as the serum-free medium to be used as a medium for production of recombinant protein, there is suitably used a medium containing amino acid at 3 to 700 mg/L, vitamins at 0.001 to 50 mg/L, monosaccharide at 0.3 to 10 g/L, inorganic salt at 0.1 to 10000 mg/L, trace elements at 0.001 to 0.1 mg/L, nucleoside at 0.1 to 50 mg/L, fatty acid at 0.001 to 10 mg/L, biotin at 0.01 to 1 mg/L, hydrocortisone at 0.1 to 20 µg/L, insulin at 0.1 to 20 mg/L, vitamin B12 at 0.1 to 10 mg/L, putrescine at 0.01 to 1 mg/L, sodium pyruvate at 10 to 500 mg/L, and a water-soluble iron compound. If desired, thymidine, hypoxanthine, a pH indicator and an antibiotic in the related art, and the like may be added to the medium.

As a serum-free medium used as a medium for the production of the fusion protein of the antibody and the M6P low-modified protein, a DMEM/F12 medium (mixed medium of DMEM and F12) may be used as a basal medium, and these media are well known to those skilled in the art. A DMEM (HG) HAM-modified (R5) medium containing sodium hydrogen carbonate, L-glutamine, D-glucose, insulin, sodium selenite, diaminobutane, hydrocortisone, iron sulfate (II), asparagine, aspartic acid, serine, and polyvinyl alcohol may also be used as a serum-free medium. Furthermore, commercially available serum-free media such as CD OptiCHO (trade name) medium, CHO-S-SFM II medium, or CD CHO medium (Thermo Fisher Scientific Inc., Old Life Technologies Corporation), EX-CELL (trade name) Advanced medium, EX-CELL (trade name) 302 medium, or EX-CELL (trade name) 325-PF medium (SAFC Biosciences Inc.) can also be used as a basal medium.

Preferred embodiments of the fusion protein of the antibody and the M6P low-modified protein include the following (1) to (4). That is:
(1) a fusion protein including a conjugate in which the M6P low-modified protein binds to the C-terminus of the heavy chain of the antibody directly or via a linker, and the light chain of the antibody;
(2) a fusion protein including a conjugate in which the M6P low-modified protein binds to the N-terminus of the heavy chain of the antibody directly or via a linker, and the light chain of the antibody;
(3) a fusion protein including a conjugate in which the M6P low-modified protein binds to the C-terminus of the light chain of the antibody directly or via a linker, and the heavy chain of the antibody; and
(4) a fusion protein including a conjugate in which the M6P low-modified protein binds to the N-terminus of the light chain of the antibody directly or via a linker, and the heavy chain of the antibody.

In a case where the antibody and the M6P low-modified protein are bound to each other via a linker sequence, the linker sequence to be disposed between the antibody and the M6P low-modified protein is a peptide chain composed of preferably from 1 to 60 or from 1 to 50 amino acids, more preferably from 1 to 17 amino acids, even more preferably from 1 to 10 amino acids, and still more preferably from 1 to 5 amino acids. The number of amino acids constituting the linker sequence can be appropriately adjusted to 1, 2, 3, from 1 to 17, from 1 to 10, from 10 to 40, from 20 to 34, from 23 to 31, from 25 to 29, 27, or the like. The amino acid sequence of such a linker sequence is not particularly limited as long as the antibody linked by the linker sequence retains the affinity for the receptor on the cerebrovascular endothelial cell, and the M6P low-modified protein linked by the linker sequence can exhibit the physiological activity of the protein under physiological conditions. The amino acid sequence preferably includes glycine and serine. Examples thereof include those including any one amino acid of glycine or serine, the amino acid sequence Gly-Ser, the amino acid sequence Ser-Ser, the amino acid sequence Gly-Gly-Ser, the amino acid sequence Gly-Gly-Gly, the amino acid sequence Gly-Gly-Gly-Gly-Ser (SEQ ID NO: 25), the amino acid sequence Gly-Gly-Gly-Gly-Gly-Ser (SEQ ID NO: 26), the amino acid sequence Ser-Gly-Gly-Gly-Gly (SEQ ID NO: 27), and those including a sequence composed of 1 to 10, or 2 to 5 of these amino acid sequences in series. For example, the amino acid sequence has a sequence composed of from 1 to 50 amino acids, a sequence composed of from 2 to 17, from 2 to 10, from 10 to 40, from 20 to 34, from 23 to 31, from 25 to 29, or 27 amino acids, or the like. For example, a sequence including the amino acid sequence Gly-Ser can be suitably used as the linker sequence. In addition, a sequence including a total of 27 amino acid sequences in which the amino acid sequence Gly-Ser is followed by five consecutive amino acid sequences Gly-Gly-Gly-Gly-Ser (SEQ ID NO: 25) can be suitably used as the linker sequence. Furthermore, a sequence containing a total of 25 amino acid sequences composed of five consecutive amino acid sequences Gly-Gly-Gly-Gly-Ser (SEQ ID NO: 25) can also be suitably used as the linker sequence.

Note that in the present invention, in a case where a plurality of linker sequences are contained in one peptide chain, for convenience, the linker sequences are named as a first linker sequence, a second linker sequence, and the like in this order from the N-terminus side.

Specific examples of the antibody to be bound to the M6P low-modified protein of the present invention include the following anti-human transferrin receptor antibodies (anti-hTfR antibodies). That is,
(1) an anti-hTfR antibody, wherein the light chain of the antibody includes the amino acid sequence of SEQ ID NO: 29 and the heavy chain includes the amino acid sequence of SEQ ID NO: 30; and
(2) an anti-hTfR antibody and a Fab antibody, wherein the light chain of the antibody includes the amino acid sequence of SEQ ID NO: 29 and the heavy chain includes the amino acid sequence of SEQ ID NO: 31. However, the anti-hTfR antibody is not limited thereto, and mutations such as substitution, deletion, and addition can be appropriately added to the amino acid sequence. Note that the anti-hTfR antibodies described in (1) and (2) above are humanized anti-hTfR antibodies.

In substitution of an amino acid or amino acids in the amino acid sequence of the light chain of the anti-human transferrin receptor antibody with another amino acid or other amino acids, the number of amino acids to be substituted is preferably from 1 to 10, more preferably from 1 to 5, even more preferably from 1 to 3, and still more preferably 1 or 2. In deletion of an amino acid or amino acids in the amino acid sequence of the light chain, the number of amino acids to be deleted is preferably from 1 to 10, more preferably from 1 to 5, even more preferably from 1 to 3, and still more preferably 1 or 2 amino acids. In addition, a mutation combining substitution and deletion of these amino acids can also be performed.

In addition of an amino acid or amino acids to the amino acid sequence of the light chain of the anti-human transferrin receptor antibody, preferably from 1 to 10, more preferably from 1 to 5, even more preferably from 1 to 3, and even more preferably 1 or 2 amino acids are added into the amino acid sequence, or to the N-terminus side or the C-terminus side of the light chain. A mutation combining addition, substitution, and deletion of these amino acids can also be performed. The amino acid sequence of the mutated light chain preferably exhibits an identity of 80% or more, more preferably exhibits an identity of 90% or more, and even more preferably exhibits an identity of 95% or more, to the amino acid sequence of the original light chain.

In substitution of an amino acid or amino acids in the amino acid sequence of the heavy chain of the anti-human transferrin receptor antibody with another amino acid or other amino acids, the number of amino acids to be substituted is preferably from 1 to 10, more preferably from 1 to 5, even more preferably from 1 to 3, and still more preferably 1 or 2 amino acids. In deletion of an amino acid or amino acids in the amino acid sequence of the heavy chain, the number of amino acids to be deleted is preferably from 1 to 10, more preferably from 1 to 5, even more preferably from 1 to 3, and still more preferably 1 or 2 amino acids. In addition, a mutation combining substitution and deletion of these amino acids can also be performed.

In addition of an amino acid or amino acids to the amino acid sequence of the heavy chain of the anti-human transferrin receptor antibody, preferably from 1 to 10, more preferably from 1 to 5, even more preferably from 1 to 3, and even more preferably 1 or 2 amino acids are added in the amino acid sequence, or to the N-terminus side or the C-terminus side of the heavy chain. A mutation combining addition, substitution, and deletion of these amino acids can also be performed. The amino acid sequence of the mutated heavy chain preferably exhibits an identity of 80% or more, more preferably exhibits an identity of 90% or more, and even more preferably exhibits an identity of 95% or more, to the amino acid sequence of the original heavy chain.

The M6P low-modified protein in one embodiment of the present invention can be used for the production of drugs for administration into blood for the treatment of a disease state of the central nervous system caused by a functional abnormality or deficiency of a protein because it can be made to pass through the blood-brain barrier and exert its function in the brain by being bound to an antibody against a receptor on a cerebrovascular endothelial cell. The protein bound to the antibody can be used in a method for treating a disease state of the central nervous system caused by a functional abnormality or deficiency of a protein, the method including administering a therapeutically effective amount of the protein into the blood of a corresponding patient (including intravenous injection such as intravenous drip). The M6P low-modified protein bound to the antibody administered into the blood can reach not only the brain but also other organs and parts. The drug may also be used to prevent the onset of the disease state.

The M6P low-modified protein can be used as a drug that should be administered into the blood to exert its efficacy in the central nervous system (CNS) by being bound to an antibody against a receptor on a cerebrovascular endothelial cell. Such a drug is generally administered to a patient by intravenous injection such as intravenous drip injection, subcutaneous injection, or intramuscular injection, but the administration route is not particularly limited.

The M6P low-modified protein in one embodiment of the present invention can be made into a fusion protein with not only an antibody but also another ligand. Such a fusion protein can also be obtained by the above-described method for producing a fusion protein with an antibody. The fusion protein of the M6P low-modified protein and another ligand can be produced as a recombinant protein by the above-described method for producing a fusion protein with an antibody. One embodiment of the present invention includes a gene encoding the fusion protein of the M6P low-modified protein and another ligand, an expression vector into which the gene is incorporated, and a host cell into which the expression vector is introduced.

The M6P low-modified protein in one embodiment of the present invention can be made into a conjugate with not only the anti-hTfR antibody but also a substance capable of binding to hTfR. The substance is not particularly limited and is, for example, human transferrin. Human transferrin is not limited to wild-type human transferrin, and may be a partial fragment or a mutant thereof as long as it has affinity for hTfR. Such a conjugate can pass through the blood-brain barrier and exert its function in the brain. The fusion protein of the M6P low-modified protein and human transferrin can be produced as a recombinant protein by the above-described method for producing a fusion protein with an antibody. One embodiment of the present invention includes a gene encoding the fusion protein of the M6P low-modified protein and human transferrin, an expression vector in which the gene is incorporated, and a host cell into which the expression vector is introduced.

In a case where a fusion protein of the wild-type protein having an N-linked sugar chain containing mannose-6-phosphate (M6P) and a ligand having affinity for a protein present on a cell (excluding an M6P receptor, referred to herein as "target protein") is administered in vivo, it is assumed that the fusion protein together with the target protein is taken up into the cell via an M6P receptor while the ligand portion retains the binding to the target protein. The target protein thus taken up into the cell together with the fusion protein is not recycled but is eventually decomposed in the lysosome. As a result, the number of target proteins present on the cell is reduced. In the M6P low-modified protein, the affinity between the protein and the M6P receptor is reduced, resulting in reduced binding between the protein and the M6P receptor. Thus, the fusion protein of the M6P low-modified protein and the ligand is less likely to be taken up into the cell together with the target protein via the M6P receptor. Accordingly, when instead of the fusion protein of a wild-type protein having N-linked sugar chains including mannose-6-phosphates (M6P), the fusion protein of the M6P low-modified protein and a ligand corresponding to the fusion protein is administered, the decrease in target protein present on a cell can be prevented.

Note that the conjugate or fusion protein of the M6P low-modified protein and the ligand can also be prepared by preparing a conjugate or fusion protein in a form containing M6Ps and then chemically treating it to delete at least one M6P or at least one N-linked sugar chain. The wording "chemically treating" means that a drug, catalyst, enzyme, or the like is caused to act on a glycoprotein to cause a decomposition reaction or a substitution reaction, thereby cleaving an M6P or an N-linked sugar chain from the glycoprotein. In a case where an enzyme is used at this time, for example, peptide N-glycanase, endo-β-N-acetylglucosaminidase or endo-β-mannosidase, which are known as endo-type enzymes acting around the site where an N-linked sugar chain binds to an asparagine residue, can be suitably used.

Pharmaceutical preparations containing the conjugate or fusion protein of the M6P low-modified protein and the ligand of the present invention as an active ingredient can be administered intravenously, intramuscularly, intraperitoneally, subcutaneously, or intracerebroventricularly as injections. These injections can be supplied as lyophilized formulations or aqueous liquid preparations. The injection being an aqueous liquid preparation may be filled in a vial or can be supplied as a prefilled preparation, in which the aqueous liquid preparation is prefilled in a syringe in advance. The injection being a lyophilized formulation is dissolved and reconstituted in an aqueous medium before use.

### Examples

Hereinafter, the present invention will be described in more detail with reference to examples, but the present invention is not intended to be limited to the examples.

### Example 1: Construction of Dual (+)pCI-neo vector

A primer having a base sequence represented by SEQ ID NO: 32 (pCI Insert F3 primer) including an NheI site, an AscI site, an NruI site, an AfeI site, and an NotI site into which an NotI nullifying sequence was inserted from the 5' side was synthesized. In addition, a primer (pCI Insert R3 primer) having a base sequence represented by SEQ ID NO: 33 including a sequence complementary thereto was synthesized. These two primers were annealed to obtain a DNA fragment having NheI and NotI-digested DNA, and complementary cohesive ends at both ends.

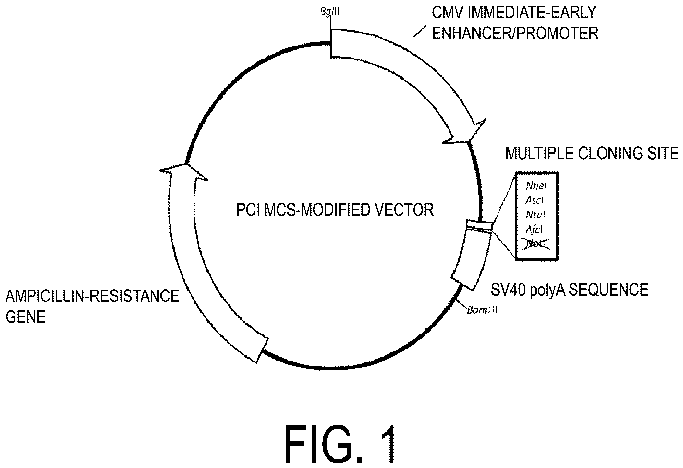

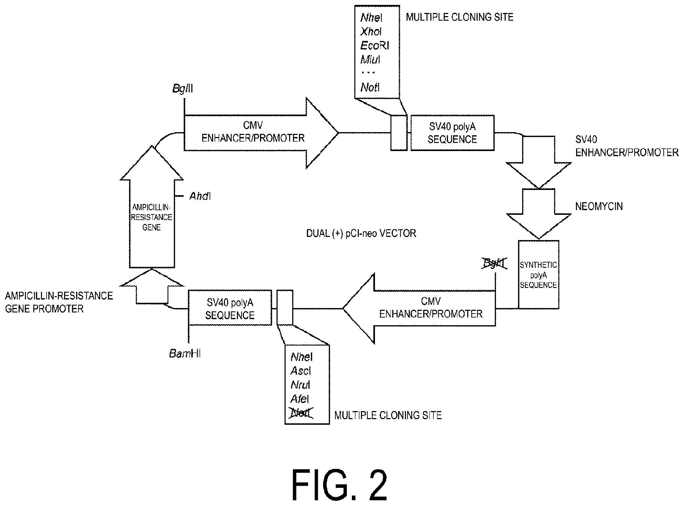

A DNA fragment pCI vector (Promega) was digested with NheI and NotI, and the above DNA fragment was inserted into the vector by a ligation reaction. The resulting plasmid was designated as a pCI MCS-modified vector (FIG. 1). The pCI MCS-modified vector was digested with BamHI and BglII to excise a DNA fragment including a CMV immediate-early enhancer/promoter region, a multiple cloning site, and a polyA sequence. A pCI-neo vector (Promega) was digested with BamHI and treated with CIAP, and the excised DNA fragment was inserted by a ligation reaction. The resulting plasmid was designated as a Dual (+)pCI-neo vector (FIG. 2).

### Example 2: Construction of vector for expressing each Fab-IDS

Three types of expression vectors for expressing the fusion proteins shown in the following (1) to (3) respectively were prepared by the method described below.
(1) A fusion protein of a wild-type hIDS and an anti-hTfR (Fab) antibody (Fab-IDS (WT)), the fusion protein including a conjugate in which a Fab heavy chain having the amino acid sequence represented by SEQ ID NO: 31 is bound to the N-terminus side of the wild-type hIDS represented by SEQ ID NO: 1 via a linker having an amino acid sequence composed of three amino acid sequences represented by SEQ ID NO: 26 in series, and a Fab light chain having the amino acid sequence of SEQ ID NO: 29.
(2) A fusion protein of a wild-type hIDS and an anti-hTfR (Fab) antibody (Fab-IDS (m1)), the fusion protein including a conjugate in which a Fab heavy chain having the amino acid sequence represented by SEQ ID NO: 31 is bound to the N-terminus side of an hIDS mutant having the amino acid sequence represented by SEQ ID NO: 5 in which threonine at position 223 and serine at position 257 in the amino acid sequence represented by the wild-type hIDS represented by SEQ ID NO: 1 are each substituted with alanine (hereinafter, referred to as muhIDS-1) via a linker having an amino acid sequence composed of three amino acid sequences represented by SEQ ID NO: 26 in series, and a Fab light chain having the amino acid sequence represented by SEQ ID NO: 29.
(3) A fusion protein of a wild-type hIDS and an anti-hTfR (Fab) antibody (Fab-IDS (m2)), the fusion protein including a conjugate in which a Fab heavy chain having the amino acid sequence represented by SEQ ID NO: 31 is bound to the N-terminus side of an hIDS mutant having the amino acid sequence represented by SEQ ID NO: 4 in which asparagines at positions 221 and 255 in the amino acid sequence of the wild-type hIDS represented by SEQ ID NO: 1 are each substituted with glutamine (hereinafter, referred to as muhIDS-2) via a linker having an amino acid sequence composed of three amino acid sequences represented by SEQ ID NO: 26 in series, and a Fab light chain having the amino acid sequence represented by SEQ ID NO: 29.

Note that the three types of fusion proteins described above are collectively referred to as Fab-IDS.

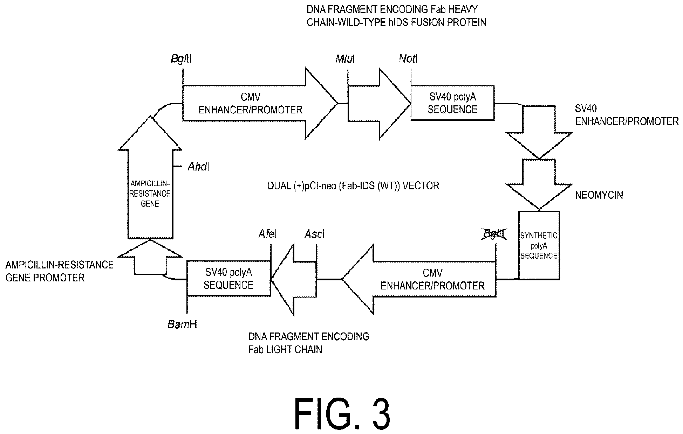

IN-FUSION (trade name) HD Cloning Kit (Takara Bio Inc.) was used to insert a DNA fragment including the base sequence represented by SEQ ID NO: 35 encoding a conjugate of the heavy chain of an anti-hTfR (Fab) antibody and the wild-type hIDS, having the amino acid sequence represented by SEQ ID NO: 34, between Mlu I and Not I of the Dual (+)pCI-neo vector obtained in Example 1. A DNA fragment including the base sequence represented by SEQ ID NO: 36 encoding the light chain of the anti-hTfR (Fab) antibody having the amino acid sequence represented by SEQ ID NO: 29 was inserted between Asc I and Afe I. The resulting vector was designated as Dual (+)pCI-neo (Fab-IDS (WT)) vector (FIG. 3). This vector was used as a Fab-IDS (WT) expression vector.

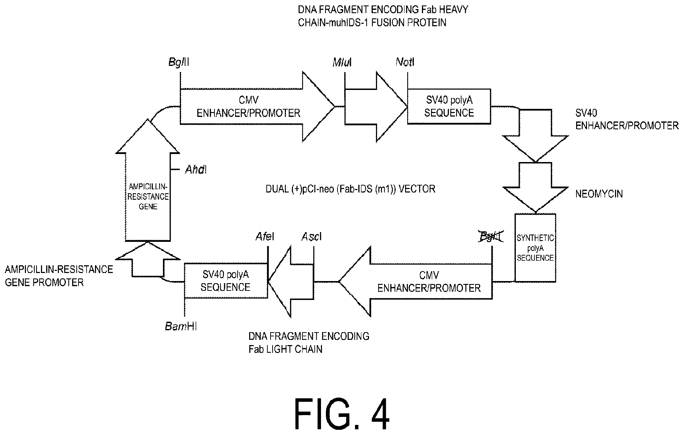

Similarly. a DNA fragment including the base sequence represented by SEQ ID NO: 38 encoding a conjugate in which muhIDS-1 was bound to the C-terminus of the heavy chain of the anti-hTfR (Fab) having the amino acid sequence represented by SEQ ID NO: 37 was inserted between Mlu I and Not I of the Dual (+)pCI-neo vector, and a DNA fragment including the base sequence represented by SEQ ID NO: 36 encoding the light chain of the anti-hTfR (Fab) antibody having the amino acid sequence represented by SEQ ID NO: 29 was inserted between Asc I and Afe I to obtain a Dual (+)pCI-neo (Fab-IDS (m1)) vector (FIG. 4). This vector was used as a Fab-IDS (m1) expression vector.

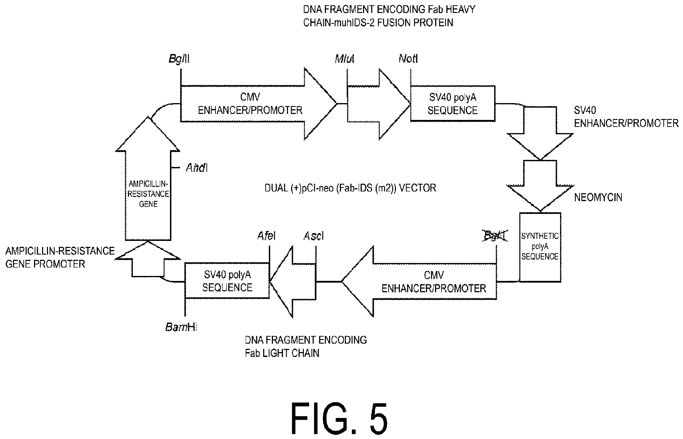

Further, similarly, a DNA fragment including the base sequence represented by SEQ ID NO: 40 encoding a conjugate in which muhIDS-2 was bound to the C-terminus of the heavy chain of the anti-hTfR (Fab) antibody having the amino acid sequence represented by SEQ ID NO: 39 was inserted between Mlu I and Not I of the Dual (+)pCI-neo vector, and a DNA fragment including the base sequence represented by SEQ ID NO: 36 encoding the light chain of the anti-hTfR (Fab) antibody having the amino acid sequence represented by SEQ ID NO: 29 was inserted between Asc I and Afe I to obtain a Dual (+)pCI-neo (Fab-IDS (m2)) vector (FIG. 5). This vector was used as a Fab-IDS (m2) expression vector.

### Example 3: Preparation of cell expressing each Fab-IDS

ExpiCHO Expression System (Thermo Fisher Scientific Inc.) was used to introduce the Fab-IDS (WT) expression vector, the Fab-IDS (m1) expression vector, and the Fab-IDS (m2) expression vector transiently into ExpiCHO cells to obtain cells expressing Fab-IDS (WT), Fab-IDS (m1), and Fab-IDS (m2), respectively.

### Example 4: Expression and purification of each Fab-IDS

The Fab-IDS (WT)-expressing cells obtained in Example 3 were seeded in a 125 mL Erlenmeyer flask at a cell density of 6 × 10⁶ cells/mL in an amount of 15 mL, and cultured in a humid environment composed of 5% CO₂ and 95% air at 37°C and at a stirring speed of about 120 rpm for 1 day. Thereafter, a feed solution and an enhancer of ExpiCHO Expression System were added to the flask, and the flask was moved to a humid environment composed of 5% CO₂ and 95% air at 32°C and cultured at a stirring speed of about 120 rpm for 6 to 7 days. The culture supernatant was collected by centrifugation and filtered through a 0.22 µm filter (Merck KGaA) to obtain a filtrate. The resulting filtrate was loaded to a Capture Select CH1-XL column (column volume: 0.5 mL, Thermo Fisher Scientific Inc.) which had been equilibrated with 5 column volume of 20 mM HEPES buffer solution (pH 7.4) containing 50 mM NaCl. Then, the column was washed twice with 5 column volume of the same buffer, and the adsorbed Fab-IDS (WT) was eluted twice with 4 column volume of 10 mM sodium citrate buffer solution (pH 4.0) containing 100 mM NaCl to collect eluted fractions. Then, the adsorbed Fab-IDS (WT) was eluted twice with 4 column volume of a 10 mM sodium citrate buffer solution containing 100 mM NaCl (pH 3.5) to collect eluted fractions. The obtained eluted fractions were combined, adjusted to around neutral by adding an appropriate amount of 500 mM MES buffer solution (pH 6.5), and then subjected to buffer exchange with 20 mM MES buffer solution (pH 6.5) containing 150 mM NaCl using Amicon Ultra 15 (Ultracel-10K, Merck KGaA) to obtain a purified product of Fab-IDS (WT). Similarly, purified products of Fab-IDS (m1) and Fab-IDS (m2) were prepared using the cells expressing Fab-IDS (m1) and Fab-IDS (m2) obtained in Example 3, respectively.

### Example 5: Determination of affinity of each Fab-IDS for human TfR

The affinity between each Fab-IDS and human TfR was measured using Octet RED96 (ForteBio), which is a biomolecular interaction analysis system using bio-layer interferometry (BLI). The basic principle of the bio-layer interferometry will be briefly described. When light of a specific wavelength is projected onto a layer of biomolecules immobilized on the surface of a sensor chip, the light is reflected from two surfaces of the layer of biomolecules and an internal reference layer to generate an interference wave of light. Binding of molecules in the measurement sample to biomolecules on the surface of the sensor chip increases the thickness of the layer at the tip of the sensor to generate a wavelength shift in the interference wave. When the change in the wavelength shift is measured, the number of molecules binding to the biomolecules immobilized on the surface of the sensor chip can be quantified and the kinetic analysis can be performed in real time. The measurement was performed generally in accordance with the operation manual attached to the Octet RED96. The human TfR (hTfR) used was obtained by adding a histidine tag to the N-terminus of the amino acid sequence of the extracellular region of hTfR from a glycine residue at the 87th position from the N-terminus side to phenylalanine at the C-terminus in the amino acid sequence of hTfR represented by SEQ ID NO: 28 via a linker composed of an amino acid sequence represented by Gly-Ser.

The purified product of each Fab-IDS prepared in Example 4 was serially diluted with HBS-P+ (1% BSA) (10 mM HEPES containing 1% BSA, 150 mM NaCl, 50 µM EDTA, and 0.05% surfactant P20) to prepare solutions having three concentrations of 10 nM, 20 nM, and 40 nM (1.08 µg/mL, 2.17 µg/mL, and 4.34 µg/mL, respectively), which were used as sample solutions for measuring hTfR affinity. In addition, hTfR was adjusted to a concentration of 25 µg/mL using HBS-P+ (1% BSA) to obtain an hTfR-ECD (His tag) solution.

The sample solution for hTfR affinity measurement was added to a 96-well plate, black (Greiner Bio-One International GmbH) at 200 µL/well. The hTfR-ECD (His tag) solution was added to predetermined wells at 200 µL/well. HBS-P+ (1% BSA) was added to wells for baseline, dissociation, and washing at 200 µL/well. 10 mM Glycine-HCl (pH 1.7) was added to wells for regeneration at 200 µL/well. 0.5 mM NiCl₂ (1% BSA) solution was added to wells for activation at 200 µL/well. This plate and a biosensor (Biosensor/Ni-NTA, ForteBio) were placed at predetermined positions in the Octet RED96.

After the Octet RED96 was operated under the conditions shown in Table 1 below to acquire data, binding curves were fitted to a 1 : 1 binding model using the analysis software attached to the Octet RED96, and an association rate constant (kₒₙ) and a dissociation rate constant (k_{off}) of each Fab-IDS with respect to human TfR were measured to calculate a dissociation constant (K_{D}). Note that the measurement was carried out at a temperature of 25 to 30°C.

**[Table 1]**

| Table 1 Operating condition of OctetRED96 1 | | | | |
|---|---|---|---|---|
| | Step | Contact time (sec) | Speed (rpm) | Threshold |
| 1 | Regeneration | 5 | 1000 | - |
| 2 | Washing | 5 | 1000 | - |
| Steps 1 to 2 above are repeated 6 times. | | | | |
| 3 | Activation | 60 | 1000 | - |
| 4 | Baseline 1 | 600 | 1000 | - |
| 5 | Load | 600 | 1000 | 1.0 |
| 6 | Baseline 2 | 150 | 1000 | - |
| 7 | Association | 120 | 1000 | - |
| 8 | Dissociation | 200 | 1000 | - |
| Steps 1 to 8 are repeated until the measurement of all samples is completed. | | | | |

Table 2 shows the measurement results of the association rate constants (kₒₙ) and dissociation rate constants (k_{off}) of each Fab-IDS with respect to human TfR, and the dissociation constants (K_{D}).

**[Table 2]**

| Table 2 Results of affinity measurement of each Fab-IDS for human TfR | | | |
|---|---|---|---|
| Sample name | kₒₙ (1/Ms) | k_{off} (1/s) | K_{D} (M) |
| Fab-IDS (WT) | 4.94 × 10⁵ | 3.31 × 10⁻⁴ | 6.70 × 10⁻¹⁰ |
| Fab-IDS (m1) | 4.89 × 10⁵ | 3.31 × 10⁻⁴ | 6.78 × 10⁻¹⁰ |
| Fab-IDS (m2) | 4.69 × 10⁵ | 3.10 × 10⁻⁴ | 6.61 × 10⁻¹⁰ |

As a result of measurement of the affinity of each Fab-IDS for human TfR, the dissociation constants of Fab-IDS (WT), Fab-IDS (m1), and Fab-IDS (m2) with respect to human TfR were 6.70 × 10⁻¹⁰ M, 6.78 × 10⁻¹⁰ M, and 6.61 × 10⁻¹⁰ M, respectively. The above results indicate that Fab-IDS maintains affinity for human TfR as a whole even when the IDS portion is replaced from the wild type to the mutant, i.e., maintains brain transferability.

### Example 6: Determination of affinity of each Fab-IDS for human M6PR

The affinity between each Fab-IDS and a human mannose-6-phosphate receptor (human M6PR) was measured using Octet RED96 (ForteBio), which is a biomolecular interaction analysis system using bio-layer interferometry (BLI). As the human M6PR, there was used a protein having the amino acid sequence represented by SEQ ID NO: 42, in which restriction enzyme-derived amino acids were added to both ends of the amino acid sequence corresponding to domain 9 from valine at the 1219th position to phenylalanine at the 1364th position from the N-terminus side in the amino acid sequence of a cation-independent mannose-6-phosphate receptor represented by SEQ ID NO: 41, and a histidine tag was further added to the C-terminus thereof.

The purified product of each Fab-IDS prepared in Example 4 was serially diluted with HBS-P+ (10 mM HEPES containing 150 mM NaCl, 50 µM EDTA, and 0.05% surfactant P20) to prepare solutions having seven concentrations of 1.6 nM to 100 nM (0.17 µg/mL to 10.84 µg/mL), which were used as sample solutions for M6PR affinity measurement (each Fab-IDS solution). In addition, human M6PR was adjusted to a concentration of 3 µ/mL with HBS-P+ to obtain an M6PR (His tag) solution.

The sample solution for M6PR affinity measurement was added to a 96-well plate, black (Greiner Bio-One International GmbH) at 200 µL/well. The M6PR (His tag) solution was added to predetermined wells at 200 µL/well. HBS-P+ was added to wells for baseline, dissociation, and washing at 200 µL/well. 10 mM Glycine-HCl (pH 1.7) was added to wells for regeneration at 200 µL/well. 0.5 mM NiCl₂ solution was added to wells for activation at 200 µL/well. This plate and a biosensor (Biosensor/Ni-NTA, ForteBio) were placed at predetermined positions in the Octet RED96.

After the Octet RED96 was operated under the conditions shown in Table 3 below to acquire data, binding curves were fitted to a 1 : 1 binding model using the analysis software attached to the Octet RED96, and an association rate constant (kₒₙ) and a dissociation rate constant (k_{off}) of each Fab-IDS with respect to M6PR were measured to calculate a dissociation constant (K_{D}). Note that the measurement was carried out at a temperature of 25 to 30°C.

**[Table 3]**

| Table 3 Operating conditions of OctetRED96 2 | | | | |
|---|---|---|---|---|
| | Step | Contact time (sec) | Speed (rpm) | Threshold |
| 1 | Regeneration | 5 | 1000 | - |
| 2 | Washing | 5 | 1000 | - |
| Steps 1 to 2 above are repeated 6 times. | | | | |
| 3 | Activation | 60 | 1000 | - |
| 4 | Baseline 1 | 600 | 1000 | - |
| 5 | Load | 600 | 1000 | 1.0 |
| 6 | Baseline 2 | 150 | 1000 | - |
| 7 | Association | 120 | 1000 | - |
| 8 | Dissociation | 200 | 1000 | - |
| Steps 1 to 8 are repeated until the measurement of all samples is completed. | | | | |

Table 4 shows the measurement results of the association rate constant (kₒₙ) and the dissociation rate constant (k_{off}), and the dissociation constant (K_{D}), of each Fab-IDS with respect to the M6PR. Fab-IDS (m1) and Fab-IDS (m2) had affinity for M6PR lower than the detection limit, and thus dissociation constants were not measurable.

**[Table 4]**

| Table 4 Results of affinity measurement of each Fab-IDS for M6PR | | | |
|---|---|---|---|
| Sample name | kₒₙ (1/Ms) | k_{off} (1/s) | K_{D} (M) |
| Fab-IDS (WT) | 2.53 × 10⁵ | 5.86 × 10⁻⁴ | 2.32 × 10⁻⁹ |
| Fab-IDS (m1) | N/A | N/A | N/A |
| Fab-IDS (m2) | N/A | N/A | N/A |

As a result of measurement of the affinity of each Fab-IDS for M6PR, the dissociation constant of Fab-IDS (WT) for human M6PR was 2.32 × 10⁻⁹ M. On the other hand, Fab-IDS (m1) and Fab-IDS (m2) did not show affinity for M6PR. These results indicate that in Fab-IDS (WT), a sugar chain containing M6P is added to the asparagine residue in the consensus sequence of the IDS moiety, whereas in Fab-IDS (m1) and Fab-IDS (m2), a sugar chain containing M6P is deleted.

### Example 7: Measurement of enzyme activity of each Fab-IDS

The enzyme activity of each Fab-IDS was measured using 4-methylumbelliferyl sulfate (4-MUS, potassium salt, Sigma-Aldrich), which was an artificial substrate for IDS.

The purified product of each Fab-IDS was diluted with a dilution buffer (5 mM acetic acid-sodium acetate buffer containing 0.05% BSA and 0.1% TritonX-100, pH 4.5) to adjust a concentration to 3.5 µg/mL, and the resulting solution was used as a sample solution for enzyme activity measurement. 4-methylumbelliferon (4-MU, Sigma-Aldrich) was diluted with the same dilution buffer to prepare 100 µM, 80 µM, 60 µM, 40 µM, 20 µM, and 10 µM solutions, which were used as standard solutions. In addition, 4-MUS was dissolved in the same dilution buffer and adjusted to a concentration of 5 mM, which was used as a substrate solution. The sample solutions and the standard solutions were added to a microplate (Nunc Fluoroplate F96 Black, Thermo Fisher Scientific Inc.) at 50 µL/well, and the substrate solution was further added to the same wells at 100 µL/well. The plate was covered with a lid and immediately agitated on a plate shaker for mixing. The plate was kept warm at 37°C for 1 hour, and then a stop solution (430 mM glycine-270 mM sodium carbonate, pH 10.7) was added at 150 µL/well to stop the reaction. Fluorescence (excitation wavelength: 355 nm, fluorescence wavelength: 460 nm) was measured with a fluorescence plate reader (Spectra Max iD3, Molecular Devices, LLC) to measure the concentration of released 4-MU. Table 5 shows the measurement results of the enzyme activity of each Fab-IDS.

**[Table 5]**

| Table 5 Results of enzyme activity measurement of each Fab-IDS | |
|---|---|
| Sample name | Enzyme activity (mU/mg) |
| Fab-IDS (WT) | 316 |
| Fab-IDS (m1) | 329 |
| Fab-IDS (m2) | 215 |

As a result of enzyme activity measurement of each Fab-IDS using the artificial substrate, the enzyme activities of Fab-IDS (WT), Fab-IDS (m1), and Fab-IDS (m2) were 316 mU/mg, 329 mU/mg, and 215 mU/mg, respectively. The above results indicate that Fab-IDS maintains the enzyme activity as IDS even when it is an IDS mutant in which the IDS moiety lacks a sugar chain containing M6P.

### Example 8: Measurement of expression level of hTfR on cell membrane using JKT beta del cell

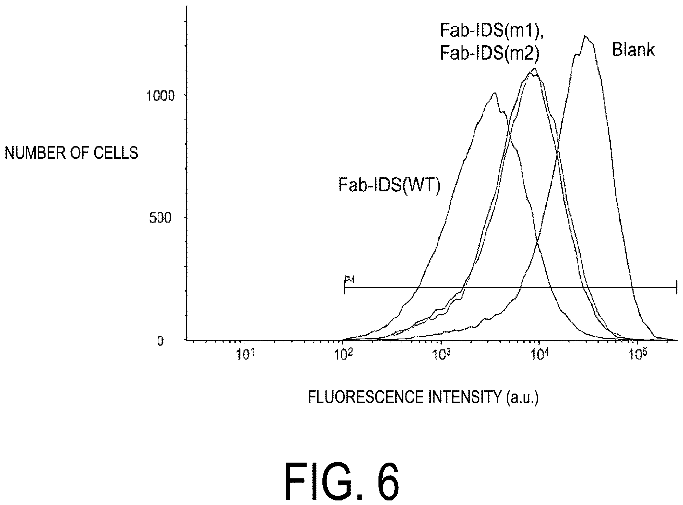

The expression level of hTfR on a cell membrane when each Fab-IDS was caused to act on the cell was measured by flow cytometry using Jurkat cells (JKT beta del cells, available from JCRB Cell Bank; cell number JCRB0147). The concentration of cells was adjusted to 2 × 10⁶ cells/mL using an RPMI1640 medium (Thermo Fisher Scientific Inc.) containing 10% FBS, 100 Units/mL penicillin, and 100 µg/mL streptomycin, and 1 mL of the adjusted cell solution was seeded in each well of a 24-well plate (round/flat-bottom Nunclon Delta-treated, Thermo Fisher Scientific). The Fab-IDS solution prepared in Example 4 was diluted to a concentration of 5000 nM using 20 mM MES buffer solution (pH 6.5) containing 150 mM NaCl, and 10 µL of each solution or 10 µL of 20 mM MES buffer solution (pH 6.5) containing 150 mM NaCl was added to each well in which the cell solution was seeded. The plate was then allowed to stand at 37°C in a humid environment composed of 5% CO₂ and 95% air for 1 day to perform culture. After completion of the culture, cells in each well were collected in a 1.5 mL centrifuge tube and centrifuged, and the supernatant was discarded and the cells were suspended in 1 mL of PBS containing 0.5% BSA. After the cells were washed by repeating the same operation three times in total, 5 µL of Transferrin From Human Serum, ALEXA FLUOR (trade name) 647 Conjugate (Thermo Fisher Scientific Inc.), which is fluorescence-labeled transferrin, was added to each solution, and the tube was allowed to stand at 37°C for 10 minutes. The cells were washed twice with PBS containing 0.5% BSA, and 1 µL of Sytox Green (Thermo Fisher Scientific Inc.) was added to each solution. The tube was allowed to stand on ice for 15 minutes or longer, and then fluorescence (excitation wavelength: 650 nm, fluorescence wavelength: 665 nm) of Alexa Fluor (trade name) 647 was measured using a flow cytometer (FACSVerse, available from BD Biosciences). The flow cytometer is a device that measures fluorescence intensities of individual cells passing through a flow cell. The measurement results (histogram plot) are shown in FIG. 6.

In FIG. 6, rightward shifting of the peak indicates a larger fluorescence intensity of the cells as a whole. The fluorescence intensity is positively correlated with the number of fluoresce-labeled transferrin binding to the cell surface. The fluorescence-labeled transferrin binds to the cell surface via hTfR, and thus the fluorescence intensity is positively correlated with the number of hTfR present on the cell surface. In the figure, the fluorescence intensity is highest for Blank to which the buffer solution was added, and lowest for the sample to which Fab-IDS (WT) was added. This result indicates that in the sample to which Fab-IDS (WT) was added, after Fab-IDS (WT) was bound to hTfR present on the cell surface, hTfR was taken up into the cell together with Fab-IDS (WT) and disappeared, and the number of hTfR present on the cell surface was reduced. The fluorescence intensity of the sample to which each of Fab-IDS (m1) and Fab-IDS (m2) was added was a value between the value of the sample to which Fab-IDS (WT) was added and the value of Blank. As shown in Table 5, hTfR and each Fab-IDS have almost the same affinity. Accordingly, these results indicate that hTfR binding to Fab-IDS (m1) remains on the cells even after Fab-IDS (m1) is taken up into the cells, or the ratio of hTfR returned to the cell membrane after being taken up into the cells together with Fab-IDS (m1) is high as compared with hTfR binding to Fab-IDS (m1). The same is true for Fab-IDS (m2).

Table 6 shows the average values of fluorescence intensities per cell calculated from the measurement results. In a case where the buffer solution (Blank), Fab-IDS (WT), Fab-IDS (m1), and Fab-IDS (m2) were added, the average values of the fluorescence intensities derived from the fluorescence-labeled transferrin per one cell were 30738, 4441, 10363, and 9520, respectively. That is, it was shown that in a case where Fab-IDS (WT) was caused to act on cells, the number of TfR present on the surfaces of the cells was reduced to about 14% as compared with Blank, and that in a case where Fab-IDS (m1) or Fab-IDS (m2) was caused to act on cells, the reduction rate of the number of TfR present on the surfaces of the cells was only about 34% and about 31% as compared with Blank.

**[Table 6]**

| Table 6 Average value of fluorescence intensities derived from fluorescent-labeled transferrin per cell | |
|---|---|
| Sample name | Average value of fluorescence intensities |
| Blank | 30738 |
| Fab-IDS (WT) | 4441 |
| Fab-IDS (m1) | 10363 |
| Fab-IDS (m2) | 9520 |

These results indicate that in a case where a fusion protein in which a wild-type glycoprotein containing M6Ps in its sugar chain is fused with an anti-TfR antibody is to be developed as a pharmaceutical, the fusion protein is made to contain no M6P or to have a reduced number of M6Ps, whereby the fusion protein can be developed as a pharmaceutical that has no or reduced side effects caused by a reduction in the number of TfR present on cells when administered to a patient by intravenous injection or the like.

### Industrial Applicability

According to the present invention, for example, it is possible to provide a conjugate of an M6P low-modified protein and a ligand for a protein present on the surface of a cell. Such a conjugate includes a fusion protein of the M6P low-modified protein and an antibody against a protein present on the surface of a cell. Such a conjugate can be used as a new type of pharmaceutical capable of suppressing a decrease in the number of proteins present on a cell surface for which the ligand has affinity when the conjugate is administered in vivo.

### Sequence table free text

SEQ ID NO: 1: amino acid sequence of wild-type hIDS
SEQ ID NO: 2: an example of base sequence encoding wild-type hIDS
SEQ ID NO: 3: amino acid sequence of wild-type hIDS precursor
SEQ ID NO: 4: amino acid sequence 1 of M6P low-modified hIDS (muhIDS-2)
SEQ ID NO: 5: amino acid sequence 2 of M6P low-modified hIDS (muhIDS-1)
SEQ ID NO: 6: amino acid sequence 3 of M6P low-modified hIDS
SEQ ID NO: 7: amino acid sequence of wild-type hIDUA
SEQ ID NO: 8: an example of base sequence encoding wild-type hIDUA
SEQ ID NO: 9: amino acid sequence of wild-type hIDUA precursor
SEQ ID NO: 10: amino acid sequence 1 of M6P low-modified hIDUA
SEQ ID NO: 11: amino acid sequence 2 of M6P low-modified hIDUA
SEQ ID NO: 12: amino acid sequence 3 of M6P low-modified hIDUA
SEQ ID NO: 13: amino acid sequence of wild-type hSGSH
SEQ ID NO: 14: an example of base sequence encoding wild-type hSGSH
SEQ ID NO: 15: amino acid sequence of wild-type hSGSH precursor
SEQ ID NO: 16: amino acid sequence 1 of M6P low-modified hSGSH
SEQ ID NO: 17: amino acid sequence 2 of M6P low-modified hSGSH
SEQ ID NO: 18: amino acid sequence 3 of M6P low-modified hSGSH
SEQ ID NO: 19: amino acid sequence of wild-type hGAA
SEQ ID NO: 20: an example of base sequence encoding wild-type hGAA
SEQ ID NO: 21: amino acid sequence of wild-type hGAA precursor
SEQ ID NO: 22: amino acid sequence 1 of M6P low-modified hGAA
SEQ ID NO: 23: amino acid sequence 2 of M6P low-modified hGAA
SEQ ID NO: 24: amino acid sequence 3 of M6P low-modified hGAA
SEQ ID NO: 25: an example 1 of amino acid sequence of linker
SEQ ID NO: 26: an example 2 of amino acid sequence of linker
SEQ ID NO: 27: an example 3 of amino acid sequence of linker
SEQ ID NO: 28: amino acid sequence of human transferrin receptor
SEQ ID NO: 29: amino acid sequence of light chain of anti-hTfR antibody
SEQ ID NO: 30: amino acid sequence of heavy chain of anti-hTfR antibody
SEQ ID NO: 31: amino acid sequence of Fab heavy chain of anti-hTfR antibody
SEQ ID NO: 32: pCI Insert F3 primer, synthetic sequence
SEQ ID NO: 33: pCI Insert R3 primer, synthetic sequence
SEQ ID NO: 34: amino acid sequence of fusion protein of Fab heavy chain of anti-hTfR antibody and wild-type hIDS
SEQ ID NO: 35: base sequence encoding fusion protein of Fab heavy chain of anti-hTfR antibody and wild-type hIDS, synthetic sequence
SEQ ID NO: 36: base sequence encoding light chain of anti-hTfR antibody, synthetic sequence
SEQ ID NO: 37: amino acid sequence of fusion protein of Fab heavy chain of anti-hTfR antibody and muhIDS-1
SEQ ID NO: 38: base sequence encoding fusion protein of Fab heavy chain of anti-hTfR antibody and muhIDS-1, synthetic sequence
SEQ ID NO: 39: amino acid sequence of fusion protein of Fab heavy chain of anti-hTfR antibody and muhIDS-2
SEQ ID NO: 40: base sequence encoding fusion protein of Fab heavy chain of anti-hTfR antibody and muhIDS-2, synthetic sequence
SEQ ID NO: 41: amino acid sequence of human M6P receptor (hM6PR)
SEQ ID NO: 42: amino acid sequence of hM6PR domain 9 recombinant protein

## Claims

1. A human lysosomal enzyme comprising:
in a wild type, one or more N-linked sugar chains binding thereto, and
the one or more N-linked sugar chains each containing one or more mannose-6-phosphates (M6Ps); wherein
the human lysosomal enzyme comprises deletion of at least one of the M6Ps.

2. The human lysosomal enzyme according to claim 1, wherein at least one of the one or more N-linked sugar chains is deleted.

3. The human lysosomal enzyme according to claim 1 or 2, wherein the human lysosomal enzyme comprises a mutation in such a manner that at least one of the one or more N-linked sugar chains is deleted from an amino acid sequence of a wild-type human lysosomal enzyme corresponding to the human lysosomal enzyme.

4. The human lysosomal enzyme according to claim 3, wherein the mutation of the amino acid sequence includes at least one mutation selected from the group consisting of the following (1-a) to (1-c) with respect to an amino acid sequence (consensus sequence) represented by Asn-Xaa-Yaa (where Xaa represents an amino acid other than proline, and Yaa represents threonine or serine) containing asparagine (Asn) to which the N-linked sugar chain binds, and
the mutation newly generates no consensus sequence to which an N-linked sugar chain binds:
(1-a) deletion of Asn or substitution of Asn with an amino acid other than asparagine;
(1-b) substitution of an amino acid represented by Xaa with proline; and
(1-c) substitution of an amino acid represented by Yaa with an amino acid other than threonine and serine.

5. The human lysosomal enzyme according to claim 1 or 2, being human iduronate-2-sulfatase (hIDS).

6. The hIDS according to claim 5, comprising at least one mutation selected from the group consisting of the following (2-a) to (2-c) with respect to an amino acid sequence represented by Asn221-Ile222-Thr223 corresponding to from asparagine at position 221 to threonine at position 223 in an amino acid sequence of a wild-type hIDS represented by SEQ ID NO: 1,
wherein the at least one mutation newly generates no consensus sequence to which an N-linked sugar chain binds:
(2-a) deletion of Asn221 or substitution of Asn221 with an amino acid other than asparagine;
(2-b) substitution of Ile222 with proline; and
(2-c) substitution of Thr223 with an amino acid other than threonine and serine.

7. The hIDS according to claim 5, wherein a mutation is added in such a manner that the N-linked sugar chain binding to asparagine at position 221 is deleted by adding a mutation to an amino acid sequence corresponding to from asparagine at position 221 to threonine at position 223 in the amino acid sequence of the wild-type hIDS represented by SEQ ID NO: 1.

8. The hIDS according to claim 6, further comprising a mutation selected from the group consisting of the following (2'-a) to (2'-h) in an amino acid sequence other than the amino acid sequence corresponding to from asparagine at position 221 to threonine at position 223 in the amino acid sequence of the wild-type hIDS represented by SEQ ID NO: 1,
wherein the mutation newly generates no consensus sequence to which an N-linked sugar chain binds:
(2'-a) substitution of 1 to 10 amino acids with another amino acid or other amino acids;
(2'-b) deletion of 1 to 10 amino acids;
(2'-c) a combination of the substitution of (2'-a) and the deletion of (2'-b);
(2'-d) addition of 1 to 10 amino acids into the amino acid sequence or to an N-terminus or a C-terminus of the amino acid sequence;
(2'-e) a combination of the substitution of (2'-a) and the addition of (2'-d);
(2'-f) a combination of the deletion of (2'-b) and the addition of (2'-d);
(2'-g) a combination of the substitution of (2'-a), the deletion of (2'-b), and the addition of (2'-d); and
(2'-h) exhibition of an identity of 80% or more.

9. The hIDS according to claim 5, comprising at least one mutation selected from the group consisting of the following (3-a) to (3-c) with respect to an amino acid sequence represented by Asn255-Ile256-Ser257 corresponding to from asparagine at position 255 to serine at position 257 in the amino acid sequence of the wild-type hIDS represented by SEQ ID NO: 1,
wherein the at least one mutation newly generates no consensus sequence to which an N-linked sugar chain binds:
(3-a) deletion of Asn255 or substitution of Asn255 with an amino acid other than Asn;
(3-b) substitution of Ile256 with proline; and
(3-c) substitution of Ser257 with an amino acid other than threonine and serine.

10. The hIDS according to claim 5, wherein a mutation is added in such a manner that the N-linked sugar chain binding to asparagine at position 255 is deleted by adding a mutation to an amino acid sequence corresponding to from asparagine at position 255 to serine at position 257 in the amino acid sequence of the wild-type hIDS represented by SEQ ID NO: 1.

11. The hIDS according to claim 10, further comprising a mutation selected from the group consisting of the following (3'-a) to (3'-h) in an amino acid sequence other than the amino acid sequence corresponding to from asparagine at position 255 to serine at position 257 in the amino acid sequence of the wild-type hIDS represented by SEQ ID NO: 1,
wherein the mutation newly generates no consensus sequence to which an N-linked sugar chain binds:
(3'-a) substitution of 1 to 10 amino acids with another amino acid or other amino acids;
(3'-b) deletion of 1 to 10 amino acids;
(3'-c) a combination of the substitution of (3'-a) and the deletion of (3'-b);
(3'-d) addition of 1 to 10 amino acids into the amino acid sequence or to an N-terminus or a C-terminus of the amino acid sequence;
(3'-e) a combination of the substitution of (3'-a) and the addition of (3'-d);
(3'-f) a combination of the deletion of (3'-b) and the addition of (3'-d);
(3'-g) a combination of the substitution of (3'-a), the deletion of (3'-b), and the addition of (3'-d); and
(3'-h) exhibition of an identity of 80% or more.

12. The hIDS according to claim 5, having an amino acid sequence selected from the following (4-a) to (4-e), and having no new consensus sequence to which an N-linked sugar chain binds:
(4-a) an amino acid sequence exhibiting, in the amino acid sequence except for amino acids at positions 221 and 255, an identity of 80% or more, 85% or more, 90% or more, 95% or more, 98% or more, or 99% or more, to an amino acid sequence of an hIDS mutant represented by SEQ ID NO: 4 in which asparagines at positions 221 and 255 in the amino acid sequence of the wild-type hIDS represented by SEQ ID NO: 1 are each substituted with glutamine;
(4-b) the amino acid sequence of the hIDS mutant represented by SEQ ID NO: 4 in which asparagines at positions 221 and 255 in the amino acid sequence of the wild-type hIDS represented by SEQ ID NO: 1 are each substituted with glutamine;
(4-c) an amino acid sequence exhibiting, in the amino acid sequence except for amino acids at positions 223 and 257, an identity of 80% or more, 85% or more, 90% or more, 95% or more, 98% or more, or 99% or more, to an amino acid sequence of an hIDS mutant represented by SEQ ID NO: 5 in which threonine at position 223 and serine at position 257 in the amino acid sequence of the wild-type hIDS represented by SEQ ID NO: 1 are each substituted with alanine;
(4-d) the amino acid sequence of the hIDS mutant represented by SEQ ID NO: 5 in which threonine at position 223 and serine at position 257 in the amino acid sequence of the wild-type hIDS represented by SEQ ID NO: 1 are each substituted with alanine; and
(4-e) an amino acid sequence of an hIDS mutant represented by SEQ ID NO: 6 in which asparagines at positions 221 and 255 are each substituted with glutamine and threonine at position 223 and serine at position 257 are each substituted with alanine in the amino acid sequence of the wild-type hIDS represented by SEQ ID NO: 1.

13. The human lysosomal enzyme according to claim 1 or 2, being human alpha-L-iduronidase (hIDUA).

14. The hIDUA according to claim 13, comprising at least one mutation selected from the group consisting of the following (5-a) to (5-c) with respect to an amino acid sequence represented by Asn311-Thr312-Thr313 corresponding to from asparagine at position 311 to threonine at position 313 in an amino acid sequence of a wild-type hIDUA represented by SEQ ID NO: 7, wherein the at least one mutation newly generates no consensus sequence to which an N-linked sugar chain binds:
(5-a) deletion of Asn311 or substitution of Asn311 with an amino acid other than asparagine;
(5-b) substitution of Thr312 with proline; and
(5-c) substitution of Thr313 with an amino acid other than threonine and serine.

15. The hIDUA according to claim 13, wherein a mutation is added in such a manner that the N-linked sugar chain binding to asparagine at position 311 is deleted by adding a mutation to the amino acid sequence corresponding to from the asparagine at position 311 to threonine at position 313 in the amino acid sequence of the wild-type hIDUA represented by SEQ ID NO: 7.

16. The hIDUA according to claim 14, further comprising a mutation selected from the group consisting of the following (5'-a) to (5'-h) in an amino acid sequence other than the amino acid sequence corresponding to from asparagine at position 311 to threonine at position 313 in the amino acid sequence of the wild-type hIDUA represented by SEQ ID NO: 7,
wherein the mutation newly generates no consensus sequence to which an N-linked sugar chain binds:
(5'-a) substitution of 1 to 10 amino acids with another amino acid or other amino acids;
(5'-b) deletion of 1 to 10 amino acids;
(5'-c) a combination of the substitution of (5'-a) and the deletion of (5'-b);
(5'-d) addition of 1 to 10 amino acids into the amino acid sequence or to an N-terminus or a C-terminus of the amino acid sequence;
(5'-e) a combination of the substitution of (5'-a) and the addition of (5'-d);
(5'-f) a combination of the deletion of (5'-b) and the addition of (5'-d);
(5'-g) a combination of the substitution of (5'-a), the deletion of (5'-b), and the addition of (5'-d); and
(5'-h) exhibition of an identity of 80% or more.

17. The hIDUA according to claim 13, comprising at least one mutation selected from the group consisting of the following (6-a) to (6-c) with respect to an amino acid sequence represented by Asn426-Arg427-Ser428 corresponding to from asparagine at position 426 to serine at position 428 in the amino acid sequence of the wild-type hIDUA represented by SEQ ID NO: 7,
wherein the at least one mutation newly generates no consensus sequence to which an N-linked sugar chain binds:
(6-a) deletion of Asn426 or substitution of Asn426 with an amino acid other than asparagine;
(6-b) substitution of Arg427 with proline; and
(6-c) substitution of Ser428 with an amino acid other than threonine and serine.

18. The hIDUA according to claim 13, wherein a mutation is added in such a manner that the N-linked sugar chain binding to asparagine at position 426 is deleted by adding a mutation to the amino acid sequence corresponding to from the asparagine at position 426 to serine at position 428 in the amino acid sequence of the wild-type hIDUA represented by SEQ ID NO: 7.

19. The hIDUA according to claim 17, further comprising a mutation selected from the group consisting of the following (6'-a) to (6'-h) in an amino acid sequence other than the amino acid sequence corresponding to from asparagine at position 426 to serine at position 428 in the amino acid sequence of the wild-type hIDUA represented by SEQ ID NO: 7,
wherein the mutation newly generates no consensus sequence to which an N-linked sugar chain binds:
(6'-a) substitution of 1 to 10 amino acids with another amino acid or other amino acids;
(6'-b) deletion of 1 to 10 amino acids;
(6'-c) a combination of the substitution of (6'-a) and the deletion of (6'-b);
(6'-d) addition of 1 to 10 amino acids into the amino acid sequence or to an N-terminus or a C-terminus of the amino acid sequence;
(6'-e) a combination of the substitution of (6'-a) and the addition of (6'-d);
(6'-f) a combination of the deletion of (6'-b) and the addition of (6'-d);
(6'-g) a combination of the substitution of (6'-a), the deletion of (6'-b), and the addition of (6'-d); and
(6'-h) exhibition of an identity of 80% or more.

20. The hIDUA according to claim 13, having an amino acid sequence selected from the following (7-a) to (7-e), and having no new consensus sequence to which an N-linked sugar chain binds:
(7-a) an amino acid sequence exhibiting, in the amino acid sequence except for amino acids at positions 311 and 426, an identity of 80% or more, 85% or more, 90% or more, 95% or more, 98% or more, or 99% or more, to an amino acid sequence of an hIDUA mutant represented by SEQ ID NO: 10 in which both asparagines at positions 311 and 426 in the amino acid sequence of the wild-type hIDUA represented by SEQ ID NO: 7 are each substituted with glutamine;
(7-b) the amino acid sequence of the hIDUA mutant represented by SEQ ID NO: 10 in which both asparagines at positions 311 and 426 in the amino acid sequence of the wild-type hIDUA represented by SEQ ID NO: 7 are each substituted with glutamine;
(7-c) an amino acid sequence exhibiting, in the amino acid sequence except for amino acids at positions 313 and 428, an identity of 80% or more, 85% or more, 90% or more, 95% or more, 98% or more, or 99% or more, to an amino acid sequence of an hIDUA mutant represented by SEQ ID NO: 11 in which threonine at position 313 and serine at position 428 in the amino acid sequence of the wild-type hIDUA represented by SEQ ID NO: 7 are each substituted with alanine;
(7-d) the amino acid sequence of the hIDUA mutant represented by SEQ ID NO: 11 in which threonine at position 313 and serine at position 428 in the amino acid sequence of the wild-type hIDUA represented by SEQ ID NO: 7 are each substituted with alanine; and
(7-e) an amino acid sequence of an hIDUA mutant represented by SEQ ID NO: 12 in which both asparagines at positions 311 and 426 in the amino acid sequence of the wild-type hIDUA represented by SEQ ID NO: 7 are each substituted with glutamine, and threonine at position 313 and serine at position 428 are each substituted with alanine.

21. The human lysosomal enzyme according to claim 1 or 2, being human heparan N-sulfatase (hSGSH).

22. The hSGSH according to claim 21, comprising at least one mutation selected from the group consisting of the following (8-a) to (8-c) with respect to an amino acid sequence represented by Asn244-Asp245-Thr246 corresponding to from asparagine at position 244 to threonine at position 246 in an amino acid sequence of a wild-type hSGSH represented by SEQ **ID** NO: 13, wherein the at least one mutation newly generates no consensus sequence to which an N-linked sugar chain binds:
(8-a) deletion of Asn244 or substitution of Asn244 with an amino acid other than asparagine;
(8-b) substitution of Asp245 with proline; and
(8-c) substitution of Thr246 with an amino acid other than threonine and serine.

23. The hSGSH according to claim 21, wherein a mutation is added in such a manner that the N-linked sugar chain binding to asparagine at 244 is deleted by adding a mutation to the amino acid sequence corresponding to from the asparagine at position 244 to threonine at position 246 in the amino acid sequence of the wild-type hSGSH represented by SEQ ID NO: 13.

24. The hSGSH according to claim 22, further comprising a mutation selected from the group consisting of the following (8'-a) to (8'-h) in an amino acid sequence other than the amino acid sequence corresponding to from asparagine at position 244 to threonine at position 246 in the amino acid sequence of the wild-type hSGSH represented by SEQ ID NO: 13, wherein the mutation newly generates no consensus sequence to which an N-linked sugar chain binds:
(8'-a) substitution of 1 to 10 amino acids with another amino acid or other amino acids;
(8'-b) deletion of 1 to 10 amino acids;
(8'-c) a combination of the substitution of (8'-a) and the deletion of (8'-b);
(8'-d) addition of 1 to 10 amino acids into the amino acid sequence or to an N-terminus or a C-terminus of the amino acid sequence;
(8'-e) a combination of the substitution of (8'-a) and the addition of (8'-d);
(8'-f) a combination of the deletion of (8'-b) and the addition of (8'-d);
(8'-g) a combination of the substitution of (8'-a), the deletion of (8'-b), and the addition of (2'-d); and
(8'-h) exhibition of an identity of 80% or more.

25. The hSGSH according to claim 21, having an amino acid sequence selected from the following (9-a) to (9-e), and having no new consensus sequence to which an N-linked sugar chain binds:
(9-a) an amino acid sequence exhibiting, in the amino acid sequence except for an amino acid at position 244, an identity of 80% or more, 85% or more, 90% or more, 95% or more, 98% or more, or 99% or more, to an amino acid sequence of an hSGSH mutant represented by SEQ ID NO: 16 in which asparagine at position 244 is substituted with glutamine in the amino acid sequence of the wild-type hSGSH represented by SEQ ID NO: 13;
(9-b) the amino acid sequence of the hSGSH mutant represented by SEQ ID NO: 16 in which asparagine at position 244 in the amino acid sequence of the wild-type hSGSH represented by SEQ ID NO: 13 is substituted with glutamine;
(9-c) an amino acid sequence exhibiting, in the amino acid sequence except for an amino acid at position 246, an identity of 80% or more, 85% or more, 90% or more, 95% or more, 98% or more, or 99% or more, to an amino acid sequence of an hSGSH mutant represented by SEQ ID NO: 17 in which threonine at position 246 in the amino acid sequence of the wild-type hSGSH represented by SEQ ID NO: 13 is substituted with alanine;
(9-d) the amino acid sequence of the hSGSH mutant represented by SEQ ID NO: 17 in which threonine at position 246 in the amino acid sequence of the wild-type hSGSH represented by SEQ ID NO: 13 is substituted with alanine; and
(9-e) an amino acid sequence of an hSGSH mutant represented by SEQ ID NO: 18 in which asparagine at position 244 is substituted with glutamine and threonine at position 246 is substituted with alanine in the amino acid sequence of the wild-type hSGSH represented by SEQ ID NO: 13.

26. The human lysosomal enzyme according to claim 1 or 2, being human acid α-glucosidase (hGAA).

27. The hGAA according to claim 26, comprising at least one mutation selected from the group consisting of the following (10-a) to (10-i) with respect to at least one of an amino acid sequence represented by Asn71-Leu72-Ser73 corresponding to from asparagine at position 71 to serine at position 73, an amino acid sequence represented by Asn164-Thr165-Thr166 corresponding to from asparagine at position 164 to threonine at position 166, and an amino acid sequence represented by Asn401-Glu402-Thr403 corresponding to from asparagine at position 401 to threonine at position 403 in an amino acid sequence of a wild-type hGAA represented by SEQ ID NO: 19,
wherein the at least one mutation newly generates no consensus sequence to which an N-linked sugar chain binds:
(10-a) deletion of Asn71 or substitution of Asn71 with an amino acid other than asparagine;
(10-b) substitution of Leu72 with proline;
(10-c) substitution of Ser73 with an amino acid other than threonine and serine;
(10-d) deletion of Asn164 or substitution of Asn164 with an amino acid other than asparagine;
(10-e) substitution of Thr165 with proline;
(10-f) substitution of Thr166 with an amino acid other than threonine and serine;
(10-g) deletion of Asn401 or substitution of Asn401 with an amino acid other than asparagine;
(10-h) substitution of Glu402 with proline; and
(10-i) substitution of Thr403 with an amino acid other than threonine and serine.

28. The hGAA according to claim 26, wherein a mutation is added in such a manner that the N-linked sugar chain binding to asparagine at any one of position 71, 164, or 401 is deleted by adding a mutation to at least one of the amino acid sequence corresponding to from asparagine at position 71 to threonine at position 73, the amino acid sequence corresponding to from asparagine at position 164 to threonine at position 166, and the amino acid sequence corresponding to from asparagine at position 401 to threonine at position 403 in the amino acid sequence of the wild-type hGAA represented by SEQ **ID** NO: 19.

29. The hGAA according to claim 27, further comprising a mutation selected from the group consisting of the following (10'-a) to (10'-h) in an amino acid sequence other than the amino acid sequences corresponding to from asparagine at position 71 to threonine at position 73, the amino acid sequences corresponding to from asparagine at position 164 to threonine at position 166, and the amino acid sequences corresponding to from asparagine at position 401 to threonine at position 403 in the amino acid sequence of the wild-type hGAA represented by SEQ ID NO: 19,
wherein the mutation newly generates no consensus sequence to which an N-linked sugar chain binds:
(10'-a) substitution of 1 to 10 amino acids with another amino acid or other amino acids;
(10'-b) deletion of 1 to 10 amino acids;
(10'-c) a combination of the substitution of (10'-a) and the deletion of (10'-b);
(10'-d) addition of 1 to 10 amino acids into the amino acid sequence or to an N-terminus or a C-terminus of the amino acid sequence;
(10'-e) a combination of the substitution of (10'-a) and the addition of (10'-d);
(10'-f) a combination of the deletion of (10'-b) and the addition of (10'-d);
(10'-g) a combination of the substitution of (10'-a), the deletion of (10'-b), and the addition of (10'-d); and
(10'-h) exhibition of an identity of 80% or more.

30. The hGAA according to claim 26, having an amino acid sequence selected from the following (11-a) to (11-e), and having no new consensus sequence to which an N-linked sugar chain binds:
(11-a) an amino acid sequence exhibiting, in the amino acid sequence except for amino acids at positions 71, 164, and 401, an identity of 80% or more, 85% or more, 90% or more, 95% or more, 98% or more, or 99% or more, to an amino acid sequence of an hGAA mutant represented by SEQ ID NO: 22 in which asparagines at positions 71, 164, and 401 in the amino acid sequence of the wild-type hGAA represented by SEQ ID NO: 19 are each substituted with glutamine;
(11-b) the amino acid sequence of the hGAA mutant represented by SEQ ID NO: 22 in which asparagines at positions 71, 164, and 401 in the amino acid sequence of the wild-type hGAA represented by SEQ ID NO: 19 are each substituted with glutamine;
(11-c) an amino acid sequence exhibiting, in the amino acid sequence except for amino acids at positions 73, 166, and 403, an identity of 80% or more, 85% or more, 90% or more, 95% or more, 98% or more, or 99% or more, to an amino acid sequence of an hGAA mutant represented by SEQ ID NO: 23 in which threonines at positions 73, 166, and 403 in the amino acid sequence of the wild-type hGAA represented by SEQ ID NO: 19 are each substituted with alanine;
(11-d) the amino acid sequence of the hGAA mutant represented by SEQ ID NO: 23 in which threonines at positions 73, 166, and 403 in the amino acid sequence of the wild-type hGAA represented by SEQ ID NO: 19 are each substituted with alanine; and
(11-e) an amino acid sequence of an hGAA mutant represented by SEQ ID NO: 24 in which asparagines at positions 71, 164, and 401 are each substituted with glutamine and threonines at positions 73, 166, and 403 are each substituted with alanine in the amino acid sequence of the wild-type hGAA represented by SEQ ID NO: 19.

31. One or more isolated nucleic acid molecules comprising a gene encoding the protein described in claim 1 or 2.

32. One or more expression vectors comprising the one or more nucleic acid molecules described in claim 31.

33. One or more mammalian cells transformed with the one or more expression vectors described in claim 32.

34. A fusion protein of the human lysosomal enzyme described in claim 1 or 2 and a ligand having affinity for a protein present on a muscle cell or a vascular endothelial cell.

35. The fusion protein according to claim 34, wherein the protein present on the muscle cell or the vascular endothelial cell is selected from the group consisting of an insulin receptor, a transferrin receptor, a leptin receptor, a lipoprotein receptor, and an IGF receptor.

36. The fusion protein according to claim 34, wherein the ligand having affinity for the protein present on the muscle cell or the vascular endothelial cell is an antibody against one selected from the group consisting of an insulin receptor, a transferrin receptor, a leptin receptor, a lipoprotein receptor, and an IGF receptor.

37. The fusion protein according to claim 36, wherein the antibody is any one of a Fab antibody, a F(ab')₂ antibody, an F(ab') antibody, a single domain antibody, a single chain antibody, or an Fc antibody.

38. One or more isolated nucleic acid molecules comprising a gene encoding the fusion protein described in claim 34.

39. One or more expression vectors comprising the one or more isolated nucleic acid molecules described in claim 38.

40. One or more mammalian cells transformed with the one or more expression vectors described in claim 39.
